# EUROPÄISCHE PATENTSCHRIFT

(11) **EP 0 882 010 B1**
(45) Veröffentlichungstag und Bekanntmachung des Hinweises auf die Patenterteilung: **02.05.2001**
(21) Anmeldenummer: 97903278.6
(22) Anmeldetag: 14.02.1997
(51) Int. Cl.: C07C 229/06, C07C 229/76, C07C 237/12, C07C 311/00, C07D 257/02, A61K 33/00, C07F 1/00, C07F 3/00, C07F 5/00, C07F 7/00

(54) **PHARMAZEUTISCHE MITTEL ENTHALTEND PERFLUORALKYLHALTIGE METALLKOMPLEXE UND IHRE VERWENDUNG IN DER TUMORTHERAPIE UND INTERVENTIONELLEN RADIOLOGIE**
PHARMACEUTICAL AGENTS CONTAINING PERFLUOROALKYL-CONTAINING METAL COMPLEXES AND THE USE THEREOF IN TUMOUR THERAPY AND INTERVENTIONAL RADIOLOGY
AGENTS PHARMACEUTIQUES CONTENANT DES COMPLEXES METALLIQUES PERFLUORALKYLES ET LEUR UTILISATION DANS LE TRAITEMENT DES TUMEURS ET EN RADIOLOGIE INTERVENTIONNELLE

(30) Priorität: 23.02.1996 DE 19608278
(43) Veröffentlichungstag der Anmeldung: 09.12.1998
(73) Patentinhaber: SCHERING AKTIENGESELLSCHAFT, 13353 Berlin (DE)
(72) Erfinder: PLATZEK, Johannes, D-12621 Berlin (DE); NIEDBALLA, Ulrich, D-14195 Berlin (DE); RADÜCHEL, Bernd, D-13465 Berlin (DE); SCHLECKER, Wolfgang, D-12047 Berlin (DE); WEINMANN, Hanns-Joachim, D-14129 Berlin (DE); FRENZEL, Thomas, D-12247 Berlin (DE)
(86) Internationale Anmeldenummer: EP9700684
(87) Internationale Veröffentlichungsnummer: WO9730969

(56) Entgegenhaltungen:
- EP-A- 0 673 655
- WO-A-93/07123
- WO-A-94/22368
- DE-A- 2 803 869
- PATENT ABSTRACTS OF JAPAN vol. 014, no. 068 (C-0686), 8.Februar 1990 & JP 01 290633 A (NIPPON KAYAKU CO LTD), 22.November 1989,
- PATENT ABSTRACTS OF JAPAN vol. 008, no. 251 (C-252), 16.November 1984 & JP 59 130812 A (MIDORI JUJI KK), 27.Juli 1984,
- PATENT ABSTRACTS OF JAPAN vol. 008, no. 251 (C-252), 16.November 1984 & JP 59 130813 A (MIDORI JUJI KK), 27.Juli 1984,

## Beschreibung

Die Erfindung betrifft die in den Patentansprüchen gekennzeichneten Gegenstände, d. h. pharmazeutische Mittel enthaltend monomere, perfluoralkylsubstituierte, Metallkomplexe und Komplexsalze in der Tumortherapie und der interventionellen Radiologie.

Die Verwendung von Fremdmaterialien - durch Injektion in den Blutkreislauf gebracht um dort eine Embolie zu induzieren - wurde bereits zu Beginn dieses Jahrhunderts vorgeschlagen [Dawbarn, Journal of the American Medical Association 43:792, (1904)].

Dieser Gedanke wurde jedoch erst vor etwa 30 Jahren wieder ernsthaft aufgegriffen. (Young, British Medicinal Journal 253, 1144, 1981). Die Embolisation wurde für diagnostische und therapeutische Zwecke eingesetzt, besonders für die Behandlung von Tumoren. Die Embolisation des Gefäßstammes, der einen Tumorbezirk mit Blut versorgt, ist eine Technik, die angewandt wird, um entweder eine dauerhafte Gefäßblockade hervorzurufen und dabei das Absterben des Tumors zu fördern oder eine zeitweilige Embolisation um die therapeutische Wirkung eines gleichzeitig mitapplizierten Chemotherapeutikums zu steigern. Die zuletzt genannte Technik wird als Chemoembolisation bezeichnet. Der Vorteil einer derartigen Behandlung ist deren lokale Begrenzung. Voraussetzung ist das Vorhandensein eines hinreichend großen (d.h. eine Kathetisierung ermöglichenden) den Tumor mit Blut versorgenden Gefäßes.

Beim Menschen sind Tumore der Leber besonders für eine Embolisationstherapie zugänglich. Lebertumore werden zu 80 - 100 % über die Leberarterie mit Blut versorgt. Hingegen wird das normale Leberparenchym hauptsächlich (ca. 75 %) über die Pfortader (portal) versorgt. Demzufolge kann durch Embolisation der Leberarterien eine selektive Behandlung von primärem und metastatischem Leberkrebs erreicht werden.

Das Hepatozelluläre Karzinom (HCC) ist in Europa und USA ein eher selten auftretendes Ereignis, gilt aber in Asien (Japan, Südkorea) und Afrika als die häufigste bösartige Tumorerkrankung überhaupt, die in der Mehrzahl der Fälle mit Leberzirrhose, hervorgerufen von Hepatitis B und C, einhergeht. [Therapie Konzepte Onkologie, S. Seber, J. Schütte (Hrsg.) Springer, 536-545, (1995)]. Trotz umfangreicher Anstrengungen ist bislang keine bedeutende Verbesserung der sehr schlechten Prognose bei dieser Erkrankung erzielt worden. Sowohl unbehandelt als auch nach systemischer Behandlung mit Cytostatika (vor allem 5-Fluoruracil, Mitomycin C, Cisplatin, Doxorubicin) beträgt die mittlere Überlebenszeit 1 - 9 Monate nach Diagnosestellung [K. Okuda et.al. Natural History of HCC and Prognosis in Relation to Treatment. Study of 850 Patients. Cancer 56, 918-928, (1985)]. Allein durch die chirurgische Entfernung des Tumors, die allerdings nur bei ca. 20 % der Patienten möglich ist, kann eine deutliche Lebensverlängerung, aber nur in den wenigsten Fällen eine wirkliche Heilung erreicht werden.

Anstrebenswertes Ziel neuer Therapieansätze ist vor allem eine Verbesserung der Lebensqualität der Patienten, da eine völlige Heilung wegen der zugrunde liegenden Primärerkrankung Hepatitis B nach heutigem Kenntnisstand kaum bei einer Mehrzahl der Patienten zu ereichen ist. Die operative Entfernung des Tumors stellt ebenso wie die systemische Chemotherapie eine hohe Belastung dar.

Als Methode der Wahl hat sich in den letzten Jahren die Chemoembolisation herausgebildet. Hierunter versteht man die gleichzeitige Verabreichung eines Cytostatikums gemischt mit einem Embolisationsmittel mit dem Ziel der Ausbildung eines lokalen, vorübergehenden Embolus, aus dem der Arzneistoff langsam und vor allem über einen langen Zeitraum (optimal 5 - 8 Tage) freigesetzt wird. Infolge der Einschränkung des Blutflusses kommt es zu einer erhöhten Arzneistoffexposition des Tumorgewebes [P.H. Madoule et. al. Chemoembolization: principles and perspectives, J. Microencapsulation 1, 21-25, (1984)]. Unterstützend bei der Bekämpfung des Tumors wirkt die sich lokal entwickelnde Ischämie.

Bei der in Japan und Südkorea am häufigsten angewandten Methode der Chemoembolisation verwendet man eine Emulsion aus Lipiodol ® (Ethylester von iodiertem Mohnöl) und wässrigen Cytostatika-Lösungen als peripheres Embolisat und als Depot. Da es keine entsprechenden Fertigpräparate auf dem Markt gibt, werden die Emulsionen nach "hausgemachten" Rezepten in den Kliniken vor Ort hergestellt. Dies bedingt, daß die Qualität der Präparationen sehr stark von Klinik zu Klinik wechselt und daß keine genauen und reproduzierbaren Daten zu den wichtigsten Parametern wie Partikelgröße, Verweildauer im Tumor und Ausscheidbarkeit vorliegen. Die Emulsion wird durch einen percutanen Katheter selektiv/superselektiv in die tumortragende Verzweigung der Leberarterie eingebracht. In der Regel wird die arterielle Versorgung anschließend durch Gelfoam ®-Partikel zusätzlich unterbunden, um ein zu schnelles Auswaschen der Lipiodolemulsion zu verhindern. Lipiodol ® reichert sich zu einem gewissen Grad in HCC an (T. Konno et. al. Selective Targeting of Anticancer Drug and Simultaneous Image Enhancement in Solid Tumors by Arterial Administered Lipid Contrast Medium. Cancer 54, 2367-2374, 1984) und wird nur zu einem kleineren Teil in gesundem Leberparenchym gefunden. Problematisch bei diesem Verfahren ist, daß ein nicht quantifizierbarer Anteil das Kapillarbett passiert und sich dann in Lunge oder Milz anreichert. Der Embolus bleibt über längere Zeit (1-4 Wochen) bestehen, und dient, neben der längeren Verweilzeit des Cytostatikums im Tumor, einer ischämischen Belastung des Tumors. Wegen mangelnder Bioabbaubarkeit wird Lipiodol ® praktisch nicht ausgeschieden und verbleibt in dem nekrotisierten Tumorgewebe, das dadurch nur ungenügend resorbiert werden kann. In der Regel wird diese Behandlung im Abstand von mehreren Wochen regelmäßig wiederholt. Die Überlebensraten liegen bei dieser Methode etwas unter der der operativen Entfernung des Tumors, aber deutlich über der von reiner Chemotherapie (T. Kanematsu, A 5-Year Experience of Lipiodolization: Selective Regional Chemotherapy for 200 Patients with HCC, Hepatology, 10, 98-102, 1989. D. Vetter et. al. Transcatheter Oily Chemoembolization in the Management of Advanced HCC in Cirrhosis: Results of a Western Comparative Study in 60 Patients, Hepatology, 13, 427-433, 1991).

Trotz der beschriebenen Probleme hat sich das Lipiodol ® -Verfahren im Vergleich zu anderen Embolisationstechniken mit mehr oder weniger bioabbaubaren Partikelsuspensionen (s. Tabelle 1) als das bislang am weitesten verbreitete Therapiekonzept im asiatischen Raum durchgesetzt.

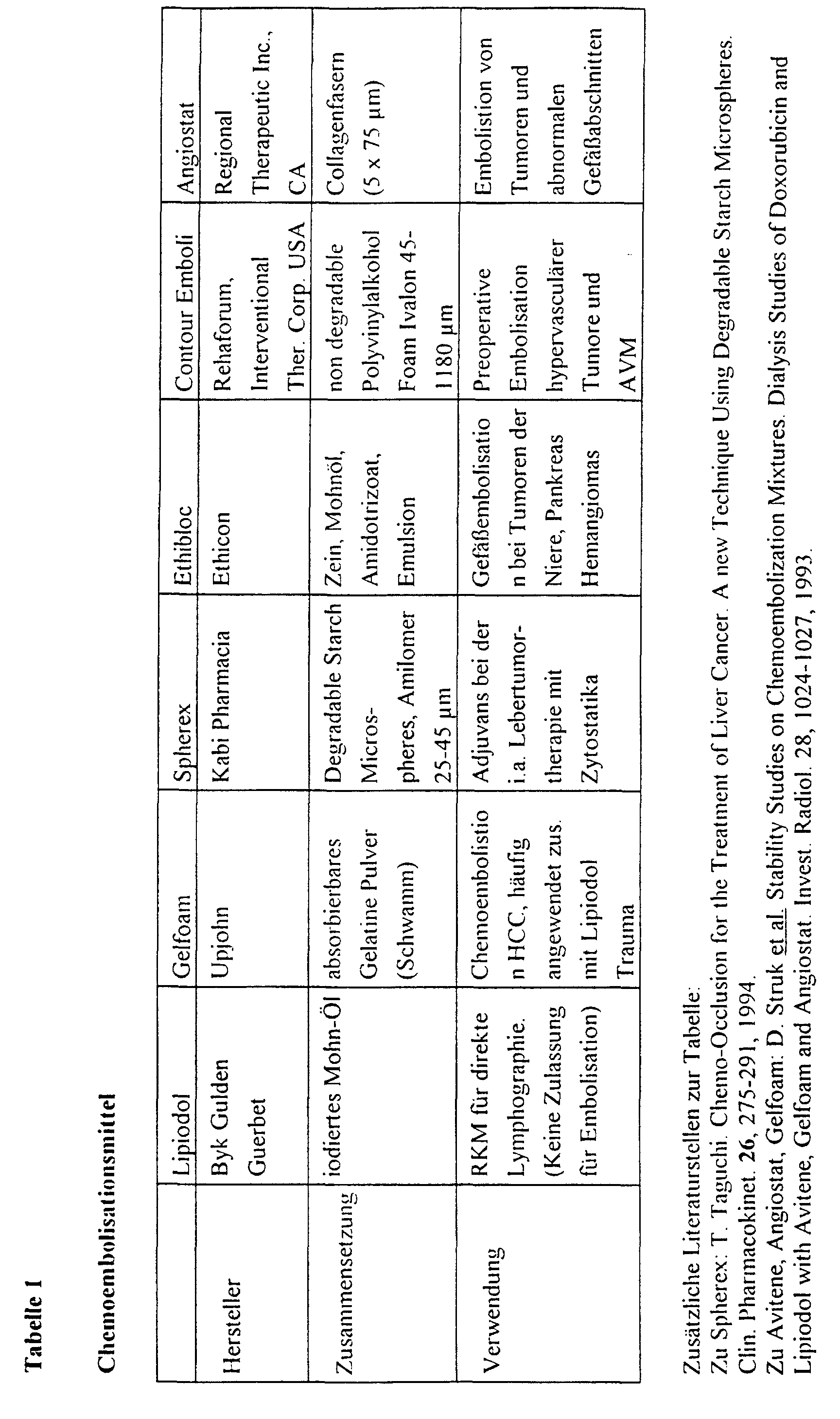

Die erfindungsgemäßen perfluoralkylhaltigen Metallkomplexe zeigen in wässriger Lösung ungewöhnliche physikochemische Eigenschaften. Sie sind in erstaunlich hohem Maße thixotrop, so daß diese Verbindungen als Embolisat sehr geeignet sind. Diese Lösungen haben gelartige Konsistenz in der Ruhelage, bei Einwirkung von Scherkräften jedoch werden sie fließfähig (förderbar durch Pumpen und durch lange Katheter).

Die hohe Viskosiät der für die erfindungsgemäße Vewendung geeigneten Verbindungen führt bei den geringen Scherkräften, wie sie im Kapillarbett von Geweben vorherrschen, zu einem verläßlichen, vorübergehenden Verschluß dieser Gefäße. Gleichzeitig verfügen diese Verbindungen über ausreichend gute Fließeigenschaften unter Druck, wie sie für die Applikation durch lange Katheter notwendig sind.

Die erfindungsgemäßen Verbindungen bieten die Möglichkeit hochwirksame Cytostatika (z.B. 5-Fluoruracil, Mitomycin C, Cisplatin, Doxorubicin) zu formulieren. Auf diese Weise wird der Wirkstoff nur lokal in hoher Konzentration in den Körper eingebracht. Die systemische Belastung mit den bekannten Nebenwirkungen bleibt dadurch gering.

Der gebildete Embolus ist nicht dauerhaft, sondern kann sich langsam auflösen. Die Bestandteile werden mit dem Blut abtransportiert und über die Niere ausgeschieden. Dies ist günstig, da das in die Formulierung eingearbeitete Cytostatikum auf diese Weise wie aus einem Depot über eine längere Zeit in direkter Nähe zum Tumor freigesetzt wird und weil nach einer Auflösung des Embolus weitere Applikationen möglich sind.

Enthalten die erfindungsgemäßen Verbindungen paramagnetische oder röntgendichte Ionen kann der Embolisationsvorgang und der Therapieerfolg diagnostisch durch NMR oder Röntgen (CT) - Diagnostik verfolgt werden (interventionelle Radiologie).

Es ist aber auch möglich Kombinationen der erfindungsgemäßen Verbindungen mit anderen Kontrastmitteln wie sie in der NMR- und -Röntgendiagnostik gebräuchlich sind (z.B. Magnevist®, Isovist®, Iopamidol®, Ultravist® etc.) zu verwenden.

Mit den erfindungsgemäßen Mitteln werden die bei den vorbekannten Mitteln beschriebenen Nebenwirkungen, wie vor allem Mikroembolien (z.B. in der Lunge), vermieden.

Die für die erfindungsgemäße Verwendung geeigneten perfluoralkylhaltigen Verbindungen werden durch die allgemeine Formel I gemäß Patentanspruch 1 beschrieben.

Die Verbindungen der allgemeinen Formel I beinhalten als bevorzugte Reste L die folgenden:
-CH₂-
-CH₂CH₂-
-(CH₂)ₛ- s = 3 - 15
-CH₂-O-CH₂CH₂-
-CH₂-(O-CH₂-CH₂-)ₜ t = 2 - 6
-CH₂-NH-CO-
-CH₂-NH-CO-CH₂-N(CH₂COOH)-SO₂-
-CH₂-NH-CO-CH₂-N(C₂H₅)-SO₂₋
-CH₂-NH-CO-CH₂-N(C₁₀H₂₁)-SO₂-
-CH₂-NH-CO-CH₂-N(C₆H₁₃)-SO₂-
-CH₂-NH-CO-(CH₂)₁₀-N(C₂H₅)-SO₂-
-CH₂-NH-CO-CH₂-N(-CH₂-C₆H₅)-SO₂-
-CH₂-NH-CO-CH₂-N(-CH₂-CH₂-OH)SO₂-
-CH₂-NHCO-(CH₂)₁₀-S-CH₂CH₂-
-CH₂NHCOCH₂-O-CH₂CH₂-
-CH₂NHCO(CH₂)₁₀-O-CH₂CH₂-
-CH₂-C₆H₄-O-CH₂CH₂-
-CH₂-O-CH₂-C(CH₂-OCH₂CH₂-C₆F₁₃)₂-CH₂-OCH₂-CH₂- 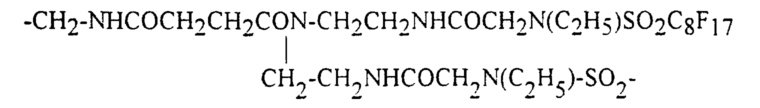
-CH₂-O-CH₂-CH(OC₁₀H₂₁)-CH₂-O-CH₂CH₂-
-(CH₂NHCO)₄-CH₂O-CH₂CH₂-
-(CH₂NHCO)₃-CH₂O-CH₂CH₂-
-CH₂-OCH₂C(CH₂OH)₂-CH₂-O-CH₂CH₂- 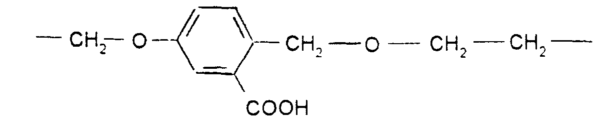
-CH₂NHCOCH₂N(C₆H₅)-SO₂-
-NHCO-CH₂-CH₂-
-NHCO-CH₂-O-CH₂CH₂-
-NH-CO-
-NH-CO-CH₂-N(CH₂COOH)-SO₂-
-NH-CO-CH₂-N(C₂H₅)-SO₂-
-NH-CO-CH₂-N(C₁₀H₂₁)-SO₂-
-NH-CO-CH₂-N(C₆H₁₃)-SO₂-
-NH-CO-(CH₂)₁₀-N(C₂H₅)-SO₂-
-NH-CO-CH₂-N(-CH₂-C₆H₅)-SO₂-
-NH-CO-CH₂-N(-CH₂-CH₂-OH)SO₂-
-NH-CO-CH₂-
-CH₂-O-C₆H₄-O-CH₂-CH₂-
-CH₂-C₆H₄-O-CH₂-CH₂-
-N(C₂H₅)-SO₂-
-N(C₆H₅)-SO₂-
-N(C₁₀H₂₁)-SO₂-
-N(C₆H₁₃)-SO₂-
-N(C₂H₄OH)-SO₂-
-N(CH₂COOH)-SO₂-
-N(CH₂C₆H₅)-SO₂-
-N-[CH(CH₂OH)₂]-SO₂-
-N-[CH(CH₂OH)CH(CH₂OH)]-SO₂-

Erfindungsgemäß ganz besonders bevorzugt sind die Reste L der in den Beispielen der vorliegenden Erfindungsbeschreibung genannten Verbindungen.

Weitere bevorzugte Verbindungen sind solche, in denen X aus der Formel -CₙF₂ₙX Fluor bedeutet, und n für die Zahlen 4 bis 15 steht.

Verbindungen der allgemeinen Formel I mit A in der Bedeutung der allgemeinen Formel IX, wobei L mindestens eine -NHCO-Gruppe enthält, kann man aus Verbindungen der allgemeinen Formel 14 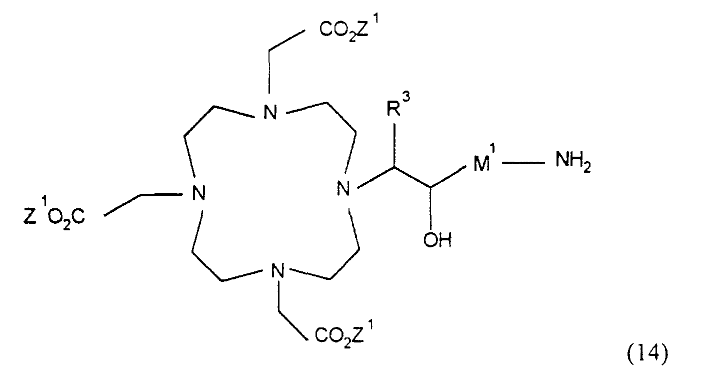 worin
- R³: in der oben genannten Bedeutung steht, Z¹, in der Bedeutung eines Metallionenequivalents der Ordnungszahlen 12, 20-30, 39, 42, 44 oder 57-83, steht und
- M¹: in der Bedeutung von L steht,
durch Umsetzung mit Verbindungen der allgemeinen Formel 15 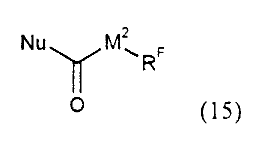 worin
- R^{F}: die oben genannte Bedeutung hat,
- M²: in der Bedeutung von L steht und
- Nu: in der Bedeutung eines Nucleofugs steht,
erhalten.

Als Nucleofug dienen vorteilhafterweise die Reste:

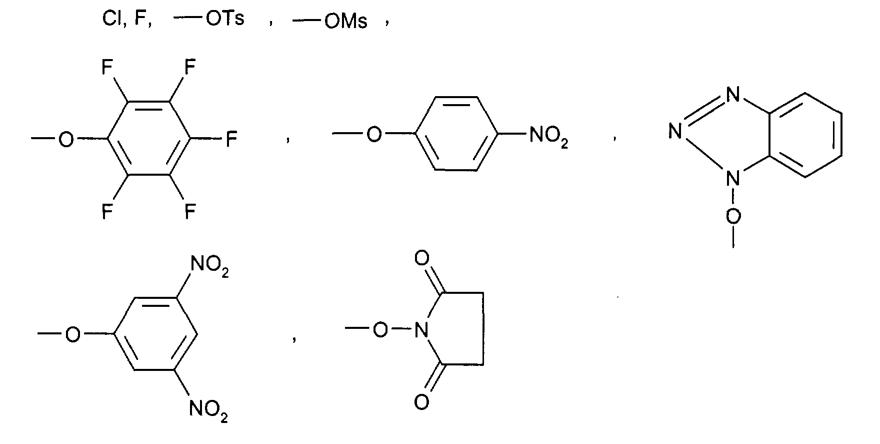

Die Umsetzung wird im Gemisch von Wasser und organischen Lösungsmitteln wie: Isopropanol, Ethanol, Methanol, Butanol, Dioxan, Tetrahydrofuran, Dimethylformamid, Dimethylacetamid, Formamid oder Dichlormethan durchgeführt. Bevorzugt sind temäre Gemische aus Wasser, Isopropanol und Dichlormethan.

Die Umsetzung wird in einem Temperaturintervall zwischen -10 °C - 100 °C, vorzugsweise zwischen 0 °C - 30 °C durchgeführt.

Als Säurefänger dienen anorganische und organische Basen wie Triethylamin, Pyridin, N-Methylmorpholin, Diisopropylethylamin, Dimethylaminopyridin, Alkali und Erdalkalihydroxyde, ihre Carbonate oder Hydrogencarbonate wie Lithiumhydroxid, Natriumhydroxid, Kaliumhydroxid, Natriumcarbonat, Natriumhydrogencarbonat, Kaliumhydrogencarbonat.

Die Verbindungen der allgemeinen Formel 15 werden aus Verbindungen der allgemeinen Formel 16

HO₂C-M²-R^{F} (16)

in der
R^{F}, M² die oben genannte Bedeutung haben, nach den dem Fachmann allgemein bekannten Verfahren der Säureaktivierung wie durch Umsetzung der Säure mit Dicyclohexylcarbodiimid, N-Hydroxysuccinimid/Dicyclohexylcarbodiimid, Carbonyldiimidazol, 2-Ethoxy-1-ethoxycarbonyl-1,2-dihydrochinolin, Oxalsäuredichlorid oder Chlorameisensäurisobutylester nach den in der in der Literatur beschriebenen Verfahren erhalten:
◆ Aktivierung von Carbonsäuren. Übersicht in Houben-Weyl, Methoden der Organischen Chemie, Band XV/2, Georg Thieme Verlag Stuttgart, 19.
◆ Aktivierung mit Carbodiimiden. R. Schwyzer u. H. Kappeler, Helv. 46: 1550 (1963).
◆ E. Wünsch et al., B. 100: 173 (1967).
◆ Aktivierung mit Carbodiimiden/Hydroxysuccinimid: J. Am. Chem. Soc. 86: 1839 (1964) sowie J. Org. Chem. 53: 3583 (1988). Synthesis 453 (1972).
◆ Anhydridmethode, 2-Ethoxy-1-ethoxycarbonyl-1,2-dihydrochinolin: B. Belleau et al., J. Am. Chem. Soc., 90: 1651 (1986), H. Kunz et al., Int. J. Pept. Prot. Res., 26: 493 (1985) und J. R. Voughn, Am. Soc. 73: 3547 (1951).
◆ Imidazolid-Methode: B.F. Gisin, R.B. Merrifield, D.C. Tosteon, Am. Soc. 91: 2691 (1969).
◆ Säurechlorid-Methoden, Thionylchlorid: Helv., 42: 1653 (1959).
◆ Oxalylchlorid: J. Org. Chem., 29: 843 (1964).

Die Verbindungen der allgemeinen Formel 16 sind Kaufware (Fluorochem, ABCR) oder werden aus Verbindungen der allgemeinen Formel 17

H-Q-M³-R^{F} (17)

mit
- M3: in der Bedeutung von L und
- Q: in der Bedeutung von Sauerstoff, Schwefel, einer >CO-Gruppe, >N-R³, 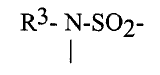 mit einer Bindung vom Stickstoffatom zum Wasserstoffatom,
durch Umsetzen mit Verbindungen der allgemeinen Formel 18 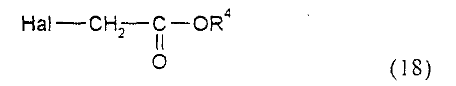 mit
- Hal: in der Bedeutung, Cl, Br, l und
- R⁴: in der Bedeutung von H, Methyl, Ethyl, t-Butyl, Benzyl, Isopropyl, dargestellt beispielsweise nach C.F. Ward, Soc. 121, 1161 (1922),
nach den dem Fachmann bekannten Methoden wie Alkylierung von Alkoholen mit Alkylhalogeniden [Houben-Weyl, Methoden der Organischen Chemie, Sauerstoffverbindungen I, Teil 3, Methoden zur Herstellung und Umwandlung von Ethern, Georg Thieme Verlag, Stuttgart 1965, Alkylierung von Alkoholen mit Alkylhalogeniden S. 24, Alkylierung von Alkoholen mit Alkylsulfaten S. 33] oder N-Alkylierung eines Sulfonamids mit Alkylsulfonaten [Houben-Weyl, Methoden der organischen Chemie, XI/2 Stickstoffverbindungen, Georg Thieme Verlag Stuttgart, 1957, S. 680; J.E. Rickman and T. Atkins, Am. Chem. Soc., 96: 2268, 1974, 96: 2268; F. Chavez and A.D. Sherry, J. Org. Chem. 1989, 54: 2990]
erhalten.

Für den Fall daß Q eine >CO-Gruppe bedeutet, wird die Umsetzung mit einem Wittig-Reagenz der Struktur 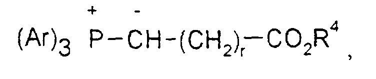 wobei r die Zahlen 0 - 16 bedeutet, vorgenommen.
Die dabei entstandene -CH=CH-Doppelbindung kann als Bestandteil der Struktur erhalten bleiben oder durch katalytische Hydrierung (Pd 5 %/C) in eine -CH₂-CH₂-Gruppierung überführt werden.

Die Verbindungen der allgemeinen Formel 18 sind Kaufware (Fluorochem, ABCR).

Alternativ können Verbindungen der allgemeinen Formel I mit A in der Bedeutung der allgemeinen Formel IX aus Verbindungen der allgemeinen Formel 19 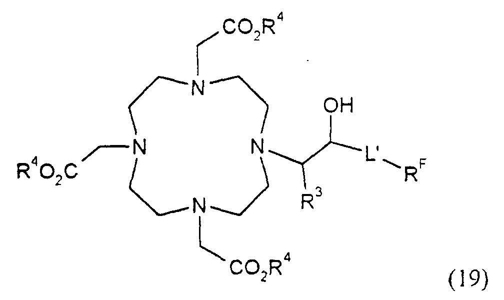 mit
- R^{F}, R³ und R⁴: in der oben genannten Bedeutung und
- L': in der Bedeutung von L, gegebenenfalls mit geschützten Hydroxyl- oder Carboxylfunktionen,
erhalten werden, indem man, falls erforderlich, vorhandene Schutzgruppen abspaltet und die so erhaltenen Komplexbildner mit den nach dem Fachmann bekannten Methoden (EP 250358, EP 255471) mit Metalloxiden oder Metallsalzen bei Raumtemperatur oder erhöhter Temperatur umsetzt, und anschließend - falls gewünscht - vorhandene acide Wasserstoffatome durch Kationen anorganischer und/oder organischer Basen, Aminosäuren oder Aminosäureamide substituiert.

Die Verbindungen der allgemeinen Formel 19 werden erhalten aus Verbindungen der allgemeinen Formel 20 (D03A bzw. der Ester) 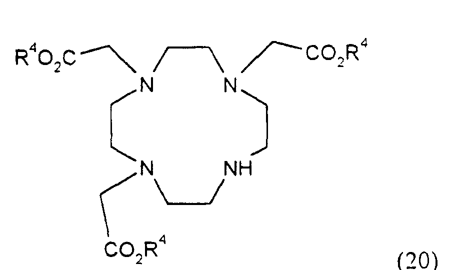 mit
- R⁴: in der oben genannten Bedeutung
durch Umsetzen mit Verbindungen der allgemeinen Formel 21 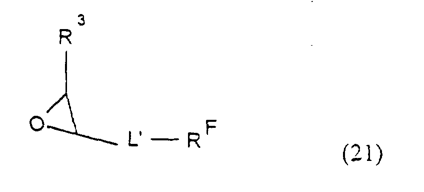 worin
R³ die Bedeutung von R¹, gegebenenfalls in geschützter Form, oder -(CH₂)ₘ-L'-R^{F} hat, wobei m 0, 1 oder 2 ist und L' und R^{F} die oben genannte Bedeutung haben. Die Umsetzung wird in Alkoholen wie Methanol, Ethanol, Isopropanol, Butanol, Ethern wie Dioxan, Tetrahydrofuran, Dimethoxyethern oder in Wasser oder in Mischungen aus Wasser und einem der genannten organischen Lösungsmittel, sowie auch Acetonitril, Aceton, Dimethylformamid, Dimethylacetamid oder Dimethylsulfoxid, Dichlormethan, Dichlorethan, Chloroform bei Temperaturen zwischen -10 °C und 180 °C, vorzugsweise bei 20 ° - 100 °C durchgeführt. Als vorteilhaft hat sich der Zusatz von organischen oder anorganischen Basen wie Triethylamin, Pyridin, Dimethylaminopyridin, N-Methylmorpholin, Diisopropylamin, Alkali- oder Endalkalihydroxiden oder ihren Carbonaten oder Hydrogencarbonaten wie Lithiumhydroxid, Natriumhydroxid, Kaliumhydroxid, Natriumcarbonat, Kaliumcarbonat, Natriumhydrogencarbonat, Kaliumhydrogencarbonat, erwiesen. Im Falle niedrigsiedender Epoxide wird die Umsetzung im Autoklaven durchgeführt.

Die Verbindungen der allgemeinen Formel 21 sind Kaufware (Fluorochem, ABCR) oder aus Verbindungen der allgemeinen Formel 22

R³-CH=CH-L'-R^{F} (22)

durch Epoxidierung nach den dem Fachmann bekannten Methoden erhältlich, beispielsweise die Wolframat-katalysierte Oxidation mit H₂O₂ nach Payne, die Cyclisierung von Halogenhydrinen oder die alkalische H₂O₂-Oxidation in Gegenwart von Nitrilen.
Besonders geeignet für diese Reaktion ist 3-Chlorperbenzoesäure in Dichlormethan bei Raumtemperatur. Houben-Weyl, Methoden der Organischen Chemie, Sauerstoffverbindungen I, Teil 3, Methoden zur Herstellung und Umwandlung dreigliedriger cyclische Ether (1,2-Epoxide), Georg Thieme Verlag, Stuttgart, 1965; G. B. Payne and P.H. Williams, J. Org. Chem., 159, 24: 54; Y. Ogata and Y. Samaki, Tetrahedron 1964, 20: 2065; K.B. Sharpless et al., Pure Appl. Chem. 55, 589 (1983).

Verbindungen der allgemeinen Formel 22 werden vorzugsweise durch Wittig-Reaktion, bzw. durch die Varianten nach Hornen Schlosser oder Bestmann, Houben-Weyl, Methoden der Organischen Chemie XII/1, Organische Phosphorverbindungen Teil 1, Georg Thieme Verlag, Stuttgart, 1963, Phosphoniumsalze S. 79, Phosphoniumylide S. 112, Wittig-Reaktion S. 121; A.W. Johnson, Ylides and Imines of Phosphorus, John Wiley & Sons, Inc., New York, Chichester, Brisbane, Toronto, Singapore, 1993, Wittig-Reaktion S. 221; Schlosser-Modifikation der Wittig-Reaktion S. 240; Wadsworth-Emmons-Reaktion S. 313; Horner Reaktion S. 362, durch Umsetzung eines Triarylphosphoniums Ylids 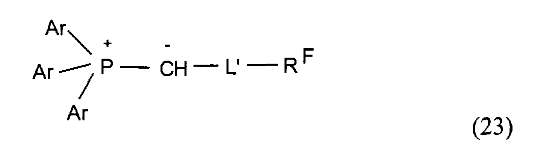 mit L' und R^{F} in der oben genannten Bedeutung und Ar in der Bedeutung Aryl, insbesondere Phenyl, mit käuflichen (Merck, Fluka) oder nach den dem Fachmann bekannten Methoden, beispielsweise der Oxidation von primären Alkoholen mit Chromtrioxid/Pyridin, Houben-Weyl, Methoden der Organischen Chemie, Sauerstoffverbindungen II, Teil 1, Aldehyde, Georg Thieme Verlag, Stuttgart, 1954, darstellbaren Aldehyden der allgemeinen Formel 20 → 24

OHC-R³ (24)

wobei
R³ auch H sein kann, erhalten.

Die Triarylphosphoniumylide 23 werden aus den entsprechenden Halogeniden der allgemeinen Formel 25

Hal-CH₂-L'-R^{F} (25)

mit Hal, L' und R^{F} in der oben genannten Bedeutung nach den dem Fachmann bekannten Methoden, beispielsweise durch Erwärmen des Triarylphosphins mit dem Alkylhalogenid, Houben-Weyl, Methoden der Organischen Chemie XII/1, Organische Phosphorverbindungen Teil 1, Georg Thieme Verlag, Stuttgart, 1963, oder A.W. Johnson, Yldes and Imines of Phosphorus, John Wiley & Sons, Inc., New York, Chichester, Brisbane, Toronto, Singapore, 1993, dargestellt. Die Verbindungen der allgemeinen Formel 25 sind Kaufware (Fluorochem, ABCR, 3M).

Die Verbindungen der allgemeinen Formel 21 mit R³ = H werden bevorzugt aus Verbindungen der allgemeinen Formel 17a

H-Q'-M³-R^{F} (17a)

worin
- Q': in der Bedeutung von Q steht, aber keine >CO-Gruppe bedeuten kann,
- M³: die Bedeutung von L mit Ausnahme der direkten Bindung hat und
- R^{F}: die o. g. Bedeutung hat,
durch Umsetzen nach der dem Fachmann bekannten Weise der Veretherung oder Sulfonamidalkylierung mit Epihalogenhydrinen: (Houben-Weyl, Methoden der Organischen Chemie, Sauerstoffverbindungen I, Teil 3, Methoden zur Herstellung und Umwandlung von Ethern, Georg Thieme Verlag, Stuttgart, 1965, Alkylierung von Alkoholen, S. 24, 33; Houben-Weyl, Methoden der Organischen Chemie, XI/2 Stickstoffverbindungen, Georg Thieme Verlag, Stuttgart, 1957, S. 680; J.E. Rickman and T.J.J. Atkins, Am. Chem. Soc. 1974, 96: 2268; F. Chavez and A.D. Sherry, 1989, 54: 2990) der allgemeinen Formel 26 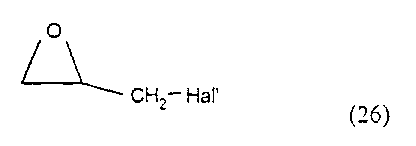 mit
- Hal': in der Bedeutung Hal, F, -OTs, OMs erhalten.

Im Falle niedrigsiedender Epoxide wird die Umsetzung im Autoklaven durchgeführt.

Verbindungen der allgemeinen Formel I mit A in der Bedeutung der allgemeinen Formel VIII erhält man aus Verbindungen der allgemeinen
Formel 27 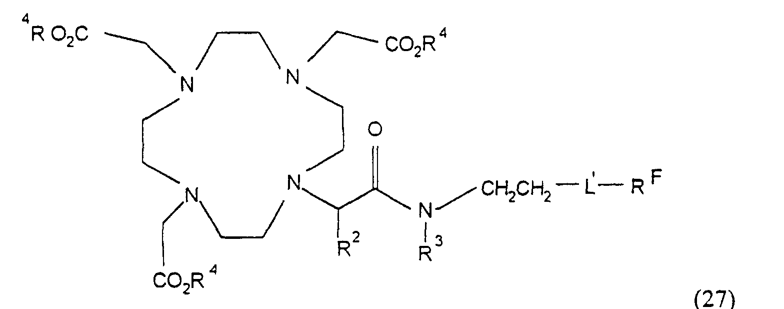 mit R², R³, R⁴, L' und R^{F} in der oben genannten Bedeutung
durch Abspalten gegebenenfalls vorhandener Schutzgruppen und Komplexieren in der dem Fachmann bekannten Art.

Verbindungen der allgemeinen Formel 27 erhält man durch Alkylierung der Verbindungen der allgemeinen Formel 20 mit Verbindungen der allgemeinen Formel 28 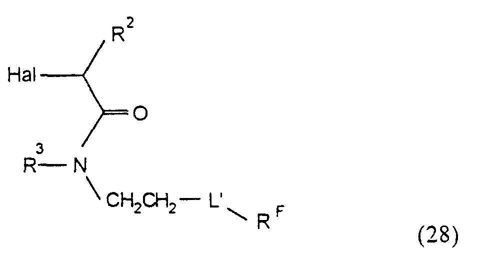 in der Hal, R², R³ , L' und R^{F} die o. g. Bedeutung haben,
in an sich bekannter Weise, beispielsweise wie unter EP 0 232 751 B1 (Squibb), beschrieben.

Verbindungen der allgemeinen Formel 28 werden aus Verbindungen der allgemeinen Formel 29 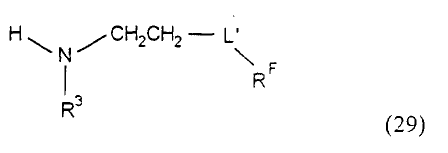 mit L', R³ und R^{F} in der oben genannten Bedeutung und einer aktivierten Halogencarbonsäure der allgemeinen Formel 30 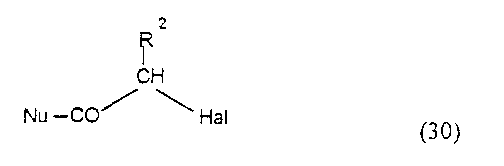 mit Nu, R² und Hal in der oben genannten Bedeutung
nach den dem Fachmann bekannten Methoden der Amidbildung über aktivierte Carbonsäuren [vgl. Lit. S. 11] dargestellt.

Verbindungen der allgemeinen Formel 30 sind aus den Säuren erhältlich nach C. Hell. B. 14: 891 (1881); J. Volhard, A 242, 141 (1887); N. Zelinsky, B. 20: 2026, (1887) oder aus den Halogensäuren nach den Aktivierungsmethoden wie sie bei der allgemeinen Formel 15 beschrieben werden.
Die Verbindungen der allgemeinen Formel 29 lassen sich nach den dem Fachmann bekannten Methoden der Aminsynthese [Houben-Weyl, Methoden der Organischen Chemie, Stickstoffverbindungen II, Amino, 1. Herstellung, Georg Thieme Verlag, Stuttgart, 1957] aus den käuflichen Verbindungen (Fluorochem, ABCR) der allgemeinen Formel 31

Hal-CH₂CH₂-L'-R^{F} (31)

oder 32

HO-CH₂CH₂-L'-R^{F} (32)

leicht darstellen, beispielsweise durch Alkylierung einer Verbindung 31 mit einem Amin PhCH₂NHR³ und anschließender Deprotektion der Aminogruppe durch katalytische Hydrierung oder durch Mitsunobu-Reaktion [H. Loibner und E. Zbiral, Helv. 59, 2100 (1976), A.K. Bose und B. Lal, Tetrahedron Lett. 3973 (1973)] einer Verbindung 32 mit Kaliumphthalimid und Deprotektion mit Hydrazinhydrat.

Verbindungen der allgemeinen Formel I mit A in der Bedeutung der allgemeinen Formel VII erhält man aus Verbindungen der allgemeinen Formel 33 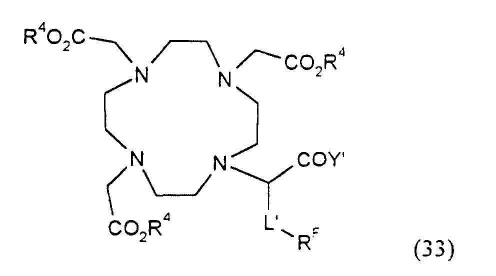 mit
L', R^{F} und R⁴ in der oben genannten Bedeutung und
Y' in der Bedeutung Y, gegebenenfalls mit Schutzgruppen, durch Abspalten gegebenenfalls vorhandener Schutzgruppen und Komplexieren nach den dem Fachmann bekannten Methoden (Protective Groups in Organic Synthesis, 2nd Edition, T.W. Greene and P.G.M. Wuts, John Wiley & Sons, Inc., New York, 1991; EP 0 130 934, EP 0 250 358).

Verbindungen der allgemeinen Formel 33 erhält man aus Verbindungen der allgemeinen Formel 20 und Verbindungen der allgemeinen Formel 34 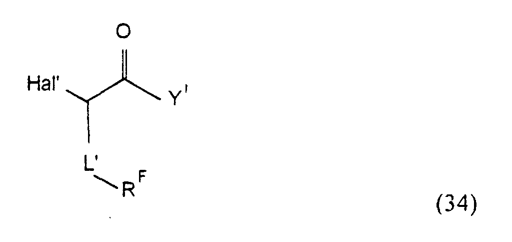 worin
Hal', L', R^{F} die oben genannte Bedeutung haben und Y' für die Reste
―OH, 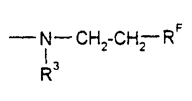 steht,
in an sich bekannter Weise, beispielsweise wie in EP 0 232 751 B1,
EP 0 292 689 A2 (beide Squibb) oder EP 0 255 471 A1 (Schering) beschrieben.

Die Darstellung von Verbindungen der allgemeinen Formel 34 erfolgt nach bekannten Methoden, beispielsweise nach Hell-Volhard-Zelinsky aus käuflichen Vorstufen (ABCR).

Verbindungen der allgemeinen Formel I mit A in der Bedeutung der allgemeinen Formel VI erhält man aus Verbindungen der allgemeinen
Formel 35 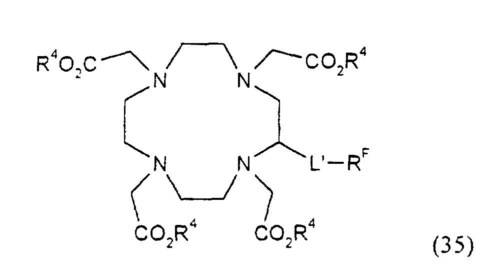 worin L', R⁴ und R^{F} die oben genannte Bedeutung haben durch gegebenenfalls Abspalten von Schutzgruppen und Komplexieren in an sich bekannter Weise [Protective Groups in Organic Synthesis, 2nd Edition, T.W. Greene and P.G.M. Wuts, John Wiley & Sons, Inc., New York, 1991 (EP 0 130 934, EP 0 250 358)].

Verbindungen der allgemeinen Formel 35 erhält man durch Umsetzen von α-Halogencarbonsäureestern oder -säuren der allgemeinen Formel 18 mit Verbindungen der allgemeinen Formel 36 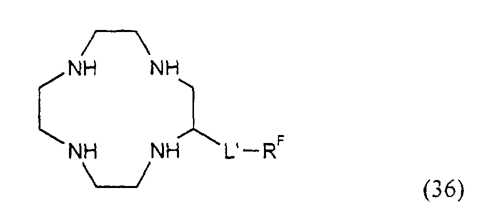 mit L' und R^{F} in der oben genannten Bedeutung, nach den dem Fachmann bekannten Methoden, wie beispielsweise in EP 0 255 471 oder US 4,885,363 beschrieben.

Verbindungen der allgemeinen Formel 36 sind durch Abspalten gegebenenfalls vorhandener Schutzgruppen und anschließender Reduktion mit Diboran nach den bekannten Verfahren aus Verbindungen der allgemeinen Formel 37 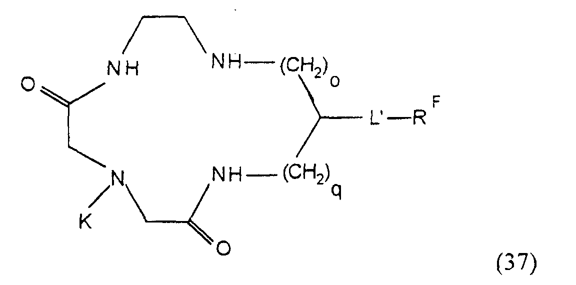 worin
L', R^{F}, o, q, die oben genannte Bedeutung haben und
K die Bedeutung einer Schutzgruppe hat,
erhältlich.

Die Verbindungen der allgemeinen Formel 37 sind durch eine Kondensationsreaktion aus einer aktivierten, N-geschützten Iminodiessigsäure 38 und dem Amin 39 zugänglich: 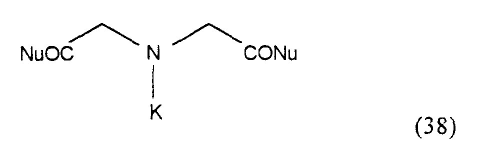 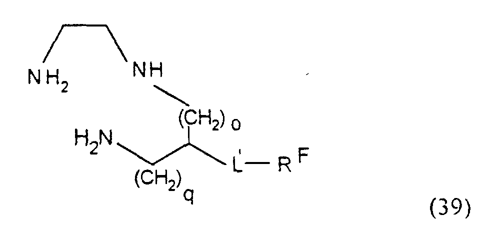 worin
L', R^{F}, o, q, Nu und K die oben genannte Bedeutung haben. Als Nucleofug dient bevorzugt das N-Hydroxysuccinimid, als Schutzgruppe die Benzyloxycarbonyl-, Trifluoracetyl oder t-Butyloxycarbonylgruppe.

Verbindungen der allgemeinen Formel 38 sind nach den dem Fachmann bekannten Verfahren des Schutzes der Aminogruppe und der Aktivierung der Carbonsäure [Protective Groups, Aktivierung von Carboxylgruppen, S. 11] über die geschützte Iminodiessigsäure 40 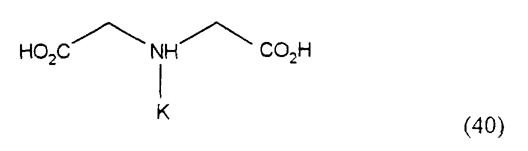 worin
K die Bedeutung einer Schutzgruppe hat aus Iminodiessigsäure 41 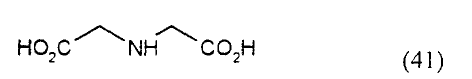 erhältlich.

Alternativ sind Verbindungen der allgemeinen Formel 36 durch gegebenenfalls Abspalten von Schutzgruppen und Reduktion mit Diboran nach dem bei 37 beschriebenen Verfahren aus Verbindungen der allgemeinen Formel 42 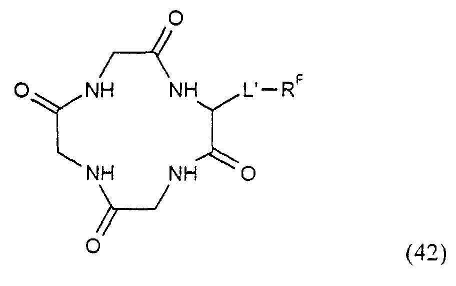 zugänglich.

Verbindungen der allgemeinen Formel 42 sind durch Ringschluß der Secco-Verbindungen 43 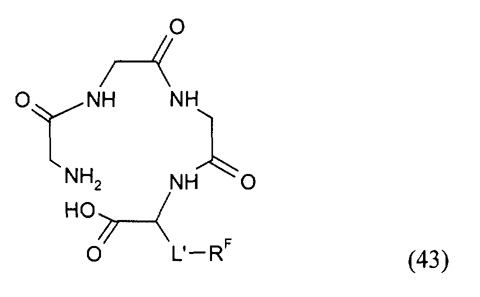 worin
L' und R^{F} die oben genannte Bedeutung haben, nach Standardverfahren erhältlich; beispielsweise durch Umsetzen mit dem Mukaiyama-Reagenz 2-Fluor-1-methylpyridinium-tosylat 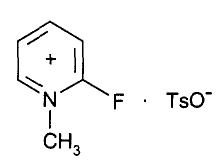 [J. Org. Chem. 1994, 59, 415; Synthetic Commununications 1995, 25, 1401] oder mit dem Phosphorsäurediphenylester-azid 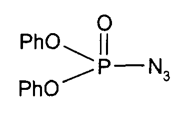 [J. Am. Chem. Soc. 1993, 115, 3420; WO 94/15925].

Verbindungen der allgemeinen Formel 43 sind durch Kondensation der aktivierten Säure 44 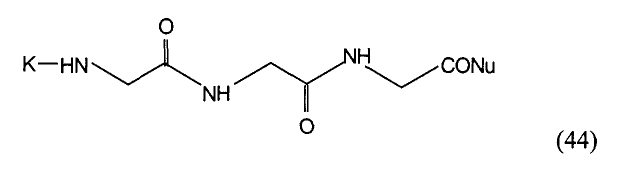 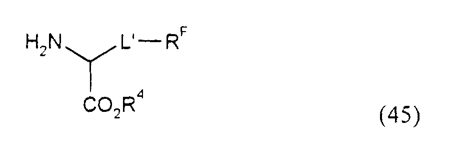 worin
L', R⁴ und R^{F} die oben genannte Bedeutung haben,
nach den beschriebenen Verfahren zugänglich.

Verbindungen der allgemeinen Formel 44 sind aus dem käuflichen Triglycin (Bachem, Fluka) 46 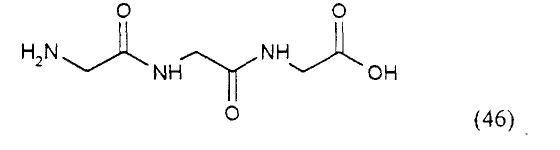 durch Schutz der Aminogruppe mit nachfolgender Aktivierung der Säurefunktion nach den dem Fachmann bekannten Verfahren für Aminschutz und Carbonsäureaktivierung (Lit. s. nach Formel 16) zugänglich.

Die Verbindungen der allgemeinen Formel 45 sind aus Verbindungen der allgemeinen Formel 62 durch Einführen der Schutzgruppe R⁴ nach den dem Fachmann bekannten Methoden - beispielsweise Umesierung eines Sulfitesters - leicht erhältlich.

Verbindungen der allgemeinen Formel I mit A in der Bedeutung der allgemeinen Formel II erhält man aus Verbindungen der allgemeinen Formel 47 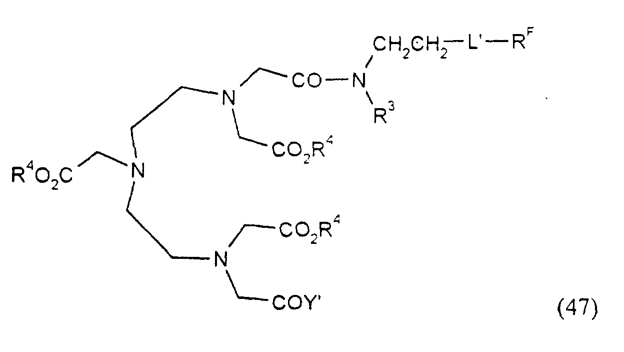 mit L', R³, R⁴, R^{F} und Y' in der oben genannten Bedeutung, durch gegebenenfalls Abspalten von Schutzgruppen und Komplexieren in einer dem Fachmann wohlbekannten Weise (Protective Groups, EP 0 250 358,
EP 0 130 934).

Für den Fall, daß Y' in der allgemeinen Formel 47 eine OH-Gruppe bedeutet, erhält man die Verbindungen durch Umsetzen einer Verbindung 48 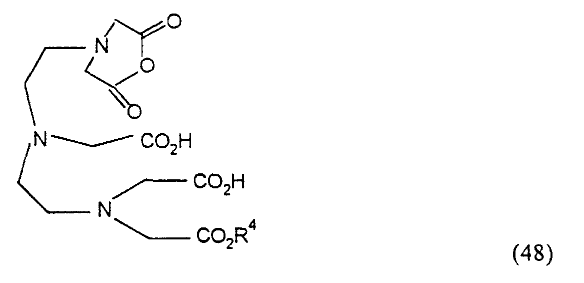 mit R⁴ in der oben genannten Bedeutung, dargestellt nach DE 3 633 243, mit einem Amin der allgemeinen Formel 29 unter den bereits beschriebenen Bedingungen und anschließender Abspaltung der Schutzgruppen.

Ist Y' in Formel 47 jedoch die Gruppe 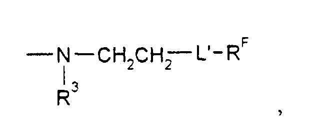 dann wird die Umsetzung mit dem DTPA-Bisanhydrid (Kaufware, Merck) 49 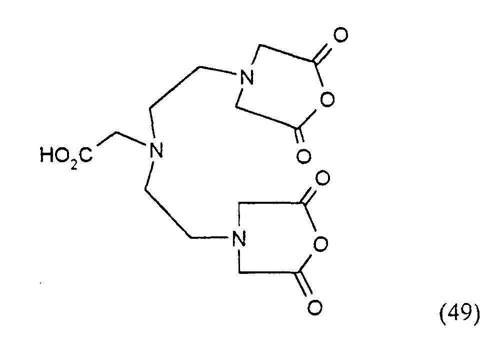 unter analogen Bedingungen vorgenommen.

Verbindungen der allgemeinen Formel I, mit A in der Bedeutung der allgemeinen Formel III erhält man aus Verbindungen der allgemeinen Formel 50 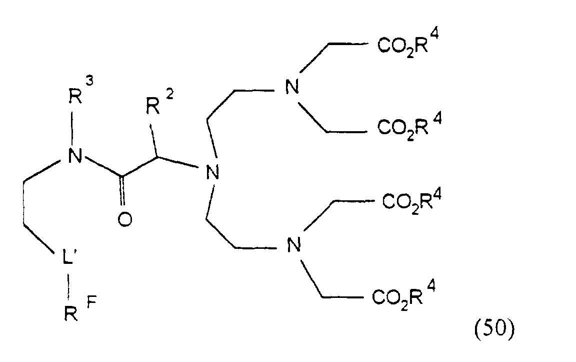 worin
L', R², R³, R⁴ und R^{F} die oben genannte Bedeutung haben, durch gegebenenfalls Abspalten von Schutzgruppen und Komplexieren in einer dem Fachmann wohlbekannten Weise [Protective Groups, EP 0 071564, EP 0 130 934, DE-OS 3 401 052].

Verbindungen der allgemeinen Formel 50 werden nach dem in J. Org. Chem. 1993, 58: 1151, beschriebenen Verfahren aus Verbindungen der allgemeinen Formel 51 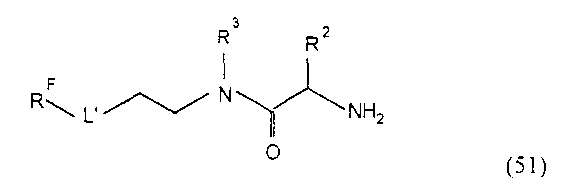 und Halogencarbonsäurederivaten der Formel 52 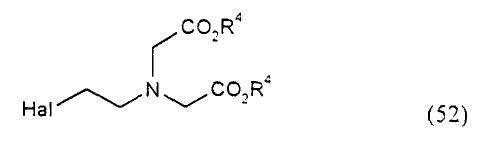 in der R⁴ und Hal die bereits beschriebene Bedeutung haben, erhalten. Die Verbindungen der allgemeinen Formel 51 sind durch Acylierung eines Amins der allgemeinen Formel 29 mit einer aktivierten N-geschützten Aminosäure der allgemeinen Formel 53 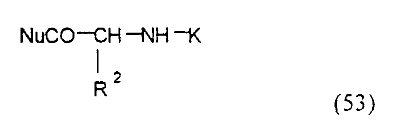 in der Nu die oben genannte Bedeutung hat und K in der Bedeutung einer Schutzgruppe wie Z, -BOC, FMOC, -COCF₃ steht, und anschließender Abspaltung der Schutzgruppe, dargestellt.

Verbindungen der allgemeinen Formel I mit A in der Bedeutung der allgemeinen Formel IV erhält man aus Verbindungen der allgemeinen
Formel 54 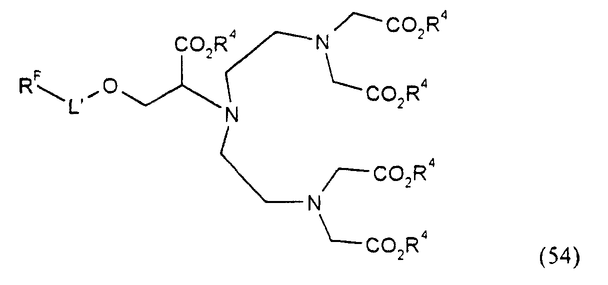 worin
L', R^{F} und R⁴ die oben genannte Bedeutung haben durch gegebenenfalls Abspalten der Schutzgruppen und Komplexieren nach einer dem Fachmann bekannten Methode, wie sie bereits beschrieben [Protective Groups,
EP 0 071 564, EP 0 130 934, DE-OS 3 401 052].

Verbindungen der allgemeinen Formel 54 lassen sich in bekannter Weise aus den Halogenverbindungen der allgemeinen Formel 55

Hal-L'-R^{F} (55)

die als Kaufware erhältlich sind (Fluorochem, ABCR), durch Umsetzen mit den Hydroxysäuren 56 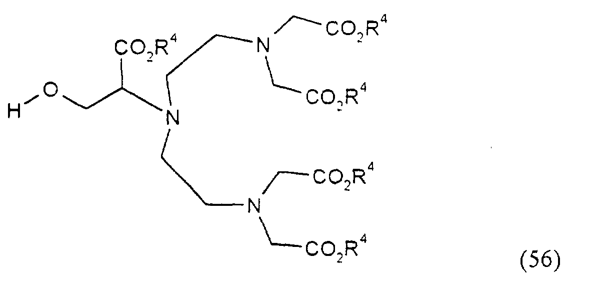 worin
R⁴ die oben genannte Bedeutung hat, erhalten. Die Verbindungen der Formel 56 sind in an sich bekannter Weise nach J. Org. Chem. 58, 1151 (1993), aus dem käuflichen Serinester 57 (Bachem, Fluka) 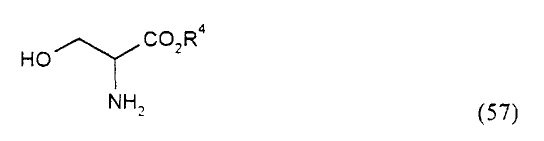 mit R⁴ in der oben genannten Bedeutung und den Halogencarbonsäureestern 58 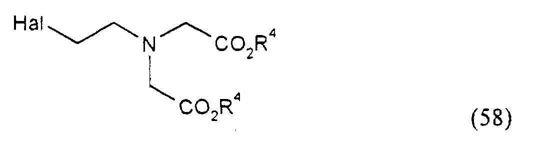 erhältlich.

Verbindungen der allgemeinen Formel I mit A in der Bedeutung der allgemeinen Formel V erhält man aus Verbindungen der allgemeinen Formel 59 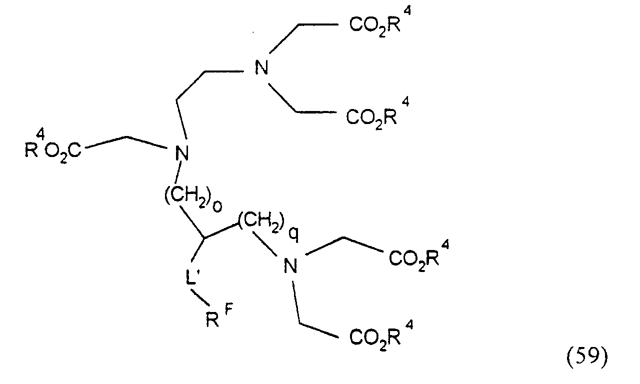 worin
L', o, q, R⁴ und R^{F} die oben genannte Bedeutung haben, durch gegebenenfalls Abspalten von Schutzgruppen und Komplexieren nach einer dem Fachmann bekannten Methode. [Protective Groups, EP 0 071 564, EP 0 130 934, DE-OS 3 401 052].

Verbindungen der allgemeinen Formel 59 lassen sich in bekannter Weise, beispielsweise nach J. Org. Chem., 58, 1151 (1993) durch Umsetzen von Halogencarbonsäureestern 18

Hal-CH₂CO₂R⁴ (18)

mit Hal und R⁴ in der oben genannten Bedeutung, und einer Verbindung der allgemeinen Formel 39 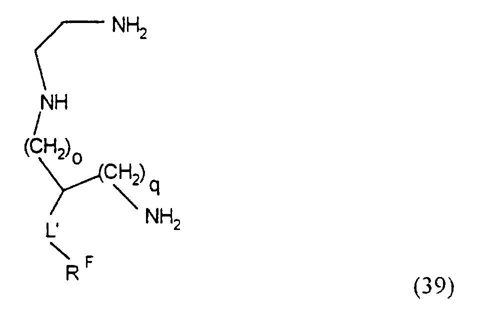 worin
L', o, q, und R^{F} die oben genannte Bedeutung haben, darstellen.

Die Verbindungen der allgemeinen Formel 39 werden für den Fall q = 0 aus den Verbindungen der allgemeinen Formel 60 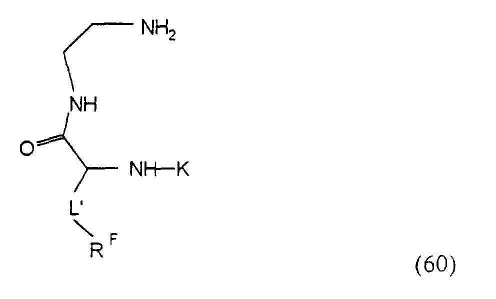 mit
L', R^{F} und K in der oben genannten Bedeutung
in an sich bekannter Weise [Helv. Chim. Acta, 77: 23 (1994)] durch Reduktion mit Diboran und Abspaltung der Schutzgruppen gewonnen. Die Verbindungen der allgemeinen Formel 60 werden durch Aminolyse der aktivierten Verbindungen der allgemeinen Formel 61 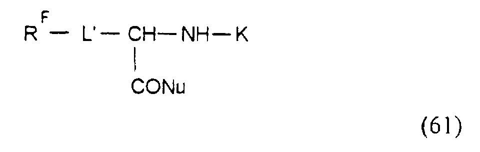 worin
L', Nu, R^{F} und K die oben genannte Bedeutung haben
mit Ethylendiamin erhalten.

Die Verbindungen der allgemeinen Formel 61 werden nach den bekannten Methoden der Schutzgruppenchemie [Protective Groups] aus der ungeschützten Säure der allgemeinen Formel 62 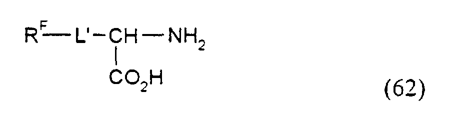 dargestellt, und zwar wird in einem ersten Schritt die Aminogruppe geschützt, gefolgt von der Aktivierung der Säuregruppe im zweiten Schritt.

Die Verbindungen der allgemeinen Formel 62 lassen sich nach den Methoden der Aminosäuresynthese [Houben-Weyl, Methoden der organischen Chemie, XI/2 Stickstoffverbindungen II und III, II Aminosäuren; Georg Thieme Verlag Stuttgart, 1958, Strecker-Reaktion, S. 305; Erlenmeyer-Reaktion, S. 306; Aminolyse von α-Halogencarbonsäuren, S. 309] aus den käuflichen Aldehyden der allgemeinen Formel 63

HOC―L'―R^{F} (63)

darstellen, beispielsweise nach Strecker, über das Azlacton oder über das Cyanhydrin.

Die Verbindungen der allgemeinen Formel 39 werden für den Fall o = 0 aus den Verbindungen der allgemeinen Formel 64 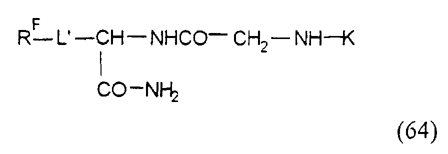 mit R^{F}, L' und K in den genannten Bedeutungen,
in an sich bekannter Weise durch Abspalten der Schutzgruppen und Reduktion mit Diboran gewonnen.

Verbindungen der allgemeinen Formel 64 sind durch Aminolyse der N-geschützten, aktivierten Glycine 53 mit Verbindungen der allgemeinen Formel 65 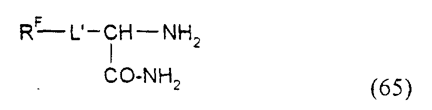 in der R^{F} und L' die genannten Bedeutungen haben,
zugänglich.

Die Verbindungen der allgemeinen Formel 65 sind in einfacher Weise aus Verbindungen der allgemeinen Formel 61 durch Amidbildung mit Ammoniak und anschließender Abspaltung der Schutzgruppe erhältlich.

Verbindungen der allgemeinen Formel XIII können analog den Verbindungen der allgemeinen Formel III hergestellt werden, indem man Halogencarbonsäurederivate der allgemeinen Formel 52 mit einer Verbindung der allgemeinen Formel 66 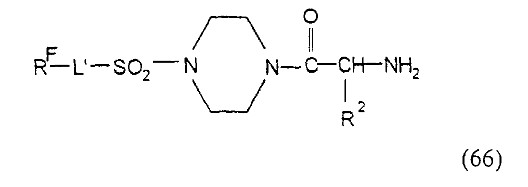 in der R^{F}, L' und R² die o. g. Bedeutungen haben,
umsetzt.

Die Verbindungen der allgemeinen Formel 66 werden durch Umsetzung einer Verbindung der allgemeinen Formel 67 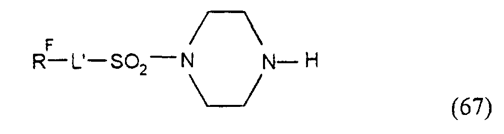 mit der aktivierten, N-geschützten Aminosäure der allgemeinen Formel 53 in Analogie zur Umsetzung des Amins 29 mit der Verbindung 53 hergestellt.

Die Verbindungen der allgemeinen Formel 67 können durch Umsetzung von Piperazin - frei oder gegebenfalls partiell geschützt - mit Perfluoralkylsulfonsäurefluoriden oder - chloriden erhalten werden. (Die Sulfonamidbildung aus Amin und Sulfofluorid ist beschrieben in
DOS 2 118 190, DOS 2 153 270, beide Bayer AG).

Verbindungen der allgemeinen Formel XI mit q in der Bedeutung der Ziffern 0 bzw. 1 werden analog zu Verbindungen der allgemeinen Formel VIII hergestellt, indem man Verbindungen der allgemeinen Formel 20 mit Verbindungen der allgemeinen Formel 68 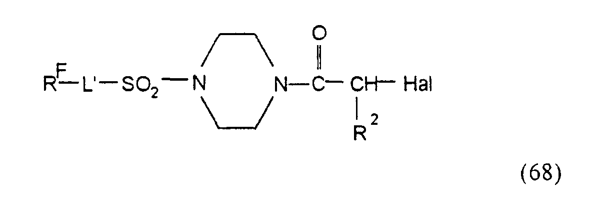 in der R^{F}, L', R² und Hal die o.g. Bedeutung haben,
bzw. mit Verbindungen der allgemeinen Formel 68a 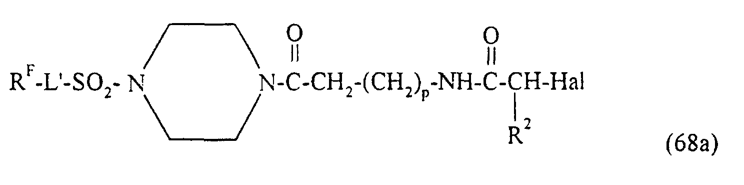 in der R^{F}, L', R², p und Hal die oben genannte Bedeutung haben, umsetzt.

Verbindungen der allgemeinen Formel 68 sind aus Verbindungen der allgemeinen Formel 30 und Piperazinderivaten der allgemeinen Formel 67 in an sich bekannter Weise erhältlich.

Verbindungen der allgemeinen Formel 68a sind aus Verbindungen der allgemeinen Formel 67 durch Amidkupplung mit Verbindungen der allgemeinen Formel 68 b

HOOC-CH₂―(CH₂)ₚ―NH-CO-CHR²-Hal (68b)

erhältlich

Verbindungen der allgemeinen Formel XII werden analog zu Verbindungen der allgemeinen Formel II hergestellt, z.B. durch Umsetzen von Verbindungen der Formel 49 mit Piperazinderivaten der allgemeinen Formel 67.

Verbindungen der allgemeinen Formel I mit A in der Bedeutung der allgemeinen Formel X erhält man aus Verbindungen der allgemeinen Formel 69 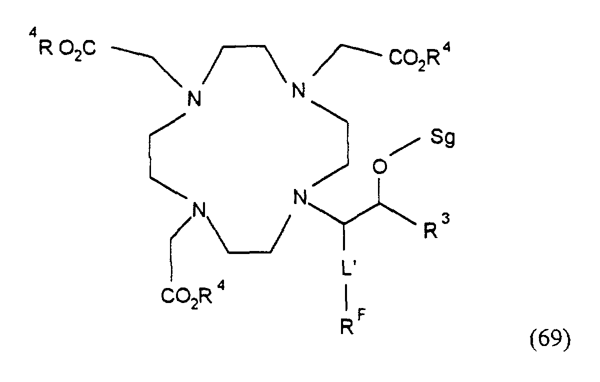 worin
L', R³, R⁴ und R^{F} die oben beschriebene Bedeutung haben und Sg in der Bedeutung einer Schutzgruppe steht,
durch gegebenenfalls Abspalten von Schutzgruppen und Komplexieren in an sich bekannter Weise [Protective Groups in Organic Synthesis, 2nd Edition, T. W. Greene and P. G. M. Wuts, John Wiley & Sons, Inc., New York, 1991
(EP 0 130 934, EP 0 250 358)].

Verbindungen der allgemeinen Formel 69 erhält man durch Umsetzen von a-Halogencarbonsäureestern oder -säuren der allgemeinen Formel 18 mit Verbindungen der allgemeinen Formel 70 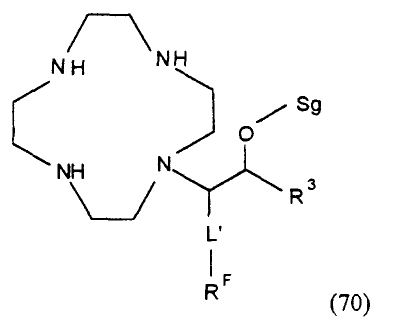 mit L', R^{F,} R³ und Sg in der oben genannten Bedeutung
nach den dem Fachmann bekannten Methoden wie beispielsweise in EP 0 255 471 oder US 4,885,363 beschrieben.

Verbindungen der allgemeinen Formel 70 sind durch Abspalten gegebenenfalls vorhandener Schutzgruppen und anschließender Reduktion mit Diboran nach den bekannten Verfahren aus Verbindungen der allgemeinen Formel 71 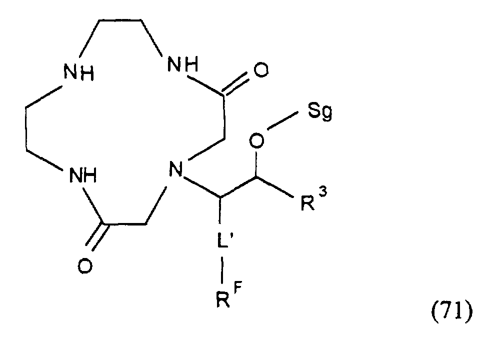 worin
L', R^{F}, R³ und Sg die oben genannte Bedeutung haben,
erhältlich.

Die Verbindungen der allgemeinen Formel 71 sind durch eine Kondensationsreaktion aus einem aktivierten Iminodiessigsäurederivat der allgemeinen Formel 72 und dem Diethylentriamin der Formel 73 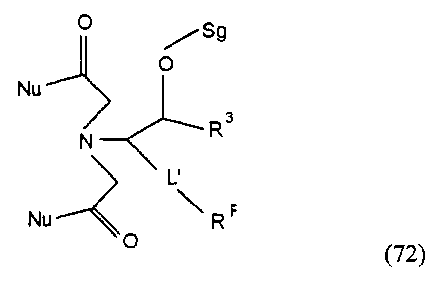 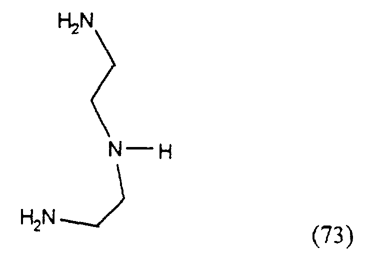 worin
L', R^{F} , R³ , Sg und Nu die oben genannte Bedeutung haben,
erhältlich.

Als Nucleofug Nu dient bevorzugt das N-Hydroxysuccinimid.

Verbindungen der allgemeinen Formel 72 sind aus Verbindungen der allgemeinen Formel 74 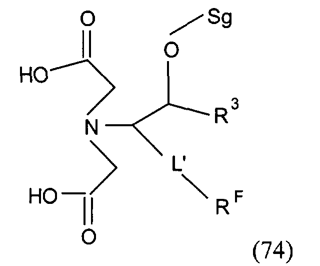 worin
L', R^{F} und Sg die oben genannte Bedeutung haben, durch Aktivierung der Carbonsäuren, wie auf Seite 11 beschrieben, erhältlich.

Verbindungen der allgemeinen Formel 74 erhält man durch Umsetzen von Halogencarbonsäureestern oder -säuren der allgemeinen Formel 18 mit Verbindungen der allgemeinen Formel 75 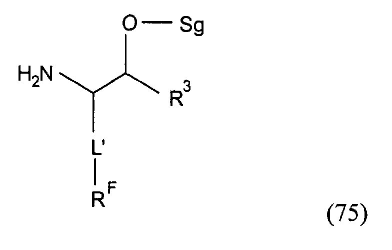 worin
L', R^{F} , R³ und Sg die oben genannte Bedeutung haben,
wobei gegebenenfalls vorhandene Estergruppen verseift werden.

Verbindungen der allgemeinen Formel 75 erhält man aus Verbindungen der allgemeinen Formel 76 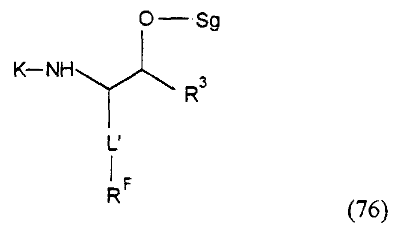 worin
L', R^{F} , R³, Sg und K die oben genannte Bedeutung haben,
durch Abspaltung der Schutzgruppe K nach den bekannten Verfahren.

Verbindungen der allgemeinen Formel 76 erhält man aus Verbindungen der allgemeinen Formel 77 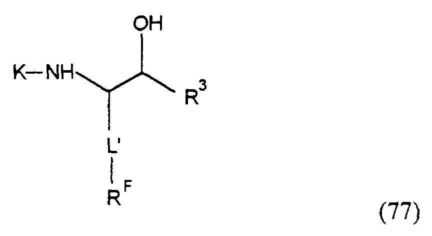 worin
L', R^{F} , R³ und K die oben genannte Bedeutung haben,
durch Einführen einer Schutzgruppe Sg in der dem Fachmann bekannten Weise.

Verbindungen der allgemeinen Formel 77 erhält man aus den Verbindungen der allgemeinen Formel 78 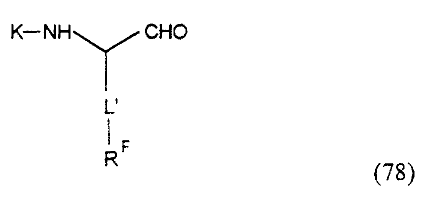 worin
L', R^{F} und K die oben genannten Bedeutung haben,
nach den dem Fachmann wohlbekannten Methoden (Houben-Weyl, Methoden der Organischen Chemie, XIII 2a, Metallorganische Verbindungen, Georg Thieme Verlag Stuttgart, 1973, S. 285 ff, Umsetzung magnesiumorganischer Verbindungen mit Aldehyden; S. 809 ff, Umsetzung von zinkorganischen Verbindungen mit Aldehyden; Houben-Weyl, Methoden der Organischen Chemie XIII/1, Metallorganische Verbindungen, Georg Thieme Verlag Stuttgart, 1970; S. 175 ff, Umsetzung lithiumorganischer Verbindungen mit Aldehyden) durch Umsetzen mit den aus Verbindungen der allgemeinen Formel 79

Hal―R³ (79)

worin
Hal und R³ die oben genannte Bedeutung haben,
erhältlichen metallorganischen Verbindungen, wie Magnesium-, Lithium- oder Zinkverbindungen.

Verbindungen der allgemeinen Formel 79 sind Kaufware (ABCR, Fluka).

Verbindungen der allgemeinen Formel 78 werden aus Verbindungen der allgemeinen Formel 80 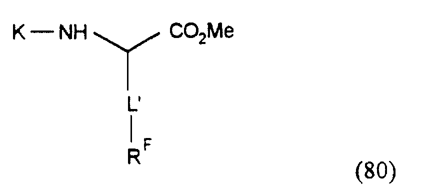 worin
L', R^{F} und K die oben genannte Bedeutung haben,
durch Reduktion mit Diisobutylaluminiumhydrid (Tett, Lett., 1962, 619;
Tett. Lett., 1969, 1779; Sythesis, 1975, 617). hergestellt.

Verbindungen der allgemeinen Formel 80 werden aus Verbindungen der allgemeinen Formel 45 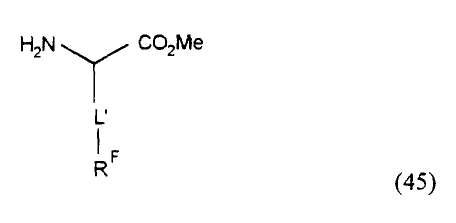 worin
L' und R^{F} die oben genannte Bedeutung haben,
auf eine dem Fachmann bekannte Weise durch Einführen der Schutzgruppe K hergestellt.

Die Neutralisation eventuell noch vorhandener freier Carboxygruppen erfolgt mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Gegenstand der Erfindung sind ferner pharmazeutische Mittel, die mindestens eine physiologisch verträgliche Verbindung der allgemeinen Formel I enthalten, ggf. mit den in der Galenik üblichen Zusätzen.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die zu den erfindungsgemäßen Metallkomplexen korrespondierenden Ca-Komplexe) oder - falls erforderlich - Elektrolyte wie zum Beispiel Natriumchlorid oder - falls erforderlich - Antioxidantien wie zum Beispiel Ascorbinsäure.

Prinzipiell ist es auch möglich, die erfindungsgemäßen pharmazeutischen Mittel ohne Isolierung der Komplexe herzustellen. In jedem Fall muß besondere Sorgfalt darauf verwendet werden, die Chelatbildung so vorzunehmen, daß die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Die erfindungsgemäßen pharmazeutischen Mittel enthalten vorzugsweise 0,1µMol - 1 Mol/l des Komplexes und werden in der Regel in Mengen von 0,0001 - 5 mMol/kg dosiert. Sie sind zur enteralen und parenteralen Applikation bestimmt. Die Komplexverbindungen gemäß Formel I finden Verwendung
1. mit den Ionen der Elemente der Ordnungszahlen 21 - 29, 39, 42, 44 und 57 - 83 für die Therapiekontrolle mittels NMR- und Röntgen-Diagnostik,
2. mit den Ionen der Elemente der Ordnungszahlen 12, 20-30, 39, 42, 44 und 57-83 im Gemisch mit Kontrastmitteln für die NMR- oder Röntgendiagnostik,
3. mit den Ionen der Elemente der Ordnungszahlen 12, 20-30, 39, 42, 44 und 57-83 im Gemisch mit Chemotheraopeutika,
4. mit den Ionen der Elemente der Ordnungszahlen 12, 20-30, 39, 42, 44 und 57-83 im Gemisch mit Kontrastmitteln für die NMR- oder Röntgendiagnostik und mit Chemotherapeutika.

Die erfindungsgemäßen Mittel zeigen die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit osmotische Effekte nicht zu lokalen unerwünschten Reaktionen führen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen gebundenen - an sich giftigen - Ionen innerhalb der Zeit, in der die Verbindungen der Formel I vollständig wieder ausgeschieden werden, nur äußerst langsam erfolgt.

Ferner können die Komplexverbindungen der Formel I vorteilhaft als Suszeptibilitäts-Reagenzien und als shift-Reagenzien für die in-vivo-NMR-Spektroskopie verwendet werden.

Die Verbindungen der Formel I zeichnen sich auch dadurch aus, daß sie vollständig aus dem Körper eliminiert werden und somit gut verträglich sind.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die Verbindungen der Formel I werden in Form ihrer wässrigen Lösungen mit den in der Pharmazie gebräuchlichen Zusatzstoffen (wie Puffer, Stabilisatoren etc.) eingesetzt.

Bei in Wasser schwerer löslichen Verbindungen hat sich der Zusatz von Lösungsvermittlern wie Ethanol, Dimethylsulfoxid, Propylenglycol oder Tween ® 80, Triton ® X - 100 bewährt.

In 96 %igem Ethanol ist die Löslichkeit der erfindungsgemäßen Substanzen sehr hoch, es können mehr als 500 mmol/l erreicht werden. Darüberhinaus fördert der hochkonzentrierte Alkohol den Embolisationsvorgang.

Die unterschiedlichen Viskositäten in den verschiedenen Lösungsmitteln (vor allem 66% Propylenglycol) können dazu genutzt werden, eine frei fließende, mit nur geringem Widerstand durch dünne Katheter applizierbare Lösung zu verabreichen. Durch den Verdünnungseffekt des Bluts im Zielorgan wird die Viskosität des Embolisierungsmittels stark erhöht, so daß sich ein Zustand wie in reinem Wasser bzw. Plasma einstellt.

In Kombinationspräparaten zur Behandlung von Tumoren werden bevorzugt 5-Fluoruracil, Mitomycin C, Cisplatin, Doxorubicin und Mitomycin eingesetzt. Zur Anwendung kommen je nach Bedarf wässrige Lösungen, wässrige Lösungen mit den in der Pharmazie gebräuchlichen Lösungsvermittlern oder Mikrokristall-Suspensionen, die mit den Verbindungen der Formel I gemischt werden können.

Die Chemotherapeutika werden in Dosen von 1 - 2000 mg, bevorzugt 5 - 1000 mg pro Applikation verabreicht, wobei eine Mehrfachgabe möglich ist.

Die erfindungsgemäßen Mittel werden vorzugsweise mittels Katheter in den Wirkort des zu therapierenden Tumors gebracht. Das verabreichte Volumen richtet sich nach der Größe des Tumors. In der Regel werden 2-80 ml appliziert.

Insgesamt ist es gelungen für die Verbindungen der allgemeinen Formel I gegebenenfalls in Kombination mit Chemotherapeutika, neue Möglichkeiten für die Therapie von Tumoren zu schaffen.

Darüberhinaus ermöglichen diese Verbindungen eine nichtinvasive Therapiekontrolle mittels NMR- oder Röntgendiagnostik (interventionelle Radiologie).

Die nachfolgenden Beispiele dienen zur näheren Erläuterung des Erfindungsgegenstands:

### Beispiel 1

a) N-Ethyl-N-(perfluoroctylsulfonyl)-amino-essigsäure-t-butylester
20 g (37,94 mmol) N-Ethylperfluorooctylsulfonamid und 15,73 g (113,8 mmol) Kaliumcarbonat werden in 200 ml Aceton suspendiert und bei 60°C 14,80 g (75,87 mmol) Bromessigsäure-tert.-butylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton= 10/10/1). Nach Eindampfen der produkthaltigen Fraktionen, kristallisiert man den Rückstand aus Methanol/Ether um.
Ausbeute: 21,66 g (89 % d. Th.) eines wachsartigen farblosen Feststoffes

| Elementaranalyse: | | | | | |
|---|---|---|---|---|---|
| ber. | C 29,96 | H 2,51 | F 50,36 | N 2,18 | S 5,00 |
| gef. | C 29,81 | H 2,70 | F 50,15 | N 2,30 | S 4,83 |

b) N-Ethyl-N-(perfluoroctylsulfonyl)-amino-essigsäure
20 g (31,18 mmol) der Titelverbindung aus Beispiel 1a) werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 17,34 g (95 % d. Th.) eines farblosen kristallinen Feststoffes

| Elementaranalyse: | | | | | |
|---|---|---|---|---|---|
| ber. | C 24,63 | H 1,38 | F 55,19 | N 2,39 | S 5,48 |
| gef. | C 24,48 | H 1,50 | F 55,01 | N 2,17 | S 5,59 |

c) Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan
10 g (17,09 mmol) der Titelverbindung aus Beispiel 1b) und 1,97 g (18,79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C und gibt 5,19 g (51,27 mmol) Triethylamin/50 ml 2-Propanol zu. Anschließend werden 10,78 g (18,79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18/Laufmittel: Gradient aus Wasser/n-Propanol/ Acetonitril).
Ausbeute: 16,37 g (78 % d. Th.) eines farblosen glasigen Feststoffes
Wassergehalt: 7,1 %
T₁-Relaxivität (L/mmol·sec) bei 20 MHz, 37°C:
41 (Wasser)
49 (Humanplasma)

| Elementaranalyse (bezogen auf wasserfreie Substanz): | | | | | | |
|---|---|---|---|---|---|---|
| ber. | C 30,58 | H 3,18 | F 28,31 | Gd 13,78 | N 7,37 | S 2,81 |
| gef. | C 30,40 | H 3,29 | F 28,14 | Gd 13,55 | N 7,28 | S 2,65 |

d) 10-[2-Hydroxy-4-aza-5-oxo-7-aza-7-(perfluoroctylsulfonyl)-nonyl]-1,4,7-tris(carboxymethyl)-1,4,7,10-tetraazacyclododecan
10 g (8,76 mmol) der Titelverbindung aus Beispiel 1c) werden in einer Mischung aus 100 ml Wasser/100 ml Ethanol gelöst und 1,73 g (13,71 mmol) Oxalsäure-Dihydrat zugesetzt. Man erhitzt 8 Stunden auf 80°C. Es wird auf 0°C abgekühlt und vom ausgefallenem Gadoliniumoxalat abfiltriert. Das Filtrat wird zur Trockne eingedampft und der Rückstand an RP-18 gereinigt
(RP-18/ Laufmittel: Gradient aus Wasser/i-Propanol/Acetonitril).
Ausbeute: 8,96 g (94 % d. Th.) eines glasigen Feststoffes
Wassergehalt: 9,3 %

| Elementaranalyse (bezogen auf wasserfreie Substanz): | | | | | |
|---|---|---|---|---|---|
| ber. | C 35,30 | H 3,98 | F 32,73 | N 8,52 | S 3,25 |
| gef. | C 35,10 | H 4,15 | F 32,51 | N 8,35 | S 3,15 |

e) Mangan-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan (als Natriumsalz)
5 g (5,07 mmol) der Titelverbindung aus Beispiel ld) werden in 100 ml Wasser gelöst und 0,58 g (5,07 mmol) Mangan(II) -carbonat zugegeben. Man rührt3 Stunden bei 80°C. Die Lösung wird filtriert und das Filtrat mit 1 N Natronlauge auf pH 7,2 gestellt, anschließend wird gefriergetrocknet.
Ausbeute: 5,87 g (quantitativ) eines farblosen amorphen Pulvers
Wassergehalt: 8,4 %
T₁-Relaxivität (L/mmol·sec) bei 20 MHz, 37°C:
2,7 (Wasser)
4,2 (Humanplasma)

| Elementaranalyse (bezogen auf wasserfreie Substanz): | | | | | | | |
|---|---|---|---|---|---|---|---|
| ber. | C 32,81 | H 3,42 | F 30,42 | Mn 5,17 | N 7,92 | Na 2,17 | S 3,02 |
| gef. | C 32,62 | H 3,57 | F 30,21 | Mn 5,06 | N 7,80 | Na 2,01 | S 2,90 |

f) Ytterbium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan
Zu 5 g (5,07 mmol) der Titelverbindung aus Beispiel 1 d) in 100 ml Wasser/30 ml Ethanol gibt man 1,33 g (2,53 mmol) Ytterbiumcarbonat und rührt 3 Stunden bei 80 °C. Die Lösung wird filtriert und das Filtrat im Vakuum zur Trockne eingedampft
Ausbeute: 6,36 g (quantitativ) eines glasigen Feststoffs.
Wassergehalt: 7,8 %.

| Elementaranalyse (bezogen auf wasserfreie Substanz): | | | | | | |
|---|---|---|---|---|---|---|
| ber. | C 30,11 | H 3,14 | F 27,92 | N 7,27 | S 2,77 | Yb 14,96 |
| gef. | C 30,02 | H 3,27 | F 27,80 | N 7,10 | S 2,68 | Yb 14,75 |

g) Dysprosium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan
Zu 5 g (5,07 mmol) der Titelverbindung aus Beispiel 1 d) in 100 ml Wasser/30 ml Ethanol gibt man 0,95 g (2,53 mmol) Dysprosiumoxid und rührt 3 Stunden bei 80 °C. Die Lösung wird filtriert und das Filtrat im Vakuum zur Trockne eingedampft.
Ausbeute: 6,35 g (quantitativ) eines farblosen glasigen Feststoffs.
Wassergehalt: 8,5 %.

| Elementaranalyse (bezogen auf wasserfreie Substanz): | | | | | | |
|---|---|---|---|---|---|---|
| ber. | C 30,39 | H 3,17 | F 28,18 | N 7,33 | S 2,80 | Dy 14,18 |
| gef. | C 30,17 | H 3,25 | F 28,03 | N 7,21 | S 2,65 | Dy 14,00 |

### Beispiel 2

a) 13,13,13,12,12,11,11,10,10,9,9,8,8,7,7,6,6-Heptadecafluor-3-oxa-tridecansäure-t.-butylester
Zu einer Mischung aus 10 g (21,55 mmol) 1H, 1H, 2H, 2H-Perfluordecan-1-ol und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäure-tert.-butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organischen Phasen werden über Magnesiumsulfat getrocknet und im Vakuum eingedampft. Der Rückstand wird an Kieselgel chromatographiert (Laufmittel: Hexan/Dichlormethan/Aceton= 20/10/1).
Ausbeute: 9,72 g (78 % d. Th.) eines farblosen viskosen Öls

| Elementaranalyse: | | | |
|---|---|---|---|
| ber. | C 33,23 | H 2,61 | F 55,85 |
| gef. | C 33,09 | H 2,78 | F 55,71 |

b) 13,13,13,12,12,11,11,10,10,9,9,8,8,7,7,6,6-Heptadecafluor-3-oxa-tridecansäure
9,0 g (15,56 mmol) der Titelverbindung aus Beispiel 2a) werden in 180 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 7,80 g (96 % d. Th.) eines farblosen Feststoffes

| Elementaranalyse: | | | |
|---|---|---|---|
| ber. | C 27,60 | H 1,35 | F 61,85 |
| gef. | C 27,48 | H 1,49 | F 61,66 |

c) Gadolinium-Komplex von 10-[2-Hydroxy-4-aza-5-oxo-7-oxa-10,10,11,11,12,12,13,13,14,14,15,15,16,16,17,17,17-heptadecafluor-heptadecyl]-1,4,7-tris(carboxymethyl)-1,4,7,10-tetraazacyclododecan
7,0 g (13,41 mmol) der Titelverbindung aus Beispiel 2b) und 1,70 g (14,75 mmol) N-Hydroxysuccinimid werden in einer Mischung aus 30 ml Dimethylformamid/ 20 ml Chloroform gelöst. Bei 0°C gibt man 3,04 g (14,75 mmol) Dicyclohexylcarbodiimid zu und rührt 1 Stunde bei 0°C, anschließend 3 Stunden bei Raumtemperatur. Man kühlt erneut auf 0°C und gibt 4,48 g (44,25 mmol) Triethylamin/50 ml 2-Propanol zu. Anschließend werden 8,46 g (14,75 mmol) Gadolinium-Komplex von 10-(3-Amino-2-hydroxy-propyl)-1,4,7-tris(carboxymethyl)-1,4,7,10-tetraazacyclododecan gelöst in 40 ml Wasser zugegeben und 3 Stunden bei Raumtemperatur gerührt. Man dampft zur Trockne ein, nimmt den Rückstand in einer Mischung aus 100 ml Methanol/30 ml Chloroform auf und filtriert vom Dicyclohexylharnstoff ab. Das Filtrat wird zur Trockne eingedampft und durch RP-Chromatographie gereinigt (RP-18/Laufmittel: Gradient aus Wasser/n-Propanol/Acetonitril).
Ausbeute: 11,8 g (75 % d. Th.) eines farblosen glasigen Feststoffes
Wassergehalt: 8,2 %
T₁-Relaxivität (L/mmol·sec) bei 20 MHz, 37°C:
19 (Wasser)
33 (Humanplasma)

| Elementaranalyse: | | | | | |
|---|---|---|---|---|---|
| ber. | C 32,32 | H 3,27 | F 29,96 | Gd 14,59 | N 6,50 |
| gef. | C 32,16 | H 3,42 | F 29,78 | Gd 14,39 | N 6,40 |

### Beispiel 3

a) 1,2-Epoxy-4-oxa-1H, 1H,2H,3H,3H,5H,5H,6H,6H-perfluor-tetradecan
Zu einer Mischung aus 20 g (43,09 mmol) 1H,1H,2H,2H-Perfluordecan-1-ol und 0,79 g (2,32 mmol) Tetrabutylammoniumhydrogensulfat in 200 ml 60%iger Kalilauge/100 ml Toluol tropft man unter starkem Rühren bei 10°C 7,97 g (86,18 mmol) Epichlorhydrin zu und achtet darauf, daß die Temperatur der Reaktionslösung nicht größer wie 20°C wird. Man läßt 2 Stunden bei 15°C rühren und tropft anschließend wie oben beschrieben 3,99 g (43,09 mmol) Epichlorhydrin zu. Dann wird über Nacht bei Raumtemperatur gerührt. Man gibt 100 ml Methyl-tert.-butylether zu und trennt die wässrige Phase ab. Diese wird 2 mal mit je 50 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 19,05 g (85 % d. Th.) eines farblosen Öls

| Elementaranalyse: | | | |
|---|---|---|---|
| ber. | C 30,02 | H 1,74 | F 62,09 |
| gef. | C 29,87 | H 1,95 | F 61,81 |

b) 10-[-2Hydroxy-4-oxa-1H,1H,2H,3H,3H,5H,5H,6H,6H-perfluor-tetradecyl]-1,4,7-tris(carboxymethyl)-1,4,7,10-tetraazacydododecan
Zu 12,0 g (34,60 mmol) 1,4,7-Tris(carboxymethyl)-1,4,7,10-tetraazacyclododecan in 50 ml Wasser gibt man 8,3 g (207,6 mmol) Natriumhydroxid. Hierzu tropft man eine Lösung aus 18,0 g (34,60 mmol) der Titelverbindung aus Beispiel 3a) gelöst in 60 ml n-Butanol/60 ml 2-Propanol und erwärmt die Lösung über Nacht auf 70°C. Man dampft im Vakuum zur Trockne ein, nimmt den Rückstand in 300 ml Wasser auf und stellt mit 3N Salzsäure auf pH 3. Dann wird 2 mal mit 200 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/ n-Butanol/Acetonitril).
Ausbeute: 26,61 g (79 % d. Th.)
Wassergehalt: 11,0 %

| Elementaranalyse (bezogen auf wasserfreie Substanz): | | | | |
|---|---|---|---|---|
| ber. | C 37,42 | H 4,07 | F 37,27 | N 6,47 |
| gef. | C 37,25 | H 4,19 | F 37,08 | N 6,30 |

c) Gadolinium-Komplex von 10-[-2Hydroxy-4-oxa-1H,1H,2H,3H,3H,5H,5H,6H,6H-perfluor-tetradecyl]-1,4,7-tris(carboxymethyl)-1,4,7,10-tetraazacyclododecan
10 g (11,54 mmol) der Titelverbindung aus Beispiel 3b) werden in einer Mischung aus 100 ml Wasser/50 ml 2-Propanol gelöst und 2,09 g (5,77 mmol) Gadoliniumoxid zugegeben. Man rührt 3 Stunden bei 80°C. Die Lösung wird filtriert und im Vakuum zur Trockne eingedampft.
Ausbeute: 12,48 g (quantitativ) eines glasigen Feststoffes
Wassergehalt: 5,6 %
T₁-Relaxivität (L/mmol·sec) bei 20 MHz, 37°C:
15,2 (Wasser)
27,5 (Humanplasma)

| Elementaranalyse (bezogen auf wasserfreie Substanz): | | | | | |
|---|---|---|---|---|---|
| ber. | C 31,77 | H 3,16 | F 31,64 | Gd 15,40 | N 5,49 |
| gef. | C 31,55 | H 3,30 | F 31,49 | Gd 15,28 | N 5,35 |

### Beispiel 4

a) 1,2-Epoxy-4-oxa-1H, 1H,2H,3H,3H,5H,5H,6H,6H-perfluordodecan
Zu einer Mischung aus 20 g (54,93 mmol) 1H,1H,2H,2H-Perfluoroctan-1-ol und 1,87 g (5,5 mmol) Tetrabutylammoniumhydrogensulfat in 200 ml 60%iger aqu. Kalilauge/100 ml Toluol tropft man unter starkem Rühren bei 10°C 10,17 g (109,9 mmol) Epichlorhydrin zu und achtet darauf, daß die Temperatur der Reaktionslösung nicht größer wie 20°C wird. Man läßt 2 Stunden bei 15°C rühren und tropft anschließend wie oben beschrieben, 5,08 g (54,93 mmol) Epichlorhydrin zu. Dann wird über Nacht bei Raumtemperatur gerührt. Man gibt 100 ml Toluol und 100 ml Methyl-tert.-butylether zu und trennt die wässrige Phase ab. Diese wird 2 mal mit je 50 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 19,15 g (83 % d. Th.) eines farblosen Öls

| Elementaranalyse: | | | |
|---|---|---|---|
| ber. | C 31,44 | H 2,16 | F 58,78 |
| gef. | C 31,40 | H 2,29 | F 58,55 |

b) 10-[2-Hydroxy-4-oxa-1H,1H,2H,3H,3H,5H,5H,6H,6H-perfluordodecyl]-1,4,7-tris(carboxymethyl)-1,4,7,10-tetraazacyclododecan
Zu 14,84 g (42,84 mmol) 1,4,7-Tris(carboxymethyl)-1,4,7,10-tetraazacyclododecan (DO3A) in 70 ml Wasser gibt man 10,3 g (257 mmol) Natriumhydroxid. Hierzu tropft man eine Lösung aus 18 g (42,84 mmol) der Titelverbindung aus Beispiel 4a) gelöst in 80 ml n-Butanol/60 ml 2-Propanol und erwärmt die Lösung über Nacht auf 70°C. Man dampft im Vakuum zur Trockne ein, nimmt den Rückstand in 300 ml Wasser auf und stellt mit 3N Salzsäure auf pH 3. Dann wird 2 mal mit 200 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/ n-Butanol/Acetonitril).
Ausbeute: 27,4 g (75 % d. Th.) eines glasigen Feststoffes
Wassergehalt: 10,1 %

| Elementaranalyse (bezogen auf wasserfreie Substanz): | | | | |
|---|---|---|---|---|
| ber. | C 39,17 | H 4,60 | F 32,22 | N 7,31 |
| gef.z | C 39,05 | H 4,85 | F 32,05 | N 7,19 |

c) Gadolinium-Komplex von 10-[2-Hydroxy-4-oxa-1H,1H,2H,3H,3H,5H,5H,6H,6H-perfluordodecyl]-1,4,7-tris(carboxymethyl)-1,4,7,10-tetraazacyclododecan
10 g (13,04 mmol) der Titelverbindung aus Beispiel 4b) werden in einer Mischung aus 100 ml Wasser/50 ml 2-Propanol gelöst und 2,36 g (6,52 mmol) Gadoliniumoxid zugegeben. Man rührt 3 Stunden bei 80°C. Die Lösung wird filtriert und im Vakuum zur Trockne eingedampft.
Ausbeute: 12,77 g (quantitativ) eines glasigen Feststoffes
Wassergehalt: 6,1 %

| Elementaranalyse (bezogen auf wasserfreie Substanz): | | | | | |
|---|---|---|---|---|---|
| ber. | C 32,61 | H 3,50 | F 26,82 | Gd 17,08 | N 6,08 |
| gef. | C 32,43 | H 3,69 | F 26,67 | Gd 16,85 | N 5,91 |

### Beispiel 5

a) 9,9,9,8,8,7,7,6,6-Nonafluor-3-oxa-nonansäure-t.-butylester
Zu einer Mischung aus 20 g (75,73 mmol) 1H,1H,2H,2H-Perfluorhexan-1-ol und 2,57 g (7,57 mmol) Tetrabutylammoniumhydrogensulfat in 300 ml 60 %iger aqu. Kalilauge/200 ml Toluol tropft man unter starkem Rühren bei 0°C 29,54 g (151,5 mmol) Bromessigsäure-tert.-butylester zu. Man rührt 1 Stunde bei 0°C. Es werden 100 ml Toluol zugegeben, die wässrige Phase abgetrennt und 2 mal mit 50 ml Toluol extrahiert. Die vereinigten organischen Phasen werden über Magnesiumsulfat getrocknet und im Vakuum eingedampft. Der Rückstand wird an Kieselgel chromatographiert (Laufmittel: Hexan/Dichlormethan/Aceton= 20/10/1).
Ausbeute: 21,48 g (75 % d. Th.) eines farblosen Öls

| Elementaranalyse: | | | |
|---|---|---|---|
| ber. | C 38,11 | H 4,00 | F 45,21 |
| gef. | C 37,95 | H 4,18 | F 45,03 |

b) 9,9,9,8,8,7,7,6,6-Nonafluor-3-oxa-nonansäure
20 g (52,88 mmol) der Titelverbindung aus Beispiel 5a) werden in 300 ml Trifluoressigsäure gelöst und über Nacht bei Raumtemperatur gerührt. Man dampft im Vakuum zur Trockne ein. Der Rückstand wird aus Hexan/Ether umkristallisiert.
Ausbeute: 14,82 g (87 % d. Th.) eines farblosen kristallinen Feststoffes

| Elementaranalyse: | | | |
|---|---|---|---|
| ber. | C 29,83 | H 2,19 | F 53,08 |
| gef. | C 29,71 | H 2,40 | F 52,90 |

c) Gadolinium-Komplex von 10-[2-Hydroxy-4-aza-5-oxo-7-oxa-10,10,11,11,12,12,13,13,13-nonafluor-tridecyl]-1,4,7-tris(carboxymethyl)-1,4,7,10-tetraazacyclododecan
7,41 g (23,01 mmol) der Titelverbindung aus Beispiel 5b) und 2,91 g (25,31 mmol) N-Hydroxysuccinimid werden in einer Mischung aus 40 ml Dimethylformamid/ 20 ml Chloroform gelöst. Bei 0°C gibt man 5,22 g (25,31 mmol) Dicyclohexylcarbodiimid zu und rührt 1 Stunde bei 0°C, anschließend 3 Stunden bei Raumtemperatur. Man kühlt erneut auf 0°C und gibt 6,98 g (69 mmol) Triethylamin/30 ml 2-Propanol zu. Anschließend werden 13,2 g (23,01 mmol) Gadolinium-Komplex von 10-(3-Amino-2-hydroxy-propyl)-1,4,7-tris(carboxymethyl)-1,4,7,10-tetraazacyclododecan gelöst in 40 ml Wasser zugegeben und 3 Stunden bei Raumtemperatur gerührt. Man dampft zur Trockne ein, nimmt den Rückstand in einer Mischung aus 200 ml Methanol/50 ml Chloroform auf und filtriert vom Dicyclohexylharnstoff ab. Das Filtrat wird zur Trockne eingedampft und durch RP-Chromatographie gereinigt (RP-18/Laufmittel: Gradient aus Wasser/n-Propanol/Acetonitril).
Ausbeute: 15,20 g (71 % d. Th.) eines farblosen glasigen Feststoffes
Wassergehalt: 5,7 %

| Elementaranalyse (bezogen auf wasserfreie Substanz): | | | | | |
|---|---|---|---|---|---|
| ber. | C 34,21 | H 4,02 | F 19,48 | Gd 17,91 | N 7,98 |
| gef. | C 34,09 | H 4,18 | F 19,31 | Gd 17,74 | N 7,87 |

### Beispiel 6

a) N-Ethyl-N-(perfluoroctylsulfonyl)-amino-essigsäure-N-(2-aminoethyl)-amid
15 g (25,63 mmol) der Titelverbindung aus Beispiel 1b) und 3,24 g (28,19 mmol) N-Hydroxysuccinimid werden in 80 ml Dimethylformamid gelöst und bei 0°C 5,82 g (28,19 mmol) Dicyclohexylcarbodiimid zugegeben. Man rührt 1 Stunde bei 0°C, anschließend 2 Stunden bei Raumtemperatur. Man filtriert vom ausgefallenem Dicyclohexylharnstoff ab und tropft das Filtrat innerhalb 30 Minuten in eine Lösung aus 46,21 g (768,9 mmol) Ethylendiamin in 300 ml Dichlormethan. Man rührt 5 Stunden bei Raumtemperatur. Man setzt 1000 ml H₂O zu und trennt die organische Phase ab. Diese wird 2 mal mit je 500 ml Wasser gewaschen, dann über Magnesiumsulfat getrocknet und im Vakuum zur Trockne eingeengt. Die Reinigung erfolgt durch Chromatographie an Kieselgel. (Laufmittel: Dichlormethan/2-Propanol= 15/1).
Ausbeute: 11,79 g (75 % d. Th.) eines farblosen, wachsartigen Feststoffes

| Elementaranalyse: | | | | | |
|---|---|---|---|---|---|
| ber. | C 27,42 | H 2,30 | F 52,66 | N 4,57 | S 5,23 |
| gef. | C 27,20 | H 2,41 | F 52,48 | N 4,38 | S 5,10 |

b) N-Ethyl-N-(perfluoroctylsulfonyl)-amino-essigsäure-N-[2-(bromacetyl)-aminoethyl]-amid
10 g (16,3 mmol) der Titelverbindung aus Beispiel 6a) und 2,02 g (20 mmol) Triethylamin werden in 40 ml Dichlormethan gelöst. Bei -10°C tropft man innerhalb von 30 Minuten 3,29 g (16,3 mmol) Bromacetylbromid zu und rührt 2 Stunden bei 0°C. Man gießt die Lösung in 300 ml 1 N Salzsäure und rührt gut durch. Die organische Phase wird abgetrennt, über Magnesiumsulfat getrocknet und im Vakuum eingeengt. Der Rückstand wird an Kieselgel chromatographiert (Laufmittel: Dichlormethan/Aceton= 20/1).
Ausbeute: 11,1 g (91 % d. Th.) eines leicht gelbgefärbten wachsartigen Feststoffes

| Elementaranalyse: | | | | | | |
|---|---|---|---|---|---|---|
| ber. | C 25,68 | H 2,02 | Br 10,68 | F 43,16 | N 5,62 | S 4,29 |
| gef. | C 25,47 | H 2,18 | Br 10,45 | F 43,29 | N 5,47 | S 4,10 |

c) 10-[2-Oxo-3-aza-6-aza-7-oxo-9-aza-9-(perfluoroctylsulfonyl)-undecyl]-1,4,7-tris(carboxymethyl)-1,4,7,10-tetraazacyclododecan
Zu 10 g (13,36 mmol) der Titelverbindung aus Beispiel 6b) in 180 ml Methanol gibt man 4,63 g (13,36 mmol) 1,4,7-Tris(carboxymethyl)-1,4,7,10-tetraazacyclododecan (DO3A) und 18,5 g (133,6 mmol) Kaliumkarbonat. Man kocht 12 Stunden unter Rückfluss. Die anorganischen Salze werden abfiltriert und das Filtrat zur Trockne eingedampft. Der Rückstand wird in 100 ml Wasser aufgenommen und mit 5 N Salzsäure auf pH 3 gestellt. Man extrahiert 2 mal mit 150 ml n-Butanol. Die vereinigten organischen Phasen werden im Vakuum zur Trockne eingeengt und der Rückstand durch RP-Chromatographie gereinigt (RP-18/Laufmittel= Gradient aus Wasser/n-Butanol/Acetonitril).
Ausbeute: 10,43 g (67 % d. Th.) eines farblosen Feststoffes
Wassergehalt: 13,0 %

| Elementaranalyse (bezogen auf wasserfreie Substanz): | | | | | |
|---|---|---|---|---|---|
| ber. | C 35,55 | H 3,98 | F 31,86 | N 9,67 | S 3,16 |
| gef. | C 35,37 | H 3,75 | F 31,64 | N 9,78 | S 3,25 |

d) Gadolinium-Komplex von 10-[2-Oxo-3-aza-6-aza-7-oxo-9-aza-9-(perfluoroctylsulfonyl)-undecyl]-1,4,7-tris(carboxymethyl)-1,4,7,10-tetraazacyclododecan
10 g (9,86 mmol) der Titelverbindung aus Beispiel 6c) werden in einer Mischung aus 50 ml Wasser/20 ml Ethanol gelöst und 1,79 g (4,93 mmol) Gadoliniumoxid zugegeben. Man rührt 4 Stunden bei 80°C. Die Lösung wird filtriert und im Vakuum zur Trockne eingedampft.
Ausbeute: 12,4 g (quantitativ)
Wassergehalt: 7,1 %

| Elementaranalyse (bezogen auf wasserfreie Substanz): | | | | | | |
|---|---|---|---|---|---|---|
| ber. | C 30,85 | H 3,19 | F 27,65 | Gd 13,46 | N 8,39 | S 2,75 |
| gef. | C 30,64 | H 3,35 | F 27,58 | Gd 13,29 | N 8,28 | S 2,65 |

### Beispiel 7

a) 1H,1H,2H,2H-Perfluordecan-1-ol-p-toluolsulfonsäureester
Zu 30 g (64,64 mmol) 1H,1H,2H,2H-Perfluordecan-1-ol in 300 ml Dichlormethan und 10,12 g (100 mmol) Triethylamin gibt man bei 0°C 12,57 g (65,93 mmol) p-Toluolsulfonsäurechlorid. Man rührt 2 Stunden bei 0°C, anschließend 2 Stunden bei Raumtemperatur. Die Lösung wird in 500 ml kalter 2 N Salzsäure gegossen und kräftig gerührt. Die organische Phase wird abgetrennt, über Magnesiumsulfat getrocknet und zur Trockne eingeengt. Der Rückstand wird aus wenig Methanol umkristallisiert.
Ausbeute: 39,97 (95 % d. Th.) eines farblosen kristallinen Pulvers

| Elementaranalyse: | | | | |
|---|---|---|---|---|
| ber. | C 33,02 | H 1,79 | F 52,23 | S 5,19 |
| gef. | C 32,81 | H 1,93 | F 52,04 | S 5,05 |

b) 10-[(1-Hydroxymethyl-1-carboxy)-methyl]-1,4,7-tris(carboxymethyl)-1,4,7,10-tetraazacyclododecan
Zu einer Lösung aus 20 g (57,78 mmol) 1,4,7-Tris(carboxymethyl)-1,4,7,10-tetraazacyclododecan (DO3A), 31,21 g (780 mmol) Natriumhydroxid und 2 g (12 mmol) Kaliumjodid in 100 ml Dimethylformamid gibt man 37,2 g (173,4 mmol) 2-Chlor-3-benzyloxy-propansäure und rührt 3 Tage bei 60°C. Man dampft zur Trockne ein und löst den Rückstand in 300 ml Wasser. Dann stellt man mit 3 N Salzsäure auf pH 3 und extrahiert 2 mal mit je 250 ml Dichlormethan. Zur Wasserphase gibt man 4 g Palladiumkatalysator (10 % Pd/C) und hydriert 5 Stunden bei 60°C. Der Katalysator wird abfiltriert und das Filtrat zur Trockne eingeengt. Der Rückstand wird durch RP-Chromatographie gereinigt (RP-18/Laufmittel= Gradient aus Wasser/2-Propanol/Acetonitril).
Ausbeute: 5,92 g (21 % d. Th. bezogen auf D03A) eines farblosen glasigen Feststoffes
Wassergehalt: 11,1 %

| Elementaranalyse (bezogen auf wasserfreie Substanz): | | | |
|---|---|---|---|
| ber. | C 47,00 | H 6,96 | N 12,90 |
| gef. | C 46,81 | H 6,78 | N 12,99 |

c) 10-[1-Hydroxymethyl-1-(methoxycarbonyl)-methyl]-1,4,7-tris(methoxycarbonylmethyl)-1,4,7,10-tetraazacyclododecan
Zu 200 ml Methanol tropft man bei 0°C 9,53 g (80 mmol) Thionylchlorid. Dann gibt man 5,8 g (13,35 mmol) der Titelverbindung aus Beispiel 7b) zu und rührt 1 Stunde bei 0°C. Dann erwärmt man 6 Stunden auf 60°C. Man dampft zur Trockne ein, nimmt den Rückstand in 150 ml Methylenchlorid auf und extrahiert 3 mal mit je 200 ml 8 %iger aqu. Soda-Lösung. Die organische Phase wird über Magnesiumsulfat getrocknet und zur Trockne eingedampft. Man erhält 6,09 g (93 % d. Th.) der Titelverbindung als leicht gelblich gefärbtes Öl.

| Elementaranalyse: | | | |
|---|---|---|---|
| ber. | C 51,42 | H 7,81 | N 11,42 |
| gef. | C 51,20 | H 7,95 | N 11,28 |

d) 10-[1-(Methoxycarbonyl)-3-oxa-1H,2H,2H,4H,4H,5H,5H-perfluortridecyl]-1,4,7-tris(methoxycarbonylmethyl)-1,4,7,10-tetraazacyclododecan
Zu 6 g (12,23 mmol) der Titelverbindung aus Beispiel 7c) in 40 ml Dimethylformamid gibt man 0,44 g (14,68 mmol) Natriumhydrid (80 %ige Suspension in Mineralöl) und rührt 30 min bei -10°C. Dann werden 8,32 g (13,45 mmol) der Titelverbindung aus Beispiel 7a) zugesetzt und 8 Stunden bei Raumtemperatur gerührt. Man setzt vorsichtig 400 ml Eiswasser zu und extrahiert 2 mal mit je 300 ml Essigsäureethylester. Die vereinigten Essigsäureethylester-phasen werden mit gesättigter aqu. Kochsalzlösung gewaschen und über Magnesiumsulfat getrocknet. Man dampft im Vakuum zur Trockne ein und chromatographiert den Rückstand an Kieselgel (Laufmittel: Dichlormethan/Methanol= 20/1).
Ausbeute: 7,68 g (67 % d. Th.) eines zähen gelben Öls

| Elementaranalyse: | | | | |
|---|---|---|---|---|
| ber. | C 39,75 | H 4,41 | F 34,48 | N 5,98 |
| gef. | C 39,58 | H 4,60 | F 34,27 | N 5,75 |

e) 10-[1-Carboxy-3-oxa-1H,2H,2H,4H,4H,5H,5H-perfluortridecyl]-1,4,7-tris(carboxymethyl)-1,4,7,10-tetraazacyclododecan
7,5 g (8,01mmol) der Titelverbindung aus Beispiel 7d) werden in einer Mischung aus 50 ml Wasser/30 ml Ethanol suspendiert und anschließend 3,84 g (96 mmol) Natriumhydroxid zugegeben. Man kocht über Nacht unter Rückfluß. Es wird auf Raumtemperatur abgekühlt und mit 3 N Salzsäure auf pH 3 gestellt. Man dampft im Vakuum zur Trockne ein und reinigt den Rückstand durch RP-Chromatographie (RP-18/Laufmittel= Gradient aus Wasser/n-Butanol/Acetonitril).
Ausbeute: 6,84 g (87 % d. Th.) eines glasigen Feststoffes
Wassergehalt: 10,3 %

| Elementaranalyse (bezogen auf wasserfreie Substanz): | | | | |
|---|---|---|---|---|
| ber. | C 36,83 | H 3,78 | F 36,68 | N 6,36 |
| gef. | C 36,67 | H 3,90 | F 36,49 | N 6,25 |

f) Gadolinium-Komplex von 10-[1-Carboxy-3-oxa-1H,2H,2H,4H,4H,5H,5H-perfluortridecyl] 1,4,7-tris(carboxymethyl)-1,4,7,10-tetraazacyclododecan (als Natriumsalz)
6 g (6,81 mmol) der Titelverbindung aus Beispiel 7e) werden in 80 ml Wasser suspendiert und 1,23 g (3,4 mmol) Gadoliniumoxid zugegeben. Man erhitzt 3 Stunden auf 90°C. Man läßt auf Raumtemperatur abkühlen und stellt mit 2 N Natronlauge auf pH 7,2 ein. Die Lösung wird filtriert und anschließend gefriergetrocknet.
Ausbeute: 7,83 g (quantitativ) eines farblosen, flockigen Pulvers
Wassergehalt: 8,1 %

| Elementaranalyse (bezogen auf wasserfreie Substanz): | | | | | | |
|---|---|---|---|---|---|---|
| ber. | C 30,69 | H 2,77 | F 30,56 | Gd 14,88 | N 5,30 | Na 2,18 |
| gef. | C 30,48 | H 2,85 | F 30,37 | Gd 14,69 | N 5,17 | Na 1,95 |

### Beispiel 8

a) 2H,2H-Perfluoroctanal
30 g (82,4 mmol) 1H,1H,2H,2H-Perfluoroctan-1-ol werden in 500 ml Dichlormethan gelöst und 17,76 g (82,4 mmol) Pyridiniumchlorochromat zugegeben. Man rührt über Nacht bei Raumtemperatur. Die Lösung wird über eine kurze Säule, gefüllt mit Aluminiumoxid (neutral) filtriert, das Filtrat zur Trockne eingedampft und der Rückstand an Kieselgel chromatographiert (Laufmittel: Dichlormethan/ Hexan/Aceton=10/10/1).
Ausbeute: 26,55 g (89 % d. Th.) eines wachsartigen Feststoffes

| Elementaranalyse: | | | |
|---|---|---|---|
| ber. | C 26,54 | H 0,84 | F 68,21 |
| gef. | C 26,47 | H 1,05 | F 68,10 |

b) 2-Amino-2H,3H,3H-perfluornonansäure (als Hydrochlorid)
7,04 g (143,6 mmol) Natriumcyanid und 8,45 g (158 mmol) Ammoniumchlorid werden in 30 ml Wasser gelöst. Zu dieser Lösung gibt man 40 ml Ethanol und 26 g (71,8 mmol) der Titelverbindung aus Beispiel 8a). Man erwärmt 2 Stunden auf 45°C. Es wird 300 ml Wasser zugesetzt und 3 mal mit je 200 ml Benzol extrahiert. Die vereinigten Benzolphasen werden 3 mal mit je 200 ml Wasser gewaschen und die organische Phase im Vakuum zur Trockne eingedampft. Der Rückstand wird in 100 ml 6 N aqu. Salzsäure/50 ml Methanol aufgenommen und 2 Stunden unter Rückfluß erhitzt. Man dampft im Vakuum zur Trockne ein. Der Rückstand wird aus wenig 2-Propanol/Methyl-tert.-butylether umkristallisiert.
Ausbeute: 11,15 g (35 % d. Th.) eines kristallinen Feststoffes

| Elementaranalyse: | | | | | |
|---|---|---|---|---|---|
| ber. | C 24,37 | H 1,59 | Cl 7,99 | F 55,68 | N 3,16 |
| gef. | C 24,15 | H 1,72 | Cl 7,65 | F 55,51 | N 3,05 |

c) 2-[(N-Benzyloxycarbonyl)-triglycidyl]-amino-2H,3H,3H-perfluornonansäure
8,37 g (24,8 mmol) N-Benzyloxycarbonyl-Triglycin und 3,14 g (27,28 mmol) N-Hydroxysuccinimid werden in 80 ml Dimethylformamid gelöst und bei 0°C 5,63 g (27,28 mmol) Dicyclohexylcarbodiimid zugegeben. Man rührt 1 Stunde bei 0°C, dann 2 Stunden bei Raumtemperatur. Man kühlt auf 0°C ab, gibt 7,53 g (74,4 mmol) Triethylamin und 11 g (24,8 mmol) der Titelverbindung aus Beispiel 8b zu und rührt anschließend über Nacht bei Raumtemperatur. Man dampft im Vakuum zur Trockne ein, nimmt den Rückstand in 300 ml 5 %iger aqu. Zitronensäure auf und extrahiert 3 mal mit je 200 ml Essigsäureäthylester. Die vereinigten organischen Phasen werden über Magnesiumsulfat getrocknet und im Vakuum zur Trockne eingedampft. Der Rückstand wird an Kieselgel chromatographiert (Laufmittel: Dichlormethan/n-Propanol= 20/1).
Ausbeute: 11,83 g (67 % d. Th.) eines farblosen, schuppenartigen Feststoffes

| Elementaranalyse: | | | | |
|---|---|---|---|---|
| ber. | C 38,78 | H 2,97 | F 34,67 | N 7,86 |
| gef. | C 38,59 | H 2,85 | F 34,48 | N 7,91 |

d) 2-[Triglycidyl]-amino-2H,3H,3H-perfluornonansäure
11,5, g (16,14 mmol) der Titelverbindung aus Beispiel 8c) werden in 200 ml 2-Propanol gelöst und 3 g Palladium-Katalysator (10 % Pd/C) zugegeben. Man hydriert über Nacht bei Raumtemperatur. Es wird vom Katalysator abfiltriert und das Filtrat zur Trockne eingedampft.
Ausbeute: 9,33 g (quantitativ) eines farblosen Feststoffes

| Elementaranalyse: | | | | |
|---|---|---|---|---|
| ber. | C 31,15 | H 2,61 | F 42,71 | N 9,69 |
| gef. | C 31,29 | H 2,80 | F 42,53 | N 9,48 |

e) 2-(1H,1H-Perfluorheptyl)-1,4,7,10-tetraaza-3,6,9,12-tetraoxo-cyclododecan
9,2 g (15,91 mmol) der Titelverbindung aus Beispiel 8d) werden in 1000 ml Dimethylformamid gelöst und 3,93 g (15,91 mmol) 2-Ethoxy-1-ethoxycarbonyl-1,2-dihydroquinolin zugegeben. Man rührt 3 Tage bei Raumtemperatur. Es wird zur Trockne eingedampft und der Rückstand an Kieselgel chromatographiert (Laufmittel: Dichlormethan/2-Propanol= 20/1).
Ausbeute: 4,54 g (51 % d. Th.) eines wachsartigen Feststoffes

| Elementaranalyse: | | | | |
|---|---|---|---|---|
| ber. | C 32,16 | H 2,34 | F 44,08 | N 10,00 |
| gef. | C 32,05 | H 2,47 | F 43,87 | N 9,89 |

f) 2-(1H,1H-Perfluorheptyl)-1,4,7,10-tetraazacyclododecan (als Tetrahydrochlorid)
Zu 4,4 g (7,85 mmol) der Titelverbindung aus Beispiel 8e) gibt man 200 ml 1 M-Boran-Tetrahydrofuran-Komplex-Lösung zu und kocht 2 Tage unter Rückfluß. Man dampft im Vakuum zur Trockne ein und nimmt den Rückstand in 50 ml konz. Salzsäure auf. Es werden 100 ml Ethanol zugegeben und 8 Stunden unter Rückfluß gekocht. Es wird im Vakuum zur Trockne eingedampft und der Rückstand aus Ethanol umkristallisiert.
Ausbeute: 4,75 g (93 % d. Th.) eines farblosen kristallinen Pulvers

| Elementaranalyse: | | | | | |
|---|---|---|---|---|---|
| ber. | C 27,71 | H 3,88 | Cl 21,81 | F 37,99 | N 8,62 |
| gef. | C 27,65 | H 3,95 | Cl 21,40 | F 37,69 | N 8,41 |

g) 2-(1H,1H-perfluorheptyl)-1,4,7,10-tetra(carboxymethyl)-1,4,7,10-tetraazacyclododecan
4,6 g (7,07 mmol) der Titelverbindung aus Beispiel 8f) und 4,0 g (42,4 mmol) Chloressigsäure werden in 40 ml Wasser gelöst und der pH durch Zugabe von 30 %iger aqu. Kalilauge auf pH 10 gestellt. Man erwärmt 8 Stunden auf 70°C und hält dabei den pH-Wert zwischen 8 und 10 (durch Zugabe von 30 %iger aqu. Kalilauge). Die Lösung wird auf Raumtemperatur abgekühlt, mit konz. Salzsäure auf pH 2 gestellt und zur Trockne eingedampft. Der Rückstand wird in 150 ml Methanol aufgenommen, die Salze abfiltriert und das Filtrat im Vakuum zur Trockne eingedampft. Der Rückstand wird durch RP-18 Chromatographie gereinigt (RP-18/Laufmittel: Gradient aus Wasser/2-Propanol/Acetonitril).
Ausbeute: 5,03 g (87 % d. Th.) eines glasigen Feststoffes
Wassergehalt: 10,1 %

| Elementaranalyse (bezogen auf wasserfreie Substanz): | | | | |
|---|---|---|---|---|
| ber. | C 37,51 | H 3,97 | F 33,53 | N 7,61 |
| gef. | C 37,35 | H 4,12 | F 33,40 | N 7,45 |

h) Gadolinium-Komplex von 2-(1H, 1H-perfluorheptyl)-1,4,7,10-tetra(carboxymethyl)-1,4,7,10-tetraazacyclododecan (als Natriumsalz)
4,5 g (6,11 mmol) der Titelverbindung aus Beispiel 8g) werden in 100 ml Wasser suspendiert und 1,107 g (3,05 mmol) Gadoliniumoxid zugegeben. Man erhitzt 3 Stunden auf 90°C. Man läßt auf Raumtemperatur abkühlen und stellt mit 2 N Natronlauge auf pH 7,2 ein. Die Lösung wird filtriert und anschließend gefriergetrocknet.
Ausbeute: 6,03 g (quantitativ) eines farblosen Pulvers
Wassergehalt: 7,5 %

| Elementaranalyse (bezogen auf wasserfreie Substanz): | | | | | | |
|---|---|---|---|---|---|---|
| ber. | C 30,23 | H 2,87 | F 27,03 | Gd 17,21 | N 6,13 | Na 2,52 |
| gef. | C 30,10 | H 3,05 | F 26,81 | Gd 17,15 | N 5,95 | Na 2,30 |

### Beispiel 9

a) 10-[2-Hydroxy-1H,1H,2H,3H,3H-perfluornonyl]-1,4,7-tris(carboxymethyl)-1,4,7,10-tetraazacyclododecan
Zu 15 g (43,3 mmol) 1,4,7-Tris(carboxymethyl)-1,4,7,10-tetraazacyclododecan in 50 ml Wasser gibt man 13,85 g (346,4 mmol) Natriumhydroxid. Hierzu tropft man eine Lösung aus 27,68 g (64,95 mmol) 1,2-Epoxy-1H,1H,2H,3H,3H-Perfluomonan gelöst in 50 ml n-Butanol/50 ml 2-Propanol und erwärmt die Lösung über Nacht auf 80°C. Man dampft im Vakuum zur Trockne ein, nimmt den Rückstand in 200 ml Wasser auf und stellt mit 3 N Salzsäure auf pH 3. Dann wird 2 mal mit 200 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 30,34 g (78 % d. Th.) eines glasigen Feststoffes
Wassergehalt: 13,7 %

| Elementaranalyse (bezogen auf wasserfreie Substanz): | | | | |
|---|---|---|---|---|
| ber. | C 37,32 | H 4,04 | F 36,89 | N 7,25 |
| gef. | C 37,15 | H 4,21 | F 36,70 | N 7,19 |

b) Gadolinium-Komplex von 10-[2-Hydroxy-1H,1H,2H,3H,3H-perfiuornonyl]-1,4,7-tris(carboxymethyl)-1,4,7,10-tetraazacyclododecan
10 g (12,94 mmol) der Titelverbindung aus Beispiel 9a) werden in 100 ml Wasser/50 ml Ethanol gelöst und 2,34 g (6,47 mmol) Gadoliniumoxid zugegeben. Man rührt 3 Stunden bei 80°C. Die Lösung wird filtriert und im Vakuum zur Trockne eingedampft.
Ausbeute: 13,16 g (quantitativ) eines farblosen glasigen Feststoffes
Wassergehalt: 9,1 %

| Elementaranalyse (bezogen auf wasserfreie Substanz): | | | | | |
|---|---|---|---|---|---|
| ber. | C 31,11 | H 3,05 | F 30,75 | Gd 16,97 | N 6,05 |
| gef. | C 31,01 | H 3,19 | F 30,55 | Gd 16,71 | N 5,88 |

### Beispiel 10

a) 9H,9H,10H,11H,12H,12H-Perfluoreicos-10-en
24,77 g (52,26 mmol) 1H,1H,2H,2H-Perfluorodecyl-1-jodid und 13,71 g (52,26 mmol) Triphenylphosphin werden in 500 ml Aceton unter Rühren auf 70°C erhitzt. Die anfangs klare Lösung wird rasch milchig trübe und scheidet das farblose Phosphoniumsalz ab. Man filtriert das Phosphoniumsalz ab, trocknet im Vakuum bei 40°C.
Ausbeute: 38,9 g (89 % d. Th.)
Dieses Phosphoniumsalz wird ohne Reinigung direkt in der folgenden Reaktion verwendet: Zum oben hergestellten Phosphoniumsalz, 38,9 g (46,5 mmol) in 250 ml Dichlormethan gibt man 5,22 g (46,5 mmol) Kalium-tert.-butylat, 0,20 g (0,75 mmol) 18-Krone-6 und 19,54 g (42,28 mmol) 2H,2H-Perfluordecanal zu und rührt 10 Stunden bei Raumtemperatur. Man dampft zur Trockne ein und chromatographiert den Rückstand an Kieselgel (Laufmittel: Dichlormethan/n-Hexan/Diethylether= 10/20/1).
Ausbeute: 30,3 g (65 % d. Th. bezogen auf eingesetztes Jodid) eines farblosen, wachsarten Feststoffes

| Elementaranalyse: | | | |
|---|---|---|---|
| ber. | C 26,92 | H 0,68 | F 72,40 |
| gef. | C 26,81 | H 0,79 | F 72,20 |

b) 10,11-Epoxy-9H,9H, 10H, 11H,12H,12H-perfluoreicosan
Zu 25 g (28,02 mmol) der Titelverbindung aus Beispiel 10a) gelöst in 250 ml Dichlormethan gibt man bei 0°C 10,47 g (36,42 mmol) 3-Chlorperoxybenzoesäure (ca. 60 %ig) und rührt über Nacht bei Raumtemperatur. Man setzt 300 ml 5 %ige aqu. Natriumcarbonat-Lösung zu und rührt gut durch. Die organische Phase wird abgetrennt, über Magnesiumsulfat getrocknet und im Vakuum zur Trockne eingedampft. Der Rückstand wird an Kieselgel chromatographiert (Laufmittel: n-Hexan/Dichlormethan/Diethylether= 10/10/1).
Ausbeute: 24,17 g (95 % d. Th.) eines farblosen Feststoffes

| Elementaranalyse: | | | |
|---|---|---|---|
| ber. | C 26,45 | H 0,67 | F 71,12 |
| gef. | C 26,25 | H 0,88 | F 71,35 |

c) 10-[1-(1H,1H-Perfluornonyl)-2-hydroxy-1H,2H,3H,3H-perfluorundecyl]-1,4,7-tris(carboxymethyl)-1,4,7,10-tetraazacyclododecan
Zu 7,63 g (22,02 mmol) 1,4,7-Tris(carboxymethyl)-1,4,7,10-tetraazacyclododecan in 35 ml Wasser gibt man 7,04 g (0,176 mmol) Natriumhydroxid. Hierzu tropft man eine Lösung aus 20 g (22,02 mmol) der Titelverbindung aus Beispiel 10b) gelöst in 50 ml n-Butanol/40 ml 2-Propanol und erwärmt die Lösung über Nacht auf 120°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 9,79 g (31 % d.Th.) eines farblosen, glasigen Feststoffes
Wassergehalt: 12,5 %

| Elementaranalyse (bezogen auf wasserfreie Substanz): | | | | |
|---|---|---|---|---|
| ber. | C 32,55 | H 2,57 | F 51,49 | N 4,47 |
| gef. | C 32,38 | H 2,75 | F 51,29 | N 4,28 |

d) Gadolinium-Komplex von 10-[1-(1H,1H-Perfluornonyl)-2-hydroxy-1H,2H,3H,3H-perfluorundecyl]-1,4,7-tris(carboxymethyl)-1,4,7,10-tetraazacyclododecan
8 g (6,38 mmol) der Titelverbindung aus Beispiel 10c) werden in 50 ml Wasser/40 ml Ethanol/20 ml Chloroform gelöst und 1,16 g (3,19 mmol) Gadoliniumoxid zugegeben. Man rührt 4 Stunden bei 90 °C im Autoklaven. Die Lösung wird filtriert und im Vakuum zur Trockne eingedampft.
Ausbeute: 9,47 g (quantitativ) eines glasigen Feststoffes
Wassergehalt: 5,2 %

| Elementaranalyse (bezogen auf wasserfreie Substanz): | | | | | |
|---|---|---|---|---|---|
| ber. | C 28,99 | H 2,07 | F 45,85 | Gd 11,16 | N 3,98 |
| gef. | C 28,81 | H 2,19 | F 45,71 | Gd 11,03 | N 4,12 |

### Beispiel 11

a) 7H,7H,8H,9H,10H,10H-Perfluorhexadec-8-en
18,7 g (50 mmol) 1H,1H,2H,2H-Perfluoroctyl-1-jodid und 13,11 g (50 mmol) Triphenyl phosphin werden in 400 ml Aceton unter Rühren auf 70°C erhitzt. Die anfangs klare Lösung wird rasch milchig trübe und scheidet das farblose Phosphoniumsalz ab. Man filtriert das Phosphoniumsalz ab, trocknet im Vakuum bei 40°C.
Ausbeute: 28,95 g (91 % d. Th.)
Dieses Phosphoniumsalz wird ohne Reinigung direkt in der folgenden Reaktion verwendet: Zum oben hergestellten Phosphoniumsalz, 28,95 g (45,5 mmol) in 200 ml Dichlormethan gibt man 5,05 g (45,5 mmol) Kalium-tert. butylat, 0,20 g (0,75 mmol) 18-Krone-6 und 14,98 g (41,36 mmol) der Titelverbindung aus Beispiel 8a) zu und rührt 10 Stunden bei Raumtemperatur. Man dampft zur Trockne ein und chromatographiert den Rückstand an Kieselgel (Laufmittel: Dichlormethan/n-Hexan/Diethylether= 10/20/1).
Ausbeute: 19,65 g (61 % d. Th.) eines farblosen wachsartigen Feststoffes

| Elementaranalyse: | | | |
|---|---|---|---|
| ber. | C 22,38 | H 0,94 | F 76,69 |
| gef. | C 22,20 | H 0,99 | F 76,51 |

b) 8,9-Epoxy-7H,7H,8H,9H,10H,10H-perfluorhexadecan
Zu 19 g (29,5 mmol) der Titelverbindung aus Beispiel 11a) gelöst in 200 ml Dichlormethan gibt man bei 0°C 11,03 g (38,35 mmol) 3-Chlorperoxybenzoesäure (ca. 60 %ig) und rührt über Nacht bei Raumtemperatur. Man setzt 300 ml 5 %ige aqu. Natriumcarbonat-Lösung zu und rührt gut durch. Die organische Phase wird abgetrennt, über Magnesiumsulfat getrocknet und im Vakuum zur Trockne eingedampft. Der Rückstand wird an Kieselgel chromatographiert (Laufmittel: n-Hexan/Dichlormethan/Diethylether= 10/10/1).
Ausbeute: 19,43 g (93 % d. Th.) eines farblosen Feststoffes

| Elementaranalyse: | | | |
|---|---|---|---|
| ber. | C 27,14 | H 0,85 | F 69,75 |
| gef. | C 27,01 | H 0,97 | F 69,60 |

c) 10-[1-(1H,1H-Perfluorheptyl)-2-hydroxy-1H,2H,3H,3H-perfluornonyl]-1,4,7-tris(carboxymethyl)-1,4,7,10-tetraazacyclododecan
Zu 9,3 g (26,83 mmol) 1,4,7-Tris(carboxymethyl)-1,4,7,10-tetraazacyclododecan in 50 ml Wasser gibt man 8,59 g (214,6 mmol) Natriumhydroxid. Hierzu tropft man eine Lösung aus 19 g (26,83 mmol) der Titelverbindung aus Beispiel 11b) gelöst in 70 ml n-Butanol/60 ml 2-Propanol und erwärmt die Lösung über Nacht auf 120°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 9,4 g (29 % d.Th.) eines glasigen Feststoffes
Wassergehalt: 12,7 %

| Elementaranalyse (bezogen auf wasserfreie Substanz): | | | | |
|---|---|---|---|---|
| ber. | C 34,17 | H 3,06 | F 46,84 | N 5,31 |
| gef. | C 33,98 | H 3,18 | F 46,65 | N 5,20 |

d) Gadolinium-Komplex von 10-[1-(1H,1H-Perfluorheptyl)-2-hydroxy-1H,2H,3H,3H-perfluornonyl]-1,4,7-tris(carboxymethyl)-1,4,7,10-tetraazacyclododecan
9 g (8,53 mmol) der Titelverbindung aus Beispiel 11c) werden in 60 ml Wasser/40 ml Ethanol/30 ml Chloroform gelöst und 1,54 g (4,27 mmol) Gadoliniumoxid zugegeben. Man rührt 4 Stunden bei 90°C im Autoklaven. Die Lösung wird filtriert und im Vakuum zur Trockne eingedampft.
Ausbeute: 11,45 g (quantitativ) eines farblosen, glasigen Feststoffes
Wassergehalt: 10,2 %

| Elementaranalyse (bezogen auf wasserfreie Substanz): | | | | | |
|---|---|---|---|---|---|
| ber. | C 29,81 | H 2,42 | F 40,86 | Gd 13,01 | N 4,63 |
| gef. | C 29,60 | H 2,60 | F 40,63 | Gd 12,84 | N 4,51 |

### Beispiel 12

a) 7,12-Dioxa-5H,5H,6H,6H,8H,8H,9H,10H,11H,11H,13H,13H,14H,14H-perfluoroctadec-9-en
30 g (91,74 mmol) 1H,1H,2H,2H-Perfluorhexyl-1-bromid werden in 100 ml Toluol gelöst dann 3,23 g (36,7 mmol) cis-1,4-Buten-diol und 1 g (2,95 mmol) Tetrabutylammoniumhydrogensulfat zugegeben. Man kühlt auf 0°C ab und fügt 16 g (400 mmol) feingepulvertes Natriumhydroxid zu. Dann wird 1 Stunde bei 0°C und über Nacht bei Raumtemperatur gerührt. Man filtriert vom Feststoff ab, wäscht das Filtrat zweimal mit je 200 ml Wasser, trocknet die organische Phase über Magnesiumsulfat und dampft dann im Vakuum zur Trockne ein. Der Rückstand wird an Kieselgel chromatographiert (Laufmittel: Dichlormethan/n-Hexan/Aceton= 15/15/1).
Ausbeute: 11,71 g (55 % d. Th. bezogen aufDiol) eines wachsartigen Feststoffes

| Elementaranalyse: | | | |
|---|---|---|---|
| ber. | C 33,12 | H 2,43 | F 58,93 |
| gef. | C 33,05 | H 2,61 | F 58,73 |

b) 9,10-Epoxy-7,12-dioxa-5H,5H,6H,6H,8H,8H,9H,10H,11H,11H,13H,13H,14H,14H-perfluoroctadecan
Zu 11 g (18,96 mmol) der Titelverbindung aus Beispiel 12a) gelöst in 100 ml Dichlormethan gibt man bei 0°C 7,08 g (24,64 mmol) 3-Chlorperoxybenzoesäure (ca. 60 %ig) und rührt über Nacht bei Raumtemperatur. Man setzt 150 ml 5 %ige aqu. Natriumcarbonat-Lösung zu und rührt gut durch. Die organische Phase wird abgetrennt, über Magnesiumsulfat getrocknet und im Vakuum zur Trockne eingedampft. Der Rückstand wird an Kieselgel chromatographiert (Laufmittel: n-Hexan/Dichlormethan/Diethylether= 10/10/1).
Ausbeute: 10,74 g (95 % d. Th.) eines farblosen Feststoffes

| Elementaranalyse: | | | |
|---|---|---|---|
| ber. | C 32,23 | H 2,37 | F 57,35 |
| gef. | C 32,13 | H 2,51 | F 57,20 |

c) 10-[1-(2-Oxa-1H, 1H,3H,3H,4H,4H-perfluoroctyl)-2-hydroxy-4-oxa-1H,2H,3H,3H,5H,5H,6H,6H-perfluordecyl]-1,4,7-tris(carboxymethyl)-1,4,7,10-tetraazacyclododecan
Zu 6,1 g (17,61 mmol) 1,4,7-Tris(carboxymethyl)-1,4,7,10-tetraazacyclododecan in 40 ml Wasser gibt man 5,63 g (141 mmol) Natriumhydroxid. Hierzu tropft man eine Lösung aus 10,5 g (17,61 mmol) der Titelverbindung aus Beispiel 12b) gelöst in 50 ml n-Butanol/40 ml 2-Propanol und erwärmt die Lösung über Nacht auf 120°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 4,96 g (27 % d.Th.) eines farblosen, glasigen Feststoffes
Wassergehalt: 9,7 %

| Elementaranalyse (bezogen auf wasserfreie Substanz): | | | | |
|---|---|---|---|---|
| ber. | C 38,27 | H 4,17 | F 36,32 | N 5,95 |
| gef. | C 38,12 | H 4,20 | F 36,20 | N 5,81 |

d) Gadolinium-Komplex von 10-[1-(2-Oxa-1H,1H,3H,3H,4H,4H-perfluoroctyl)-2-hydroxy-4-oxa- 1H,2H,3H,3H,5H,5H,6H,6H-perfluordecyl]-1,4,7-tris(carboxymethyl)-1,4,7,10-tetraazacyclododecan
4,7 g (5 mmol) der Titelverbindung aus Beispiel 12c) werden in 30 ml Wasser/ 30 ml Ethanol/20 ml Chloroform gelöst und 0,90 g (2,5 mmol) Gadoliniumoxid zugegeben. Man rührt 3,5 Stunden bei 90°C im Autoklaven. Die Lösung wird filtriert und im Vakuum zur Trockne eingedampft.
Ausbeute: 5,89 g (quantitativ) eines farblosen, glasigen Feststoffes
Wassergehalt: 7,1 %

| Elementaranalyse (bezogen auf wasserfreie Substanz): | | | | | |
|---|---|---|---|---|---|
| ber. | C 32,88 | H 3,31 | F 31,21 | Gd 14,35 | N 5,11 |
| gef. | C 32,67 | H 3,45 | F 31,04 | Gd 14,18 | N 5,02 |

### Beispiel 13

a) 1-Phenyl-2,6-dioxa-1H,1H,3H,3H,4H,5H,5H,7H,7H,8H,8H-perfluorhexadecan-4-ol
Zu 7,14 g (39,2 mmol) Glycerin-1-monobenzylether und 25 g (43,55 mmol) 1H,1H,2H,2H-Perfluordecyl-1-jodid in 100 ml Toluol gibt man 1 g (2,94 mmol) Tetrabutylammoniumhydrogensulfat und 15,6 g (390 mmol) feingepulvertes Natriumhydroxid. Man rührt 24 Stunden bei Raumtemperatur. Man trennt die organische Phase vom Feststoff ab und wäscht 2 mal mit je 5 %iger aqu. Salzsäure. Die organische Phase wird über Magnesiumsulfat getrocknet und im Vakuum zur Trockne eingedampft. Der Rückstand wird an Kieselgel chromatographiert (Laufmittel: n-Hexan/Aceton= 15/1).
Ausbeute: 19,95 g (81 % d. Th.) eines farblosen Öls

| Elementaranalyse: | | | |
|---|---|---|---|
| ber. | C 38,23 | H 2,73 | F 51,40 |
| gef. | C 38,10 | H 2,89 | F 51,25 |

b) 1-Phenyl-4-(decyloxy)-2,6-dioxa-1H,1H,3H,3H,4H,5H,5H,7H,7H,8H,8H-perfluorhexadecan
Zu 19,5 g (31,03 mmol) der Titelverbindung aus Beispiel 13a) gelöst in 100 ml Dimethylformamid gibt man portionsweise 1,12 g (37,24 mmol) Natriumhydrid (80 %ige Suspension in Mineralöl) und rührt 2 Stunden bei Raumtemperatur. Anschließend werden 8,24 g (37,24 mmol) n-Decylbromid zugegeben und über Nacht bei 50°C gerührt. Man gibt 150 ml Eiswasser zu und extrahiert 2 mal mit je 150 ml Essigsäureethylester. Die vereinigten organischen Phasen werden 2 mal mit je 150 ml Wasser gewaschen, über Magnesiumsulfat getrocknet und zur Trockne eingedampft. Der Rückstand wird an Kieselgel chromatographiert (Laufmittel: n-Hexan/Aceton= 20:1).
Ausbeute: 22,66 g (95 % d. Th.) eines wachsartigen Feststoffes

| Elementaranalyse: | | | |
|---|---|---|---|
| ber. | C 46,88 | H 4,85 | F 42,02 |
| gef. | C 46,64 | H 4,97 | F 41,87 |

c) 2-(Decyloxy)-4-oxa-1H,1H,2H,3H,3H,5H,5H,6H,6H-perfluortetradecan-1-ol
20 g (26,02 mmol) der Titelverbindung aus Beispiel 13b) werden in 200 ml Isopropanol gelöst und 3 g Palladium-Katalysator (10 % Pd/C) zugegeben. Man hydriert über Nacht bei Raumtemperatur. Der Katalysator wird abfiltriert und das Filtrat im Vakuum zur Trockne eingeengt.
Ausbeute: 17,65 g (quantitativ) eines farblosen Feststoffes

| Elementaranalyse: | | | |
|---|---|---|---|
| ber. | C 40,72 | H 4,61 | F 47,60 |
| gef. | C 40,55 | H 4,76 | F 47,43 |

d) 1,2-Epoxy-4-oxa-6-(decyloxy)-8-oxa-1H,1H,2H,3H,3H,5H,5H,6H,7H,7H,9H,9H,10H,10H-perfluoroctadecan
Zu einer Mischung aus 17 g (25,06 mmol) der Titelverbindung aus Beispiel 13c) und 2 g (5,89 mmol) Tetrabutylammoniumhydrogensulfat in 300 ml 60%iger aqu. Kalilauge/ 100 ml Toluol tropft man unter starkem Rühren bei 10°C 9,25 g (100 mmol) Epichlorhydrin zu und achtet darauf, daß die Temperatur der Reaktionslösung nicht über 20°C steigt. Man läßt 2 Stunden bei 15°C rühren und tropft anschließend wie oben beschrieben 4,63 g (50 mmol) Epichlorhydrin zu. Dann wird über Nacht bei Raumtemperatur gerührt. Man gibt 100 ml Toluol und Methyl-tert.-butylether zu und trennt die wässrige Phase ab. Diese wird 2 mal mit je 100 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 14,91 g (81 % d. Th.) eines farblosen Feststoffes

| Elementaranalyse: | | | |
|---|---|---|---|
| ber. | C 42,51 | H 4,80 | F 43,97 |
| gef. | C 42,37 | H 4,96 | F 43,68 |

e) 10-[2-Hydroxy-4,8-dioxa-6-(decyloxy)-1H,1H,2H,3H,3H,5H,5H,6H,7H,7H,9H,9H,10H,10H-perfluoroctadecyl]-1,4,7-tris(carboxymethyl)-1,4,7,10-tetraazacyclododecan
Zu 6,6 g (19,06 mmol) 1,4,7-Tris(carboxymethyl)-1,4,7,10-tetraazacyclodo- decan in 60 ml Wasser gibt man 6,11 g (152,8 mmol) Natriumhydroxid. Hierzu tropft man eine Lösung aus 14 g (19,06 mmol) der Titelverbindung aus Beispiel 13d) gelöst in 80 ml n-Butanol/40 ml 2-Propanol und erwärmt die Lösung über Nacht auf 80°C im Autoklaven. Man dampft im Vakuum zur Trockne ein, nimmt den Rückstand in 200 ml Wasser auf und stellt mit 3 N Salzsäure auf pH 3. Dann wird 2 mal mit 300 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 17,88 g (76 % d.Th.) eines glasigen Feststoffes
Wassergehalt: 12,5 %

| Elementaranalyse (bezogen auf wasserfreie Substanz): | | | | |
|---|---|---|---|---|
| ber. | C 44,49 | H 5,60 | F 29,91 | N 5,19 |
| gef. | C 44,31 | H 5,75 | F 29,70 | N 5,03 |

f) Gadoliniumkomplex von 10-[2-Hydroxy-4,8-dioxa-6-(decyloxy)-1H,1H,2H,3H,3H,5H,5H,6H,7H,7H,9H,9H,10H,10H-perfluoroctadecyl]-1,4,7-tris(carboxymethyl)-1,4,7,10-tetraazacyclododecan
10 g (9,26 mmol) der Titelverbindung aus Beispiel 13e) werden in 30 ml Wasser/100 ml Ethanol/30 ml Chloroform gelöst und 1,68 g (4,63 mmol) Gadoliniumoxid zugegeben. Man rührt 3,5 Stunden bei 90°C im Autoklaven. Die Lösung wird filtriert und im Vakuum zur Trockne eingedampft.
Ausbeute: 12,39 g (quantitativ) eines farblosen, glasigen Feststoffes
Wassergehalt: 7,8 %

| Elementaranalyse (bezogen auf wasserfreie Substanz): | | | | | |
|---|---|---|---|---|---|
| ber. | C 38,93 | H 4,66 | F 26,17 | Gd 12,74 | N 4,54 |
| gef. | C 38,71 | H 4,82 | F 26,01 | Gd 12,55 | N 4,38 |

### Beispiel 14

a) 1-Phenyl-2-oxa-4,4,4-tris(2-oxa-1H,1H,3H,3H,4H,4H-perfluordecyl)-butan
Zu 4,24 g (18,74 mmol) Pentaerythrit-monobenzylether und 40 g (93,7 mmol) 1H,1H,2H,2H-Perfluoroctyl-1-bromid in 150 ml Toluol gibt man 2 g (5,89 mmol) Tetrabutylammoniumhydrogensulfat und 22,48 g (562 mmol) feingepulvertes Natriumhydroxid. Man rührt 24 Stunden bei Raumtemperatur. Man trennt die organische Phase vom Feststoff ab und wäscht 2 mal mit je 5 %iger aqu. Salzsäure. Die organische Phase wird über Magnesiumsulfat getrocknet und im Vakuum zur Trockne eingedampft. Der Rückstand wird an Kieselgel chromatographiert (Laufmittel: n-Hexan/Aceton= 25/1).
Ausbeute: 14,45 g (61 % d. Th. bezogen auf den Benzylether) eines farblosen, wachsarten Feststoffes

| Elementaranalyse: | | | |
|---|---|---|---|
| ber. | C 34,19 | H 2,15 | F 58,59 |
| gef. | C 34,02 | H 2,31 | F 58,41 |

b) 2,2,2-Tris(2-oxa-1H,1H,3H,3H,4H,4H-perfluordecyl)-ethan-1-ol
14 g (11,07 mmoL) der Titelverbindung aus Beispiel 14a) werden in 100 ml Isopropanol/100 ml Tetrahydrofuran gelöst und 3 g Palladium-Katalysator (10 % Pd/C) zugegeben. Man hydriert über Nacht bei Raumtemperatur. Der Katalysator wird abfiltriert und das Filtrat im Vakuum zur Trockne eingeengt.
Ausbeute: 13 g (quantitativ) eines farblosen Feststoffes

| Elementaranalyse: | | | |
|---|---|---|---|
| ber. | C 29,66 | H 1,80 | F 63,09 |
| gef. | C 29,45 | H 1,97 | F 62,91 |

c) 1,2-Epoxy-4-oxa-6,6,6-tris(2-oxa-1H,1H,3H,3H,4H,4H-perfluordecyl)-hexan
Zu einer Mischung aus 12,5 g (10,64 mmol) der Titelverbindung aus Beispiel 14b) und 1 g (2,95 mmol) Tetrabutylammoniumhydrogensulfat in 150 ml 60%iger aqu. Kalilauge/ 50 ml Toluol tropft man unter starkem Rühren bei 10°C 3,94 g (42,57 mmol) Epichlorhydrin zu
c) 1,2-Epoxy-4-oxa-6,6,6-tris(2-oxa-1H,1H,3H,3H,4H,4H-perfluordecyl)-hexan
Zu einer Mischung aus 12,5 g (10,64 mmol) der Titelverbindung aus Beispiel 14b) und 1 g (2,95 mmol) Tetrabutylammoniumhydrogensulfat in 150 ml 60%iger aqu. Kalilauge/50 ml Toluol tropft man unter starkem Rühren bei 10°C 3,94 g (42,57 mmol) Epichlorhydrin zu und achtet darauf, daß die Temperatur der Reaktionslösung nicht über 20°C steigt. Man läßt 2 Stunden bei 15°C rühren und tropft anschließend wie oben beschrieben 1,97 g (21,29 mmol) Epichlorhydrin zu. Dann wird über Nacht bei Raumtemperatur gerührt. Man gibt 100 ml Toluol und 100 ml Methyl-tert.-butylether zu und trennt die wässrige Phase ab. Diese wird 2 mal mit je 50 ml Toluol nachextrahiert. Die organischen Phasen werden vereinigt, über Magnesiumsulfat getrocknet und im Vakuum eingedampft. Der Rückstand wird an Kieselgel chromatographiert (Laufmittel: Dichlormethan/Hexan/Aceton= 20/10/1).
Ausbeute: 8,12 g (62 % d. Th.) eines farblosen Feststoffes

| Elementaranalyse: | | | |
|---|---|---|---|
| ber. | C 31,24 | H 2,05 | F 60,22 |
| gef. | C 31,09 | H 2,19 | F 60,10 |

d) 10-[2-Hydroxy-4-oxa-6,6,6-tris(2-oxa-1H,1H,3H,3H,4H,4H-perfluordecyl)-hexyl]-1,4,7-tris(carboxymethyl)-1,4,7,10-tetraazacyclododecan
Zu 2,25 g (6,50 mmol) 1,4,7-Tris(carboxymethyl)-1,4,7,10-tetraazacyclododecan in 30 778 → 30 ml Wasser gibt man 2,08 g (52 mmol) Natriumhydroxid. Hierzu tropft man eine Lösung aus 8,0 g (6,50 mmol) der Titelverbindung aus Beispiel 14c) gelöst in 50 ml n-Butanol/30 ml 2-Propanol und erwärmt die Lösung über Nacht auf 100°C im Autoklaven. Man dampft im Vakuum zur Trockne ein, nimmt den Rückstand in 200 ml Wasser auf und stellt mit 3 N Salzsäure auf pH 3. Dann wird 2 mal mit 100 ml n-Butanol extrahiert. Die vereinigten Butanolphasen werden im Vakuum zur Trockne eingeengt und der Rückstand durch RP-Chromatographie gereinigt (RP-18/Laufmittel: Gradient aus Wasser/n-Butanol/Acetonitril).
Ausbeute: 7,79 g (67 % d.Th.) eines farblosen, glasigen Feststoffes
Wassergehalt: 11,9%
e) Gadolinium-Komplex von 10-[2-Hydroxy-4-oxa-6,6,6-tris(2-oxa-1H,1H,3H,3H,4H,4H-perfluordecyl)-hexyl]-1,4,7-tris(carboxymethyl)-1,4,7,10-tetraazacyclododecan
7 g (4,44 mmol) der Titelverbindung aus Beispiel 14d) werden in 30 ml Wasser/50 ml Ethanol/50 ml Chloroform gelöst und 0,80 g (2,22 mmol) Gadoliniumoxid zugegeben. Man rührt 5 Stunden bei 90°C im Autoklaven. Die Lösung wird filtriert und im Vakuum zur Trockne eingedampft.
Ausbeute: 8,34 g (quantitativ) eines farblosen, glasigen Feststoffes
Wassergehalt: 8,1 %

| Elementaranalyse (bezogen auf wasserfreie Substanz): | | | | | |
|---|---|---|---|---|---|
| ber. | C 31,94 | H 2,74 | F 42,83 | Gd 9,09 | N 3,24 |
| gef. | C 31,74 | H 2,91 | F 42,67 | Gd 8,85 | N 3,15 |

### Beispiel 15

a) 1,7-Bis[acetyl-(2-(N-ethyl-N-perfluoroctylsulfonylamino)]-1,4,7-triazaheptan
20 g (34,17 mmol) der Titelverbindung aus Beispiel 1b) und 4,33 g (37,59 mmol) N-Hydroxysuccinimid werden in 150 ml Dimethylformamid gelöst. Bei 0°C gibt man 7,76 g (37,59 mmol) Dicyclohexylcarbodiimid hinzu und rührt 3 Stunden bei Raumtemperatur Man filtriert vom Dicyclohexylharnstoff ab und tropft das Filtrat zu einer Lösung aus 1,76 g (17,09 mmol) Diethylentriamin und 13,83 g (136,7 mmol) Triethylamin in 200 ml Dimethylformamid bei Raumtemperatur. Man rührt über Nacht bei Raumtemperatur. Es wird im Vakuum zur Trockne eingedampft und der Rückstand in 200 ml 5 %iger aqu. Soda-Lösung aufgenommen. Man extrahiert 2 mal mit je 150 ml Dichlormethan, trocknet die vereinigten organischen Phasen über Magnesiumsulfat und dampft im Vakuum zur Trockne ein. Der Rückstand wird an Kieselgel chromatographiert (Laufmittel: Dichlormethan/2-Propanol= 20/1).
Ausbeute: 16,5 g (78 % d. Th.) eines wachsartigen Feststoffes

| Elementaranalyse: | | | | | |
|---|---|---|---|---|---|
| ber. | C 27,17 | H 2,04 | F 52,19 | N 5,66 | S 5,18 |
| gef. | C 27,03 | H 2,17 | F 52,04 | N 5,49 | S 5,07 |

b) 4-(3-Carboxy-propanoyl)-1,7-bis-{acetyl-[2-(N-ethyl-N-perfluoroctylsulfonylamino)] }-1,4,7-triazaheptan
Zu 16 g (12,93 mmol) der Titelverbindung aus Beispiel 15a) in 100 ml Methylenchlorid gibt man 3,92 g (38,78 mmol) Triethylamin und kühlt die Lösung auf 0°C ab. Dann gibt man 2,59 g (25,86 mmol) Bernsteinsäureanhydrid zu und rührt 3 Stunden bei 0°C, über Nacht bei Raumtemperatur. Man gibt 200 ml 5 %ige aqu. Salzsäure zu und schüttelt gut durch. Die organische Phase wird abgetrennt und über Magnesiumsulfat getrocknet. Man dampft im Vakuum zur Trockne ein und chromatographiert der Rückstand an Kieselgel (Laufmittel: Dichlormethan/2-Propanol= 15/1).
Ausbeute: 15,74 g (91 % d. Th.) eines farblosen Feststoffes

| Elementaranalyse: | | | | | |
|---|---|---|---|---|---|
| ber. | C 28,73 | H 2,19 | F 48,29 | N 5,24 | S 4,79 |
| gef. | C 28,58 | H 2,40 | F 48,17 | N 5,17 | S 4,65 |

c) 10-[7-Hydroxy-5-aza-4-oxo-octansäure-N,N-bis(3-aza-4-oxo-6-aza-6-(perfluoroctylsulfonyl)-octyl)-amid]-1,4,7-tris(carboxymethyl)-1,4,7,10-tetraazacyclododecan 15 g (11,21 mmol) der Titelverbindung aus beispiel 15b) und 1,42 g (12,33 mmol) N-Hydroxysuccinimid werden in einer Mischung aus 80 ml Dimethylformamid/30 ml Chloroform gelöst. Bei 0°C gibt man 2,54 g (12,33 mmol) Dicyclohexylcarbodiimid zu und rührt 1 Stunde bei 0°C, anschließend 3 Stunden bei Raumtemperatur. Man kühlt erneut auf 0°C und gibt 4,05 g (40 mol) Triethylamin/50 ml 2-Propanol zu. Anschliessend werden 7,07 g (12,33 mmol) Gadolinium-Komplex von 10-[2-Hydroxy-3-amino-propyl]-1,4,7-tris(carboxymethyl)-1,4,7,10-tetraazacyclododecan gelöst in 30 ml Wasser zugegeben und 3 Stunden bei Raumtemperatur gerührt. Man dampft zur Trockne ein, nimmt den Rückstand in einer Mischung aus 100 ml Methanol/ 50 ml Chloroform auf und filtriert vom Dicyclohexylharnstoff ab. Das Filtrat wird zur Trockne eingedampft und durch RP-Chromatographie gereinigt (RP-18/Laufmittel: Gradient aus Wasser/n-Propanol/Acetonitril).
Ausbeute: 17,76 g (78 % d. Th.) eines farblosen, glasigen Feststoffes
Wassergehalt: 6,8 %

| Elementaranalyse (bezogen auf wasserfreie Substanz): | | | | | | |
|---|---|---|---|---|---|---|
| ber.: | C 31,08 | H 3,03 | F 34,12 | Gd 8,31 | N 7,40 | S 3,39 |
| gef. | C 30,89 | H 3,15 | F 34,01 | Gd 8,14 | N 7,25 | S 3,24 |

### Beispiel 16

### Gadoliniumkomplex von 1,4,7-Tris(carboxylatomethyl)-10-(2-hydroxy-19,19,20,20,-21,21,22,22,23,23,24,24,25,25,26,26,26-heptadecafluor-4,7,10,13,16-penta-oxahexacosan)-1,4,7,10-tetraazacyclododecan

a) 16,16,17,17,18,18,19,19,20,20,21,21,22,22,22-Heptadecafluor-3,6,9,12-tetra-oxadocosan-1-ol
Eine Mischung aus 20 g (32,35 mmol) 1-p-Toluolsulfonyloxy-1H,1H,2H,2H-perfluordecan [s. Beispiel 7a], 1 g Tetrabutylammoniumhydrogensulfat, 62,83 g (323,5 mmol) Tetraethylenglykol, 300 ml Dichlormethan und 100 ml 50 %ige Natronlauge wird 24 Stunden intensiv bei ca. 5 °C gerührt. Man verdünnt dann mit 200 ml Dichlormethan, trennt die Phasen und wäscht die Dichlormethanphase mit Wasser. Die organische Phase wird über Magnesiumsulfat getrocknet und im Vakuum eingedampft. Man erhält 18,5 g der gewünschten Titelverbindung als hellgelbes Öl.
b) 1,2-Epoxy-19,19,20,20,21,21,22,22,23,23,24,24,25,25,26,26,26-heptadecafluor4,7,10,13,16-penta-oxa-hexacosan
Eine Mischung aus 17 g (26,5 mmol) 16,16,17,17,18,18,19,19,20,20,21,21,22,22,22-Heptadecafluor-3,6,9,12-tetra-oxa-docosan-1-ol, 0,5 g Tetrabutylammoniumhydrogensulfat, 2,94 g Epichlorhydrin, 200 ml Dichlormethan und 50 ml 50 %ige Natronlauge wird 8 Stunden intensiv bei Raumtemperatur gerührt. Man trennt die Phasen, schüttelt die wäßrige Phase mit 100 ml Dichlormethan, vereinigt die organischen Phasen, schüttelt mit 50 ml Wasser, trocknet über Magnesiumsulfat und dampft im Vakuum ein. Den Rückstand chromatographiert man an Kieselgel mit Hexan / 5 - 50 % Ethylacetat und erhält 12,92 g der Titelverbindung als Öl.

| Elementaranalyse: | | | |
|---|---|---|---|
| ber. | C 36,22 | H 3,62 | F 46,38 |
| gef. | C 36,00 | H 3,78 | F 46,20 |

c) 1,4,7-Tris(carboxylatomethyl)-10-(2-hydroxy-19,19,20,20,21,21,22,22,23,23,24,24,-25,25,26,26,26-heptadecafluor-4,7,10,13,16-penta-oxa-hexacosan)-1,4,7,10-tetraazacyclododecan
Zu einer Lösung von 6 g (17,3 mmol) 1,4,7-(Triscarboxylatomethyl)-1,4,7,10-tetraazacyclododecan und 4 g Natriumhydroxid in 30 ml Wasser gibt man eine Lösung von 12,05 g (17,3 mmol) 1,2-Epoxy-19,19,20,20,21,21,22,22,23,23,24,24,25,25,26,26,26-heptadecafluor-4,7,10,13,16-penta-oxa-hexacosan in 50 ml Tetrahydrofuran. Man rührt über Nacht bei 70 °C, dampft im Vakuum dann weitgehend ein, nimmt den Rückstand in 150 ml Wasser auf und stellt mit 6N Salzsäure auf pH3 und extrahiert mehrmals mit n-Butanol. Die vereinigten Extrakte werden im Vakuum eingedampft, und der Rückstand wird durch Chromatographie an RP-18 mit einem Gradienten aus Wasser/n-Butanol/Acetonitril gereinigt. Man erhält 13,71 g der Titelverbindung als gelbes viskoses Öl.

| Elementaranalyse | | | | |
|---|---|---|---|---|
| ber. | C 40,31 | H 4,93 | F 30,97 | N 5,37 |
| gef. | C 40,08 | H 5,21 | F 30,77 | N 5,29 |

d) Gadoliniumkomplex von 1,4,7-Tris(carboxylatomethyl)-10-(2-hydroxy-19,19,20,20,-21,21,22,22,23,23,24,24,25,25,26,26,26-heptadecafluor-4,7,10,13,16-penta-oxa-hexacosan)-1,4,7,10-tetraazacyclododecan
Man versetzt eine Mischung aus 5 g (4,79 mmol) 1,4,7-Tris(carboxylatomethyl)-10-(2-hydroxy-19,19,20,20,21,21,22,22,23,23,24,24,25,25,26,26,26-heptadecafluor-4,7,10,13,16-penta-oxa-hexacosan)-1,4,7,10-tetraazacyclododecan, 50 ml Wasser und 30 ml Ethanol mit 869 mg (2,397 mmol) Gadoliniumoxid und erhitzt 5 Stunden unter Rückfluß. Man filtriert die heiße Lösung und dampft im Vakuum ein. Man erhält 5,60 g der Titelverbindung als glasigen festen Stoff mit einem Wassergehalt von 4,1 %.

| Elementaranalyse (bezogen auf wasserfreie Substanz): | | | | | |
|---|---|---|---|---|---|
| ber. | C 35,12 | H 4,04 | F 26,98 | Gd 13,14 | N 4,68 |
| gef. | C34,90 | H 4,38 | F 26,70 | Gd 13,10 | N 4,62 |

### Beispiel 17

### Gadoliniumkomplex von 1,4,7-Tris(carboxylatomethyl)-10-(4-aza-2-hydroxy-26,26,26,25,25,24,24,23,23,22,22,21,21,20,20,19,19-heptadecafluor-5-oxo-16-thiahexacosyl)-1,4,7,10-tetraazacyclododecan

a) 22,22,22,21,21,20,20,19,19,18,18,17,17,16,16,15,15,-Heptadecafluor-12-thiadocosansäure
Man versetzt eine Lösung von 10 g (37,71 mmol) 11-Bromundecansäure in 150 ml Dichlormethan mit 11,43 g Triethylamin und 18,11 g (37,71 mmol) 1H,1H,2H,2H-Perfluordecylmercaptan und rührt über Nacht bei Raumtemperatur. Man extrahiert die Lösung mehrmals mit 2N Salzsäure, wäscht mit Kochsalzlösung, trocknet über Magnesiumsulfat und dampft im Vakuum ein. Man erhält 21,5 g der Titelverbindung als gelbes Öl.

| Elementaranalyse: | | | | |
|---|---|---|---|---|
| ber. | C 37,96 | H 3,79 | F 48,61 | S 4,83 |
| gef. | C 38,30 | H 4,01 | F 48,40 | S 5,20 |

b) Gadoliniumkomplex von 1,4,7-Tris(carboxylatomethyl)-10-(4-aza-2-hydroxy-26,26,26,25,25,24,24,23,23,22,22,21,21,20,20,19,19-heptadecafluor-5-oxo-16-thiahexacosyl)-1,4,7,10-tetraazacyclododecan
5 g (7,52 mmol) der Titelverbindung aus Beispiel 17a) und 0,95 g N-Hydroxysuccinimid werden in einer Mischung aus 25 ml Dimethylformamid und 15 ml Chloroform gelöst. Bei 0 °C gibt man 1,71 g Dicyclohexylcarbodiimid zu und rührt 1 Stunde bei 0 °C, dann 3 Stunden bei Raumtemperatur. Man kühlt dann wieder auf 0 °C und versetzt mit 3 ml Triethylamin und 20 ml n-Propanol. Anschließend werden 4,75 g (8,27 mmol) des Gadolinium-Komplexes von 10-(3-Amino-2-hydroxy-propyl)-1,4,7-tris(carboxylatomethyl)-1,4,7,10-tetraazacyclododecan gelöst in 25 ml Wasser zugegeben und 3 Stunden bei 20 °C gerührt.
Man dampft zur Trockne ein, nimmt den Rückstand in einer Mischung aus 55 ml Methanol und 20 ml Chloroform auf und filtriert vom Dicyclohexylharnstoff ab. Das Filtrat dampft man zur Trockne ein und reinigt durch Chromatographie an RP-18 mit einem Gradienten aus Wasser/n-Propanol/Acetonitril. Man erhält 6,15 g der Titelverbindung als glasigen Feststoff, mit einem Wassergehalt von 2,3 %.

| Elementaranalyse (berechnet auf wasserfreie Substanz): | | | | | | |
|---|---|---|---|---|---|---|
| ber. | C 37,41 | H 4,38 | F 26,47 | Gd 12,89 | N 5,74 | S 2,63 |
| gef. | C 37,08 | H 4,60 | F 26,30 | Gd 12,68 | N 5,91 | S 2,49 |

### Beispiel 18

### Gadolinium-Komplex von 1,4,7-Tris(carboxylatomethyl)-10-[1-(1,2-dihydroxy- ethyl)3-oxa-6,6,7,7,8,8,9,9,10,10,11,11,11-tridecafluor]undecan-1,4,7,10-tetraazacyclododecan

a) 1-p-Toluolsulfonyloxy-1H,1H,2H,2H-perfluoroctan
Zu einer Lösung von 25 g (68,7 mmol) 1H,1H,2H,2H-Perfluoroctan-1-ol in 300 ml Dichlormethan gibt man bei 0 °C 20 ml Pyridin und trägt unter Rühren in Portionen 13,49 g (70,76 mmol) p-Toluolsulfonsäurechlorid ein. Man rührt noch 3 Stunden bei 0 °C, zieht bei Raumtemperatur im Vakuum das Dichlormethan ab. Die zurückbleibende Pyridinlösung versetzt man mit Eiswasser, wobei das gewünschte Produkt ausfällt. Mandekantiert und löst den Rückstand in Dichlormethan, wäscht die Lösung mit Wasser, trocknet über Magnesiumsulfat und dampft im Vakuum ein. Der Rückstand wird durch Chromatographie an Kieselgel mit Hexan / 5 - 40 % Ethylacetat gereinigt. Man erhält 29,2 g der Titelverbindung als zähen Schaum.

| Elementaranalyse: | | | | |
|---|---|---|---|---|
| ber. | C 34,76 | H 2,14 | F 47,65 | S 6,19 |
| gef. | C 34,98 | H 2,38 | F 47,39 | S 6,42 |

b) 1,4,7-Tris(Benzyloxycarbonyl)-10-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan
Zu 7,33 g (10mmol) 1,4,7-Tris(Benzyloxycarbonyl)-10-[2-hydroxy-1-(2,2-dimethyl-1,3-dioxolan-4-yl)]-ethyl-1,4,7,10-tetraazacyclododecan [J. Mag. Res. Imag. 5: 7-10, (1955)] gelöst in 100 ml Dichlormethan gibt man nacheinander 20 ml 50 %ige Natronlauge, 0,5 g Tetrabutylammoniumhydrogensulfat und 5,18 g (10 mmol) 1-p-Toluol-sulfonyloxy-1H,1H,2H,2H-perfluoroctan [s. Beispiel 18a)] und rührt die Mischung intensiv über Nacht bei Raumtemperatur. Man trennt die Phasen, wäscht die organische Phase mehrmals mit Wasser, trocknet über Magnesiumsulfat und dampft im Vakuum ein. Den Rückstand reinigt man durch Chromatographie an Kieselgel mit Dichlormethan / 1 - 10 % Ethanol. Man erhält 8,02 g der Titelverbindung als zähes Öl.

| Elementaranalyse: | | | | |
|---|---|---|---|---|
| ber. | C 53,01 | H 5,02 | F 23,19 | N 5,26 |
| gef. | C 53,30 | H 5,39 | F 23,01 | N 5,40 |

c) 1-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan
Eine Lösung von 7 g (6,57 mmol) 1,4,7-Tris(Benzyloxycarbonyl)-10-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan in 100 ml Isopropylalkohol versetzt man mit 0,7 g Palladium auf Kohle (10 %ig) und schüttelt 3 Stunden unter einer Wasserstoffatmosphäre. Man filtriert vom Katalysator und dampft die Lösung im Vakuum ein. Man erhält 4,20 g der Titelverbindung als glasigen Schaum.

| Elementaranalyse: | | | | |
|---|---|---|---|---|
| ber. | C 41,70 | H 5,32 | F 37,28 | N 8,46 |
| gef. | C 41,61 | H 5,57 | F 37,10 | N 8,59 |

d) 1,4,7-Tris(carboxylatomethyl)-10-[1-(1,2-dihydroxy-ethyl)-3-oxa-6,6,7,7,8,8,9,9,-10,10,11,11,11 1-tridecafluor]undecan-1,4,7,10-tetraazacyclododecan
Man löst 3,36 g (24,15 mmol) Bromessigsäure in 50 ml Wasser und versetzt mit 6N Natronlauge bis pH7. Bei 40 °C tropft man unter Rühren gleichzeitig eine Lösung von 4 g (6,04 mmol) 1-[1-(2,2-dimethyl-1,3-dioxolan-4-yl)-6,6,7,7,8,8,9,9,10,10,11,11,11-tridecafluor-3-oxa]-undecan-1,4,7,10-tetraazacyclododecan, gelöst in 20 ml Isopropylalkohol, und soviel 6N Natronlauge zu, daß der pH bei 9-10 gehalten wird. Anschließend versetzt man mit halbkonzentrierter Salzsäure bis zu einem pH 1 und rührt weitere 3 Stunden bei 60 °C. Man kühlt auf Raumtemperatur ab und extrahiert die Lösung mehrmals mit n-Butanol. Der organische Extrakt wird eingedampft und der Rückstand durch Chromatographie an RP-18 mit einem Gradienten aus Wasser / n-Butanol / Acetonitril gereinigt. Man erhält 3,85 g der Titelverbindung als gelbes Öl mit einem Wassergehalt von 3,9 %.

| Elementaranalyse (bezogen auf wasserfreie Substanz): | | | | |
|---|---|---|---|---|
| ber. | C 39,20 | H 4,68 | F 31,00 | N 7,03 |
| gef. | C 39,08 | H 4,98 | F 30,72 | N 7,29 |

e) Gadolinium-Komplex von 1,4,7-Tris(carboxylatomethyl)-10-[1-(1,2-dihydroxy-ethyl)-3-oxa-6,6,7,7,8,8,9,9,10,10,11,11,11 1-tridecafluor]undecan-1,4,7,10-tetraazacyclododecan
Man versetzt eine Mischung aus 1,59 g (2 mmol) 1,4,7-Tris(carboxylatomethyl)-10-[1-(1,2-dihydroxy-ethyl)-3-oxa-6,6,7,7,8,8,9,9,10,10,11,11,11-tridecafluor] undecan-1,4,7,10-tetraazacyclododecan, 25 ml Wasser und 15 ml Ethanol mit 363 mg (1 mmol) Gadoliniumoxid und erhitzt 5 Stunden unter Rückfluß. Man filtriert die heiße Lösung, dampft im Vakuum ein und erhält 1,85 g der Titelverbindung als glasigen festen Stoff mit einem Wassergehalt von 4,2 %.

| Elementaranalyse (bezogen auf wasserfreie Substanz): | | | | | |
|---|---|---|---|---|---|
| ber. | C 32,84 | H 3,60 | F 25,98 | Gd 16,54 | N 5,89 |
| gef. | C 32,53 | H 3,71 | F 25,72 | Gd 16,39 | N 5,93 |

### Beispiel 19

### Gadolinium-Komplex von 1,4,7-Tris(carboxylatomethyl)-10-{2-hydroxy-4-oxa-4-[4-(2H,2H,3H,3H-1-oxa-perfluorundec-1-yl)]-phenyl}-but-1-yl-1,4,7,10-tetraazacyclododecan

a) 1-Hydroxy-4-(2H,2H,3H,3H-1-oxa-perfluorundec-1-yl)-benzol
5 g (45,41 mmol) Hydrochinon werden mit 100 ml Aceton versetzt und unter Rühren nacheinander mit 13,8 g Kaliumcarbonat und 14,04 g (22,7 mmol) 1-p-Toluolsulfonyloxy-1H,1H,2H,2H-perfluordecan [s. Beispiel 7a)] versetzt. Man erhitzt 6 Stunden unter Rückfluß, engt dann im Vakuum weitgehend ein, verdünnt mit 200 ml Wasser, stellt mit Zitronensäure auf pH3 und extrahiert mehrmals mit Dichlormethan. Der organische Extrakt wird über Magnesiumsulfat getrocknet und im Vakuum eingedampft. Der Rückstand wird durch Chromatographie an Kieselgel mit Hexan / 5 - 30 % Ethylacetat gereinigt. Man erhält 8,20 g der gewünschten Titelverbindung als zähes Öl.

| Elementaranalyse: | | | |
|---|---|---|---|
| ber. | C 34,55 | H 1,63 | F 58,07 |
| gef. | C 34,31 | H 1,79 | F 58,01 |

b) 1-(3,4-Epoxy-1-oxa-but-1-yl)-4-(2H,2H,3H,3H-1-oxa-perfluorundec-1-yl)- benzol
Eine Mischung aus 8 g (14,38 mmol) 1-Hydroxy-4-(2H,2H,3H,3H-1-oxa-perfluorundec-1-yl)-benzol, 0,4 g Tetrabutylammoniumhydrogensulfat, 1,60 g (17,26 mmol) Epichlorhydrin, 150 ml Dichlormethan und 30 ml 50 %ige Natronlauge wird 30 Minuten im Eisbad, dann 5 Stunden bei Raumtemperatur intensiv gerührt. plan trennt die Phasen, wäscht die organische Phase mit Wasser, trocknet über Magnesiumsulfat und dampft im Vakuum ein. Den Rückstand reinigt man durch Chromatographie an Kieselgel mit Hexan / 5 - 30 % Ethylacetat und erhält 6,60 g der Titelverbindung als zähes Öl.

| Elementaranalyse: | | | |
|---|---|---|---|
| ber. | C 37,27 | H 2,41 | F 52,75 |
| gef. | C 37,10 | H 2,66 | F 52,80 |

c) 1,4,7-Tris(carboxylatomethyl)-10-{2-hydroxy-4-oxa-4-[4-(2H,2H,3H,3H-1-oxa-perfluorundec-1-yl)]-phenyl}-but-1-yl-1,4,7,10-tetraazacyclododecan
Zu einer Lösung von 3,46 g (10 mmol) 1,4,7-Tris(carboxylatomethyl)-1,4,7,10-tetraazacyclododecan und 2,5 g Natriumhydroxid in 25 ml Wasser gibt man eine Lösung von 6,12 g (10 mmol) 1-(3,4-Epoxy-1-oxa-but-1-yl)-4-(2H,2H,3H,3H-1-oxa-perfluorundec-1-yl)-benzol in 25 ml Tetrahydrofuran und erhitzt 24 Stunden unter Rückfluß, dampft dann im Vakuum weitgehend ein, löst den Rückstand in 100 ml Wasser, stellt mit 6N Salzsäure auf pH 3 und extrahiert mehrmals mit n-Butanol. Die vereinigten Extrakte werden im Vakuum eingedampft. Der Rückstand wird durch Chromatographie an RP-18 mit einem Gradienten aus Wasser/ n-Butanol/ Acetonitril gereinigt. Man erhält 6,71 g der Titelverbindung als viskoses Öl.

| Elementaranalyse: | | | | |
|---|---|---|---|---|
| ber. | C 41,35 | H 4,10 | F 33,69 | N 5,84 |
| gef. | C 41,58 | H 4,38 | F 33,50 | N 5,91 |

d) Gadolinium-Komplex von 1,4,7-Tris(carboxylatomethyl)-10-{2-hydroxy-4-oxa-4-[4-(2H,2H,3H,3H-1-oxa-perfluorundec-1-yl)]-phenyl}-but-1-yl-1,4,7,10-tetraazacyclododecan
Man versetzt eine Mischung von 4,79 g (5 mmol) 1,4,7-Tris(carboxylatomethyl)-10-{2-hydroxy-4-oxa-4-[4-(2H,2H,3H,3H-1-oxa-perfluorundec-1-yl)]-phenyl)}-but-1-yl-1,4,7,10-tetraazacyclododecan, 50 ml Wasser und 30 ml Ethanol mit 906 mg (2,5 mmol) Gadoliniumoxid und erhitzt 5 Stunden unter Rückfluß. Man filtriert die heiße Lösung und dampft im Vakuum ein. Man erhält 5,50 g der Titelverbindung als glasigen festen Stoff mit einem Wassergehalt von 4,9 %.

| Elementaranalyse (bezogen auf wasserfreie Substanz): | | | | | |
|---|---|---|---|---|---|
| ber. | C 35,62 | H 3,26 | F 29.02 | Gd 14,13 | N 5,03 |
| gef. | C 35,40 | H 3,50 | F 28,81 | Gd 14,01 | N 5,18 |

### Beispiel 20

### Gadoliniumkomplex, Dinatriumsalz von 3,9-Bis(carboxymethyl)-6-[(1-carboxy)-1H,2H,2H,4H,4H,5H,5H-3-oxa-perfluortridecyl]-3,6,9-triazaundecandisäure

a) N-t-Butoxycarbonyl-serin-(1H,1H,2H,2H-perfluordecyl)-ether-benzylester
In eine Lösung von 2,953 g (10 mmol) N-t-Butyloxycarbonyl-serinbenzylester (Kaufware Bachem) in 30 ml trockenem Dimethylformamid werden 300 mg (10 mmol) Natriumhydrid (80 % in Öl) anteilweise gegeben. Nach erfolgter Auflösung versetzt man mit 6,072 g (10 mmol) des unter 7a) hergestellten Tosylats. Man rührt 12 Stunden bei Raumtemperatur. Dann gießt man in 500 ml Eiswasser, nimmt das Produkt in Dichlormethan auf, wäscht die organische Lösung mit Wasser, trocknet über Natriumsulfat und engt zur Trockne ein. Der Rückstand wird durch Chromatographie an Kieselgel gereinigt. Als Elutionsmittel dient ein Gemisch aus Dichlormethan mit steigendem Methanolzusatz.
Die Titelverbindung wird als Sirup erhalten.
Ausbeute: 5,902 g (79,6 % d. Th.)

| Elementaranalyse: | | | | |
|---|---|---|---|---|
| ber. | C 40,50 | H 3,26 | F 43,56 | N 1,89 |
| gef. | C 40,64 | H 3,37 | F 43,49 | N 1,83 |

b) Serin-( 1 H, 1H,2H,2H-perfluordecyl)-ether-benzylester (als Salz der Trifluoressigsäure)
In 50 ml eines Gemisches aus Trifluoressigsäure und Dichlormethan im Verhältnis 2:1 werden 7,414 g (10 mmol) des unter 20a) hergestellten N-geschützten Verbindung gelöst und über Nacht bei Raumtemperatur gerührt. Man engt zur Trockne ein und entfernt Reste von Trifluoressigsäure durch Kodestillation mit Ethanol. Die Titelverbindung wird als Salz der Trifluoressigsäure isoliert.
Ausbeute: 7,418 g (98,2 % d. Th.)

| Elementaranalyse: | | | | |
|---|---|---|---|---|
| ber. | C 34,98 | H 2,27 | F 50,30 | N 1,85 |
| gef. | C 34,89 | H 2,31 | F 50,39 | N 1,80 |

c) 3,9-Bis(t-butoxycarbonylmethyl)-6-[(1-benzyloxycarbonyl)-1H,2H,2H,4H,4H,5H,5H-3-oxa-perfluortridecyl)]-3,6,9-triazaundecandisäure-di(t-butyl)-ester
In ein Gemisch aus 10 ml Acetonitril und 20 ml Phosphatpuffer von pH-Wert 8,0 werden 3,777 g (5 mmol) des unter 20b) hergestellten Amin-Trifluoracetates und 3,523 g (10 mmol) N,N-Bis(t-butyloxycarbonylmethyl)-2-(bromethyl)-amin gegeben und 2 Stunden intensiv bei Raumtemperatur gerührt. Dann trennt man die Pufferphase ab, extrahiert sie mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8,0) und 100 ml Essigester. Die organische Phase wird mit gesättigter Kochsalzlösung gewaschen, über Natriumsulfat getrocknet und eingeengt. Die Titelverbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 3,162 g (53,4 % d. Th.)

| Elementaranalyse: | | | | |
|---|---|---|---|---|
| ber. | C 48,69 | H 5,62 | F 27,28 | N 3,55 |
| gef. | C 48,82 | H 5,72 | F 27,37 | N 3,50 |

d) 3,9-Bis(carboxymethyl)-6-[(1-carboxy)-1H,2H,2H,4H,4H,5H,5H-3-oxa-perfluortridecyl]-3,6,9-triazaundecandisäure
In eine Mischung von 25 ml Trifluoressigsäure/Dichlormethan im Verhältnis 2:1 werden 5,920 g (5 mmol) der unter 20c) hergestellten Verbindung gegeben. Man läßt über Nacht bei Raumtemperatur rühren, engt dann zur Trockne ein, nimmt den Rückstand in 100 ml 3 N Salzsäure auf, erhitzt 3 Stunden unter Rückfluß, engt dann im Vakuum zur Trockne ein und nimmt in 160 ml eines Gemisches aus Wasser, Ethanol und Chloroform (10:5:1) auf. Die Lösung wird durch Zugabe von Ionenaustauscher IRA 67 (OH⁻-Form) auf einen konstanten pH-Wert (etwa 3) eingestellt. Man saugt rasch ab, engt ein und erhält die Titelverbindung als glasigen Feststoff.
Ausbeute: 3,080 g (71,3 % d. Th.)

| Elementaranalyse (auf wasserfreie Substanz bezogen): | | | | |
|---|---|---|---|---|
| ber. | C 34,53 | H 3,25 | F 37,15 | N 4,83 |
| gef. | C 34,41 | H 3,32 | F 37,29 | N 4,90 |

e) Gadoliniumkomplex, Dinatriumsalz von 3,9-Bis(carboxymethyl)-6-[(1-carboxy)-1H,2H,2H,4H,4H,5H,5H-3-oxa-perfluortridecyl]-3,6,9-triazaundecandisäure
In einem Gemisch aus 60 ml destilliertem Wasser und 30 ml Ethanol werden 2,941 g (3,0 mmol, berechnet auf 11,3 % Wassergehalt) der unter 20d) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Man stellt dann den pH-Wert der Lösung durch Zugabe von Natrionlauge auf 7,2 ein. Die Lösung wird dann eingeengt, wobei starkes Schäumen zu beobachten ist. Der Rückstand wird mit destilliertem Wasser kodestilliert. Die Titelverbindung wird als glasiger Feststoff erhalten.
Ausbeute: 3,489 g (quantitativ)
Wassergehalt: 8,2 %.

| Elementaranalyse (auf wasserfreie Substanz bezogen): | | | | | | |
|---|---|---|---|---|---|---|
| ber. | C 28,12 | H 2,17 | F 30,25 | Gd 14,73 | N 3,94 | Na 4,31 |
| gef. | C 28,25 | H 2,26 | F 30,40 | Gd 14,85 | N 3,99 | Na 4,38 |

### Beispiel 21

### Gadoliniumkomplex, Mononatriumsalz von 3,6,9-Tris(carboxymethyl)-3,6,9-triazaundecandisäure-mono-N-{ethyl-2-amino-[carbonylmethyl-amino-(N-ethyl-N-perfluoroctylsulfonyl)]}-amid

a) 3,6,9-Tris(carboxylatomethyl)-3,6,9-triazaundecandisäure-mono-N-{ethyl-2-amino-[carbonylmethyl-amino-(N-ethyl-N-perfluoroctylsulfonyl)]}-amid
In 200 ml eines Gemisches aus Dimethylformamid und Dichlormethan im Verhältnis 4:1 werden 17,87 g (50 mmol) Diethylentriaminpentaessigsäure-bisanhydrid suspeniert und anteilweise unter starkem Rühren mit dem Gemisch aus 3,137 g (5 mmol) [N-(2-Aminoethyl)-N-perfluoroctylsulfonyl]-aminoessigsäure-N-(2-aminoethyl)-amid und 6,50 g (64,2 mmol) Triethylamin versetzt. Man läßt 5 Stunden nachrühren, engt zur Trockne ein, versetzt mit 300 ml Eiswasser und stellt den pH-Wert des Ansatzes mit 3 N Salzsäure auf etwa 3 ein. Man extrahiert zweimal mit je 200 ml n Butanol, vereinigt die organischen Lösungen und engt sie ein. Das Produkt wird durch Chromatographie an Kieselgel RP-18 gereinigt. Als Elutionsmittel dienen Wasser und Tetrahydrofuran. Die Titelverbindung wird als glasiger Feststoff erhalten.
Ausbeute: 2,722 g (54,3 % d. Th.)
Wassergehalt: 9,7 %

| Elementaranalyse (bezogen auf wasserfreie Substanz): | | | | | |
|---|---|---|---|---|---|
| ber. | C 33,54 | H 3,52 | F 32,21 | N 8,38 | S 3,20 |
| gef. | C 33,65 | H 3,60 | F 32,14 | N 8,51 | S 3,29 |

b) Gadoliniumkomplex, Mononatriumsalz von 3,6,9-Tris(carboxymethyl)-3,6,9-triazaundecandisäure-mono-N-{ethyl-2-amino-[carbonylmethyl-amino-(N-ethyl-N-perfluoroctylsulfonyl)]}-amid
In 90 ml eines Gemisches aus destilliertem Wasser und Ethanol (2:1) werden 3,259 g (3 mmol, berechnet auf 9,7 % Wasser) der unter 21a) hergestellten Verbindung gegeben Unter Rühren werden anteilweise 543,8 mg (1,5 mmol) Gadoliniumoxid zugefügt plan rührt bis zur Lösung, stellt dann durch Zugabe von Natronlauge den pH-Wert der Lösung auf 7,2 ein und engt ein, wobei starkes Schäumen auftritt. Der Rückstand wird mit destilliertem Wasser kodestilliert. Die Titelverbindung wird als glasartiger Feststoff erhalten.
Ausbeute: 3,861 g (quantitativ)
Wassergehalt: 8,4 %
Die Elementaranalyse ist auf wasserfreie Substanz berechnet.

| | | | | | | | |
|---|---|---|---|---|---|---|---|
| ber. | C 28,53 | H 2,65 | F 27,40 | Gd 13,34 | N 7,13 | Na 1,95 | S 2,72 |
| gef. | C 28,61 | H 2,68 | F 27,48 | Gd 13,40 | N 7,08 | Na 1,99 | S 2,76 |

### Beispiel 22

### Gadoliniumkomplex, Mononatriumsalz von 3,9-Bis(carboxymethyl)-6-1H, 1H,4H,4H,5H,5H,8H,8H,10H,10H,11H,11H-2,7-dioxo-3,6-diaza-9-oxa-perfluormonodecyl)-3,6,9-triazaundecandisäure

a) Glycolsäure-(1H, 1H,2H,2H-perfluordecyl)-ether-N-(2-aminoethyl)-amid
In 80 ml Dichlormethan werden 10,44 g (20 mmol) der Verbindung 2b) gelöst und mit 2,30 g (20 mmol) N-Hydroxysuccinimid sowie 4,13 g (20 mmol) Dicyclohexylcarbodiimid versetzt. Man läßt über Nacht rühren, filtriert vom Dicyclohexylharnstoff ab und rührt das Filtrat in eine Lösung von 60,1 g (1000 mmol) Ethylendiamin in 100 ml Dichlormethan. Man läßt über Nacht rühren, versetzt mit 1,5 l Wasser und trennt die organische Phase ab. Man wäscht die Dichlormethanlösung mit Wasser, trocknet über Natriumsulfat, engt zur Trockne ein und reinigt den Rückstand durch Chromatographie an Kieselgel. Als Elutionsmittel dient ein Gemisch aus Dichlormethan mit steigendem Isopropanolzusatz.
Ausbeute: 9,615 g (85,2 % d. Th.)

| Elementaranalyse: | | | | |
|---|---|---|---|---|
| ber. | C 29,80 | H 2,32 | F 57,24 | N 4,96 |
| gef. | C 29,96 | H 2,37 | F 57,12 | N 5,01 |

b) Glycolsäure-(1H, 1H,2H,2H-perfluordecyl)-ether-N-[ethyl-2-(benzyloxycarbonylaminomethylcarbonylamino)]-amid
In 15 ml Dichlormethan werden 2,092 g (10 mmol) Benzyloxycarbonylglycin gelöst und mit 1,151 g (10 mmol) N-Hydroxysuccinimid sowie 2,063 g (10 mmol) Dicyclohexylcarbodiimid versetzt. Man läßt über Nacht rühren, filtriert vom Dicyclohexylharnstoff ab und engt zur Trockne ein. Der Rückstand wird an Kieselgel durch Säulenchromatographie gereinigt. Als Elutionsmittel dient ein Gemisch aus Dichlormethan und Ethanol. Die Titelverbindung wird als glasiger Feststoff erhalten.
Ausbeute: 6,905 g (91,4 % d. Th.)

| Elementaranalyse: | | | | |
|---|---|---|---|---|
| ber. | C 38,16 | H 2,94 | F 42,75 | N 5,56 |
| gef. | C 38,28 | H 2,98 | F 42,82 | N 5,50 |

c) Glycolsäure-(1H,1H,2H,2H-perfluordecyl)-ether-N-[ethyl-(2-aminomethylcarboxylamino)-amid
In 100 ml eines Gemisches aus Tetrahydrofuran und Ethanol im Verhältnis 2:1 werden 3,777 g (5 mmol) der unter 22b) hergestellten Verbindung in Gegenwart von 0,2 g Pearlman-Katalysator (Pd 20 % /C) bis zur Aufnahme von 112 ml Wasserstoff hydriert. Man saugt vom Katalysator ab, wäscht mit Ethanol gut nach und engt zur Trockne ein. Die Titelverbindung wird als glasartiger Feststoff erhalten.
Ausbeute: 3,097 g (99,7 % d. Th.)

| Elementaranalyse: | | | | |
|---|---|---|---|---|
| ber. | C 30,93 | H 2,60 | F 51,98 | N 6,76 |
| gef. | C 30,87 | H 2,64 | F 52,11 | N 6,82 |

d) 3,9-Bis(t-butyloxycarbonylmethyl)-6-(1H,1H,4H,4H,5H,5H,8H,8H,10H,10H,11H,11H,-2,7-dioxo-3,6-diaza-9-oxa-perfluornonadecyl)3,6,9-triazaundecandisäure-bis(t-butylester)
In ein Gemisch aus 10 ml Acetonitril und 20 ml Phosphatpuffer vom pH-Wert 8 werden 3,107 g (5 mmol) des unter 22c) hergestellten Amins und 3,523 g (10 mmol) N,N-Bis(t-butyloxycarbonylmethyl)-2-(bromethyl)-amin gegeben und 2 Stunden intensiv bei Raumtemperatur gerührt. Dann trennt man die Pufferphase ab, extrahiert sie mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8,0) und 100 ml Essigester. Die organische Phase wird mit gesättigter Kochsalzlösung gewaschen, über Natriumsulfat getrocknet und eingeengt. Die Verbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 3,044 g (52,3 % d. Th.)

| Elementaranalyse: | | | | |
|---|---|---|---|---|
| ber. | C 45,40 | H 5,71 | F 27,75 | N 6,02 |
| gef. | C 45,47 | H 5,78 | F 27,68 | N 6,10 |

e) 3,9-Bis(carboxymethyl)-6-(1H,1H,4H,4H,5H,5H,8H,8H,10H,10H,11H,11H-2,7-dioxo-3,6-diaza-9-oxa-perfluormonodecyl)-3,6,9-triazaundecan-disäure
In eine Mischung von 120 ml Trifluoressigsäure / Dichlormethan im Verhältnis 2:1 werden 5,820 g (5 mmol) der unter 22d) hergestellten Verbindung gegeben. Man läßt über Nacht bei Raumtemperatur rühren, engt zur Trockne ein, entfernt Reste von Trifluoressigsäure durch Kodestillation mit Ethanol und nimmt in 240 ml eines Gemisches aus Wasser, Ethanol und Chloroform auf. Die Lösung wird durch Zugabe von Ionenaustauscher IRA-67 (OH⁻-Form) auf einen konstant bleibenden pH-Wert (etwa 3) eingestellt. Man saugt rasch ab, engt ein und erhält die Titelverbindung als glasigen Feststoff.
Ausbeute: 3,214 g (68,4 % d. Th.)
Wassergehalt: 10,3 %

| Elementaranalyse (auf wasserfreie Substanz bezogen): | | | | |
|---|---|---|---|---|
| ber. | C 35,79 | H 3,65 | F 34,37 | N 7,45 |
| gef. | C 35,90 | H 3,72 | F 34,31 | N 7,51 |

f) Gadoliniumkomplex, Mononatriumsalz von 3,9-Bis(carboxymethyl)-6-(1H,1H,4H,4H,5H,5H,8H,8H,10H,10H,11H,11H-2,7-dioxo-3,6-diaza-9-oxa-perfluornonadecyl)-3,6,9-triaza-undecandisäure
In einem Gemisch aus 60 ml destilliertem Wasser und 30 ml Ethanol werden 3,143 g (3,0 mmol, berechnet auf 10,3 % Wassergehalt) der unter 22e) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Man stellt dann den pH-Wert der Lösung durch Zugabe von Natronlauge auf 7,2 ein, engt die Lösung ein, wobei starkes Schäumen zu beobachten ist. Der Rückstand wird mit destilliertem Wasser kodestilliert. Die Titelverbindung wird als glasiger Feststoff erhalten.
Ausbeute: 3,635 g (quantitativ)
Wassergehalt: 7,9 %

| Elementaranalyse (auf wasserfreie Substanz bezogen): | | | | | | |
|---|---|---|---|---|---|---|
| ber. | C 30,14 | H 2,71 | F 28,95 | Gd 14,09 | N 6,28 | Na 2,06 |
| gef. | C 30,21 | H 2,78 | F 29,03 | Gd 14,16 | N 6,22 | Na 2,11 |

### Beispiel 23

### Gadoliniumkomplex von 3,6,9-Tris(carboxymethyl)-3,6,9-triazaundecandisäure-bis{N-[2-aminoethyl-(N-ethyl-N-perfluoroctylsulfonyl]-amid}

a) N-Ethyl-(2-benzyloxycarbonylamino-ethyl)-perfluoroctylsulfonsäureamid
In 30 ml Dimethylformamid werden 5,272 g (10 mmol) Perfluoroctylsulfonsäure-N-ethylamid gelöst. Unter Feuchtigkeitsausschluß versetzt man mit 330 mg (11 mmol) Natriumhydrid (80 % in Öl). Nach beendeter Gasentwicklung tropft man die Lösung von 2,093 g 10 mmol)
N-Benzyloxycarbonylaziridin dazu und gießt in 300 ml Eiswasser, extrahiert mit Dichlormethan, wäscht die organische Lösung mit Wasser, trocknet sie über Natriumsulfat und engt zur Trockne ein. Der Rückstand wird an Kieselgel mit Dichlormethan / Methanol chromatographiert. Die Titelverbindung ist ein glasartiger Feststoff.
Ausbeute: 6,149 g (87,3 % d. Th.)

| Elementaranalyse: | | | | | |
|---|---|---|---|---|---|
| ber. | C 34,10 | H 2,43 | F 45,85 | N 3,98 | S 4,55 |
| gef. | C 34,00 | H 2,49 | F 45,97 | N 4,06 | S 4,49 |

b) N-Ethyl-N-2-(aminoethyl)-perfluoroctylsulfonamid

| Elementaranalyse: | | | | | |
|---|---|---|---|---|---|
| ber. | C 25,27 | H 1,94 | F 56,64 | N 4,91 | S 5,62 |
| gef. | C 25,39 | H 1,99 | F 56,57 | N 4,96 | S 5,53 |

c) 3,6,9-Tris(carboxymethyl)-3,6,9-triazaundecandisäure-bis{N-[2-aminoethyl-(N-ethyl-N-perfluoroctylsulfonyl)]-amid}
In 30 ml trockenem Dimethylformamid werden 5,703 g (10 mmol) der unter 23b) hergestellten Verbindung sowie 1,518 g (15 mmol) Triethylamin gelöst und anteilweise unter Rühren und Feuchtigkeitsausschluß mit 1,787 g (5 mmol) Diethylentriaminpentaessigsäure-Bisanhydrid versetzt. Man läßt über Nacht rühren, engt dann ein, versetzt mit Wasser, stellt den pH-Wert mit 3N Salzsäure auf etwa 3 ein und extrahiert zweimal mit je 100 ml n Butanol. Die organischen Lösungen werden vereinigt, eingeengt und einer Chromatographie an Kieselgel RP-18 unterworfen. Als Elutionsmittel dienen Wasser und Tetrahydrofuran. Die Titelverbindung wird als glasartiger Feststoff erhalten.
Ausbeute: 6,172 g (82,4 % d. Th.)
Wassergehalt: 9,8 %

| Elementaranalyse (auf wasserfreie Substanz bezogen): | | | | | |
|---|---|---|---|---|---|
| ber. | C 30,47 | H 2,76 | F 43,12 | N 6,55 | S 4,28 |
| gef. | C 30,59 | H 2,81 | F 43,00 | N 6,61 | S 4,33 |

d) Gadoliniumkomplex von 3,6,9-Tris(carboxymethyl)-3,6,9-triazaundecandisäure-bis{N-[2-aminoethyl-(N-ethyl-N-perfluoroctylsulfonyl)]-amid}
In einem Gemisch aus 120 ml destilliertem Wasser, 60 ml Ethanol und 20 ml Chloroform werden 6,570 g (4 mmol, berechnet auf 9,8 % Wassergehalt) der unter 23c) hergestellten Verbindung gegeben. Unter rühren und Erwärmen auf 50 °C werden anteilweise 725 mg (82,0 mmol) Gadoliniumoxid zugegeben. Man rührt bis zur Lösung, engt dann ein, wobei starkes Schäumen auftritt und unterwirft den Rückstand der Kodestillation mit destilliertem Wasser. Die Kodestillation wird zweimal wiederholt. Die Titelverbindung wird als glasartiger Feststoff erhalten.
Ausbeute: 7,191 g (quantitativ)
Wassergehalt: 8,1 %

| Elementaranalyse (auf wasserfreie Substanz bezogen): | | | | | | |
|---|---|---|---|---|---|---|
| ber. | C 27,63 | H 2,32 | F 39,10 | Gd 9,52 | N 5,93 | S 3,88 |
| gef. | C 27,50 | H 2,37 | F 39,22 | Gd 9,61 | N 5,85 | S 3,95 |

### Beispiel 24

### Gadoliniumkomplex von 3,6,9-Tris(carboxymethyl)-3,6,9-triazaundecandisäure-bis{N-<2-aminoethyl-[glycolsäure-(1H, 1H,2H,2H-perfluordecyl-ether)-amid]>-amid}

a) 3,6,9-Tris(carboxymethyl)-3,6,9-triazaundecandisäure-bis{N-<2-aminoethyl-glycolsäure-(1H, 1H,2H,2H-perfluordecyl-ether)-amid]>-amid}
In 40 ml trockenem Dimethylformamid werden 6,771 g (12 mmol) der unter Beispiel 22a) hergestellten Verbindung sowie 1,821 g (18 mmol) Triethylamin gelöst und anteilweise unter Rühren und Feuchtigkeitsausschluß mit 2,144 g (6 mmol) Diethylentriaminpentaessigsäure-Bisanhydrid versetzt. Man läßt über Nacht rühren, engt dann ein, versetzt mit 20 ml Wasser, stellt den pH-Wert auf etwa 3 ein und extrahiert mit 3N Salzsäure zweimal mit je 150 ml Butanol. Die organischen Lösungen werden vereinigt, eingeengt und man unterwirft den Rückstand einer Chromatographie an Kieselgel RP-18. Als Elutionsmittel dienen Wasser und Tetrahydrofuran. Die Titelverbindung wird als glasartiger Feststoff erhalten.
Ausbeute: 6,989 g (78,4 % d. Th.)
Wassergehalt: 7,1 %

| Elementaranalyse (auf wasserfreie Substanz bezogen): | | | | |
|---|---|---|---|---|
| ber. | C 33,95 | H 3,05 | F 43,47 | N 6,60 |
| gef. | C 34,06 | H 3,11 | F 43,40 | N 6,67 |

b) Gadoliniumkomplex von 3,6,9-Tris(carboxymethyl)-3,6,9-triazaundecandisäure-bis {N-<2-aminoethyl-[glycolsäure-( 1H,1H,2H,2H-perfluordecyl-ether)-amid]>-amid}
In einem Gemisch aus 100 ml destilliertem Wasser, 50 ml Ethanol und 20 ml Chloroform werden 4,798 g (3 mmol, berechnet auf 7,1 % Wasser) der unter 24a) hergestellten Verbindung gegeben. Unter Rühren und Erwärmen auf 50 °C werden anteilweise 543,8 mg (1,5 mmol) Gadoliniumoxid zugefügt. Man rührt bis zur Lösung, engt dann ein, wobei starkes Schäumen auftritt. Der Rückstand wird mehrfach mit destilliertem Wasser kodestilliert. Die Titelverbindung wird als glasartiger Feststoff erhalten.
Ausbeute: 5,285 g (quantitativ)
Wassergehalt: 6,9 %
Die Elementaranalyse ist auf wasserfreie Substanz berechnet.

| | | | | | |
|---|---|---|---|---|---|
| ber. | C 30,76 | H 2,58 | F 39,39 | Gd 9,59 | N 5,98 |
| gef. | C 30,87 | H 2,65 | F 39,51 | Gd 9,69 | N 6,11 |

### Beispiel 25

### Gadoliniumkomplex, Natriumsalz von 3,9-Bis(carboxymethyl)-6-[N-(1H,1H,2H,2H-perfluordecyl)-aminocarbonylmethyl-3,6,9-triazaundecandisäure

a) N-Benzyloxycarbonylglycin-N-(1H,1H,2H,2H-perfluordecyl)-amid
In 70 ml Dichlormethan werden 7,877 g (15 mmol) 1H,1H,2H,2H-Perfluordecylamin (J Fluor. Chem. 55, 85 (1991)) gelöst und mit 1,726 g (15 mmol) N-Hydroxysuccinimid. 3,095 g (15 mmol) Dicyclohexylcarbodiimid und 3,138 g (15 mmol) N-Benzyloxycarbonylglycin (Kaufware, Bachem) versetzt. Man läßt über Nacht rühren, filtriert den Dicyclohexylharnstoff ab, engt ein und unterwirft den Rückstand einer Säulenchromatographie an Kieselgel. Als Elutionsmittel dienen Gemische aus Dichlormethan und Ethanol Die Titelverbindung wird als Feststoff erhalten.
Ausbeute: 8,951 g (91,2% d. Th.)

| Elementaranalyse: | | | | |
|---|---|---|---|---|
| ber. | C 36,71 | H 2,31 | F 49,36 | N 4,28 |
| gef. | C 36,87 | H 2,39 | F 49,51 | N 4,37 |

b) Glycin-N-(1H, 1H,2H,2H-perfluordecyl)-amid
In 150 ml eines Gemisches aus Tetrahydrofuran und Ethanol im Verhältnis 2:1 werden 7,594 g (10 mmol) der unter 28a) hergestellten Verbindung gelöst und in Gegenwart von
b) Glycin-N-(1H,1H,2H,2H-perfluordecyl)-amid
In 150 ml eines Gemisches aus Tetrahydrofuran und Ethanol im Verhältnis 2:1 werden 7,594 g (10 mmol) der unter 28a) hergestellten Verbindung gelöst und in Gegenwart von 0,25 g Pearlman-Katalysator (Pd 20 % / C) bis zur Aufnahme von 224 ml Wasserstoff hydriert. Man saugt vom Katalysator ab, wäscht gut mit Ethanol nach und engt zur Trockne ein. Die Titelverbindung wird als amorpher Feststoff erhalten.
Ausbeute: 6,21 g (99,3 % d. Th.)

| Elementaranalyse: | | | | |
|---|---|---|---|---|
| ber. | C 25,37 | H 1,60 | F 56,84 | N 4,93 |
| gef. | C 25,28 | H 1,65 | F 56,92 | N 4,99 |

c) 3,9-Bis(t-butyloxycarbonylmethyl)-6-N-(1H,1H,2H,2H-perfluordecyl)-aminocarbonylmethyl-3,6,9-triazaundecandisäure-di(t-butylester)
In ein Gemisch aus 10 ml Acetonitril und 20 ml Phosphatpuffer vom pH-Wert 8,0 werden 2,841 g (5 mmol) des unter 25b) hergestellten Amins und 3,875 g (11 mmol) N,N-Bis(t-butyloxycarbonylmethyl)-2-(bromethyl)-amin gegeben und 2 Stunden intensiv bei Raumtemperatur gerührt. Dann trennt man die Pufferphase ab, extrahiert sie mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8.0) und 100 ml Essigester. Die organische Phase wird mit gesättigter Kochsalzlösung gewaschen, über Natriumsulfat getrocknet und eingeengt. Die Titelverbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 4,161 g (78,3 % d. Th.)

| Elementaranalyse: | | | | |
|---|---|---|---|---|
| ber. | C 45,20 | H 5,59 | F 30,39 | N 5,27 |
| gef. | C 45,35 | H 5,67 | F 30,47 | N 5,34 |

Trifluoressigsäure durch Kodestillation mit Ethanol und nimmt in 160 ml eines Gemisches aus Wasser, Ethanol und Chloroform (10:5:1) auf. Durch Zugabe von Ionenaustauscher IR A-67 (OH⁻-Form) wird ein pH-Wert von etwa 3 (pH-Konstanz) eingestellt. Man saugt rasch ab, engt ein und erhält die Titelverbindung als glasigen Feststoff
Ausbeute: 3,007 g (79,7 % d. Th.)
Wassergehalt: 10,9 %

| Elementaranalyse (auf wasserfreie Substanz bezogen): | | | | |
|---|---|---|---|---|
| ber. | C 34,38 | H 3,25 | F 38,52 | N 6,68 |
| gef. | C 34,29 | H 3,33 | F 38,65 | N 6,77 |

e) Gadoliniumkomplex, Mononatriumsalz von 3,9-Bis(carboxymethyl)-6-N-(1H,1H,2H,2H-perfluordecyl)-aminocarbonylmethyl)-3,6,9-triazaundecandisäure
In ein Gemisch aus 60 ml destilliertem Wasser und 30 ml Ethanol werden 2,823 g (3,0 mmol, berechnet auf 10,9 % Wassergehalt) der unter Beispiel 25d) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Dann stellt man den pH-Wert der Lösung durch Zugabe von Natronlauge auf 7,2 ein. Die Lösung wird eingeengt. Es tritt dabei starkes Schäumen auf. Der Rückstand wird zweimal mit destilliertem Wasser kodestilliert. Die Titelverbindung wird als glasiger Feststoff erhalten.
Ausbeute: 3,353 g (quant)
Wassergehalt: 9,2 %
Die Elementaranalyse wird auf wasserfreie Substanz berechnet.

| | | | | | | |
|---|---|---|---|---|---|---|
| ber. | C 28,41 | H 2,28 | F 31,83 | Gd 15,50 | N 5,52 | Na 2,27 |
| gef. | C 28,51 | H 2,33 | F 31,76 | Gd 15,57 | N 5,46 | Na 2,35 |

### Beispiel 26

### Gadoliniumkomplex, Dinatriumsalz von 3,6,9-Tris(carboxymethyl)-4-[N-1H,1H,2H,2H-perfluordecyloxy)-benzyl]-3,6,9-triaza-undecandisäure

a) 3,6,9-Tris-(t-butyloxycarbonylmethyl)-4-[4-(1H,1H,2H,2H-perfluordecyloxy)-benzyl]-3,6,9-triazaundecandisäure-di(t-butylester)
In 50 ml trockenes Dimethylformamid werden 6,131 g (5 mmol) 3,6,9-Tris (t-butyloxycarbonylmethyl)-4-(4-hydroxybenzyl)-3,6,9-triazaundecandisäure-di(t-butylester), dargestellt nach PCT WO 88/07521 gegeben und anteilweise unter Rühren und Feuchtigkeitsausschluß mit 150 g (5 mmol) Natriumhydrid (80 % in Öl) versetzt. Nach erfolgter Auflösung versetzt man mit 3,092 g (5 mmol) des unter Beispiel 7a) hergestellten Tosylats. Man rührt 12 Stunden bei 40°C. Dann gießt man in 500 ml Eiswasser, nimmt das Produkt in Dichlormethan auf, wäscht die organische Lösung mit Wasser, trocknet über Natriumsulfat und engt zur Trockne ein. Der Rückstand wird durch Chromatographie an Kieselgel gereinigt. Als Elutionsmittel dient ein Gemisch aus Dichlormethan, Isopropanol, Hexan im Verhältnis 20:1:5. Die Titelverbindung wird als amorpher Feststoff erhalten.
Ausbeute: 5,015 g (81,8 % d. Th.)

| Elementaranalyse: | | | | |
|---|---|---|---|---|
| ber. | C 49,96 | H 5,92 | F 26,34 | N 3,43 |
| gef. | C 50,11 | H 6,00 | F 26,43 | N 3,38 |

b) 3,6,9-Tris(carboxymethyl)-4-[4-(1H,1H,2H,2H-perfluordecyloxy)-benzyl]-3,6,9-triazaundecandisäure
In 100 ml eines Gemisches aus Trifluoressigsäure und Dichlormethan im Verhältnis 2:1 werden 3,678 g (3 mmol) der unter Beispiel 26a) hergestellten Verbindung gelöst und über Nacht bei Raumtemperatur gerührt. Man engt zur Trockne ein und entfernt Reste von Trifluoressigsäure durch Kodestillation mit Ethanol. Man nimmt den Rückstand in 160 ml eines Gemisches aus Wasser, Ethanol und Chloroform (10:5:1) auf. Durch Zugabe von Ionenaustauscher IRA-67 (OH⁻-Form) wird ein pH-Wert von etwa 3 (pH-Konstanz) eingestellt. Man saugt rasch ab, engt ein und erhält die Titelverbindung als glasigen Feststoff.
Ausbeute: 2,357 g (83,1 % d. Th.)
Wassergehalt: 11,3 %
Die Elementaranalyse ist auf wasserfreie Substanz berechnet.

| | | | | |
|---|---|---|---|---|
| ber. | C 39,38 | H 3,41 | F 34,16 | N 4,44 |
| gef. | C 39,52 | H 3,47 | F 34,32 | N 4,36 |

c) Gadoliniumkomplex, Dinatriumsalz von 3,6,9-Tris(carboxymethyl)-4-[N-(1H,1H,2H,2H-perfluordecyloxy)-benzyl]-3,6,9-triaza-undecandisäure
In ein Gemisch aus 60 ml destilliertem Wasser und 30 ml Ethanol werden 3,145 g (3,0 mmol, berechnet auf 11,3 % Wassergehalt) der unter Beispiel 26b) hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Dann stellt man den pH-Wert der Lösung durch Zugabe von Natronlauge auf 7,2 ein und engt ein. Dabei tritt starkes Schäumen auf Der Rückstand wird zweimal mit destilliertem Wasser kodestilliert. Die Titelverbindung wird als glasiger Feststoff erhalten.
Ausbeute: 3,804 g (quantitativ)
Wassergehalt: 9,8 %

| Elementaranalyse (auf wasserfreie Substanz bezogen): | | | | | | |
|---|---|---|---|---|---|---|
| ber. | C 32,55 | H 2,38 | F 28,24 | Gd 13,75 | N 3,67 | Na 4,02 |
| gef. | C 32,44 | H 2,43 | F 28,30 | Gd 13,66 | N 3,71 | Na 4,10 |

### Beispiel 27

### Gadoliniumkomplex von 10-[(-perfluoroctyl-sulfonyl)-piperazin-1-yl-carbonylmethyl]-1,4,7-tris(carboxymethyl)-1,4,7,10-tetraazacyclododecan

a) 1-Perfluoroctylsulfonyl-piperazin
34,39 g (398,3 mmol) Piperazin, 50 g (99,6 mmol) Perfluoroctylsulfonylfluorid und 10,12 g (100 mmol) Triethylamin werden 24 Stunden auf 85°C erhitzt. Man gibt 500 ml Wasser zu und extrahiert zweimal mit je 200 ml Dichlormethan. Die organische Phase wird über Magnesiumsulfat getrocknet und im Vakuum zur Trockne eingedampft. Der Rückstand wird an Kieselgel chromatographiert (Laufmittel: Dichlormethan/2-Propanol= 25:1).
Ausbeute: 17,55 g (31 % d. Th.) eines farblosen amorphen Feststoffes

| Elementaranalyse: | | | | | |
|---|---|---|---|---|---|
| ber. | C 25,36 | H 1,60 | F 56,84 | N 4,93 | S 5,64 |
| gef. | C 25,15 | H 1,80 | F 56,65 | N 4,81 | S 5,70 |

b) 1-(2-Bromacetyl)-4-perfluoroctylsulfonyl-piperazin
17 g (29,9 mmol) der Titelverbindung aus Beispiel 27a) und 5,1 g (50 mmol) Triethylamin werden in 100 ml Dichlormethan gelöst. Bei -10°C tropft man innerhalb von 30 Minuten 9,1 g (44,9 mmol) Bromacetylbromid zu und rührt 2 Stunden bei 0°C. Man gießt die Lösung in 200 ml 2 N Salzsäure und rührt gut durch. Die organische Phase wird abgetrennt, über Magnesiumsulfat getrocknet und im Vakuum eingeengt. Der Rückstand wird an Kieselgel chromatographiert (Laufmittel: Dichlormethan/Aceton= 20/1).
Ausbeute: 18,55 g (90 % d. Th.) eines leicht gelbgefärbten wachsartigen Feststoffes

| Elementaranalyse: | | | | | | |
|---|---|---|---|---|---|---|
| ber. | C 24,40 | H 1,46 | F 46,86 | N 4,06 | S 4,65 | Br 11,59 |
| gef. | C 24,22 | H 1,60 | F 46,75 | N 3,97 | S 4,48 | Br 11,41 |

c) 10-[(-perfluoroctyl-sulfonyl)-piperazin-1-yl-carbonylmethyl)-1,4,7-tris(carboxymethyl)-1,4,7,10-tetraazacyclododecan
Zu 17,78 g (20 mmol) der Titelverbindung aus Beispiel 27b) in 180 ml Methanol gibt man 4,63 g (13,36 mmol) 1,4,7-Tris(carboxymethyl)-1,4,7,10-tetraazacyclododecan (≅ DO3A) und 18,5 g (133,6 mmol) Kaliumkarbonat. Man kocht 12 Stunden unter Rückfluss. Die anorganischen Salze werden abfiltriert und das Filtrat zur Trockne eingedampft. Der Rückstand wird in 100 ml Wasser aufgenommen und mit 5 N Salzsäure auf pH 3 gestellt. Man extrahiert 2 mal mit 150 ml n-Butanol. Die vereinigten organischen Phasen werden im Vakuum zur Trockne eingeengt und der Rückstand durch RP-Chromatographie gereinigt (RP-18/Laufmittel= Gradient aus Wasser/n-Butanol/Acetonitril).
Ausbeute: 12,79 g (67 % d. Th.) eines farblosen Feststoffes
Wassergehalt: 8,5 %

| Elementaranalyse (bezogen auf wasserfreie Substanz): | | | | | |
|---|---|---|---|---|---|
| ber. | C 35,23 | H 3,70 | F 33,83 | N 8,80 | S 3,36 |
| gef. | C 35,17 | H 3,81 | F 33,67 | N 8,65 | S 3,18 |

d) Gadoliniumkomplex von 10-[(-perfluoroctyl-sulfonyl)-piperazin-1-yl-carbonylmethyl]-1,4,7-tris(carboxymethyl)-1,4,7,10-tetraazacyclododecan
10 g (10,47 mmol) der Titelverbindung aus Beispiel 27c) werden in einer Mischung aus 50 ml Wasser/20 ml Ethanol gelöst und 1,90 g (5,23 mmol) Gadoliniumoxid zugegeben. Man rührt 4 Stunden bei 80°C. Die Lösung wird filtriert und im Vakuum zur Trockne eingedampft.
Ausbeute: 12,2 g (quantitativ)
Wassergehalt: 5,1 %

| Elementaranalyse (bezogen auf wasserfreie Substanz): | | | | | |
|---|---|---|---|---|---|
| ber.: | C 30,33 | H 2,91 | F 29,13 | Gd 14,18 | S 2,89 |
| gef.: | C 30,39 | H 2,81 | F 29,02 | Gd 14,01 | S 2,78 |

### Beispiel 28

### Gadoliniumkomplex, Mononatriumsalz von 3,9-Bis(carboxymethyl)-6-[(4-perfluoroctylsulfonyl)-piperazin-1-carbonylmethyl]-3,6,9-triazaundecandisäure

a) 1-(2-Benzyloxycarbonylamino)-methyl-carbonyl-4-(perfluoroctylsulfonyl)-piperazin
In 80 ml Dichlormethan werden 8,524 g (15 mmol) des unter 27a) hergestellten Piperazinderivates gelöst und mit 1,726 g (15 mmol) N-Hydroxysuccinimid, 3,095 g (15 mmol) Dicyclohexylcarbodiimid und 3,138 g (15 mmol) N-Benzyloxycarbonylglycin (Kaufware, Bachem) versetzt. Man läßt über Nacht rühren, filtriert den Dicyclohexylhamstoff ab, engt ein und unterwirft den Rückstand einer Säulenchromatographie an Kieselgel. Als Elutionsmittel dienen Gemische aus Dichlormethan und Ethanol Die Titelverbindung wird als Feststoff erhalten.
Ausbeute: 10,16 g (89,2 % d. Th.)

| Elementaranalyse: | | | | | |
|---|---|---|---|---|---|
| ber. | C 34,79 | H 2,39 | F 42,53 | N 5,53 | S 4,22 |
| gef. | C 34,60 | H 2,43 | F 42,65 | N 5,66 | S 4,17 |

b) 1-(2-Amino)-acetyl-4-(perfluoroctyl)-sulfonyl-piperazin
In 150 ml eines Gemisches aus Tetrahydrofuran und Ethanol im Verhältnis 2:1 werden 7,594 g (10 mmol) der unter 28a) hergestellten Verbindung gelöst und in Gegenwart von 0,25 g Pearlman-Katalysator (Pd 20 % / C) bis zur Aufnahme von 224 ml Wasserstoff hydriert. Man saugt vom Katalysator ab, wäscht gut mit Ethanol nach und engt zur Trockne ein. Die Titelverbindung wird als amorpher Feststoff erhalten.
Ausbeute: 6,21 g (99,3 % d. Th.)

| Elementaranalyse: | | | | | |
|---|---|---|---|---|---|
| ber. | C 26,89 | H 1,93 | F 51,65 | N 6,72 | S 5,13 |
| gef. | C 27,03 | H 1,97 | F 51,77 | N 6,58 | S 5,20 |

c) 3, 9-Bis(t-butyloxycarbonylmethyl)-6-[(4-perfluoroctylsulfonyl)-piperazin-1-carbonylmethyl]-3,6,9-triazaundecandicarbonsäure-di(t-butylester)
In ein Gemisch aus 10 ml Acetonitril und 20 ml Phosphatpuffer vom pH-Wert 8,0 werden 3,127 g (5 mmol) des unter 28b) hergestellten Amins und 3,875 g (11 mmol) N,N-Bis(t-butyloxycarbonylmethyl)-2-(bromethyl)-amin gegeben und 2 Stunden intensiv bei Raumtemperatur gerührt. Dann trennt man den Puffer ab, extrahiert mit 10 ml Acetonitril und gibt dieses zur organischen Phase. Nach Zugabe von 20 ml frischem Puffer rührt man noch 20 Stunden bei Raumtemperatur. Man trennt die organische Phase ab, engt sie ein und verteilt den Rückstand zwischen 100 ml Phosphatpuffer (pH 8.0) und 100 ml Essigester. Die organische Phase wird mit gesättigter Kochsalzlösung gewaschen, über Natriumsulfat getrocknet und eingeengt. Die Titelverbindung wird durch Chromatographie an Kieselgel gereinigt. Als Elutionsmittel dient Dichlormethan mit steigendem Zusatz von Methanol. Die Titelverbindung wird als glasartiger Feststoff erhalten.
Ausbeute: 4,481 g (76,3 % d. Th.)

| Elementaranalyse: | | | | | |
|---|---|---|---|---|---|
| ber. | C 43,71 | H 5,42 | F 27,99 | N 4,85 | S 2,78 |
| gef. | C 43,84 | H 5,47 | F 28,10 | N 5,00 | S 2,69 |

d) 3,9-Bis(carboxymethyl)-6-[(4-perfluoroctyl-sulfonyl)-piperazin-1-yl-carbonylmethyl]-3,6,9-triazaundecandisäure
In eine Mischung von 100 ml Trifluoressigsäure / Dichlormethan im Verhältnis 2:1 werden 5,193 g (4,5 mmol) der unter 28c) hergestellten Verbindung gegeben. Man läßt über Nacht bei Raumtemperatur rühren, engt dann zur Trockne ein, entfernt Reste von Trifluoressigsäure durch Kodestillation mit Ethanol und nimmt in 160 ml eines Gemisches aus Wasser, Ethanol und Chloroform (10:5:1) auf. Durch Zugabe von Ionenaustauscher IRA-67 (OH⁻-Form) wird ein pH-Wert von etwa 3 (pH-Konstanz) eingestellt. Man saugt rasch ab, engt ein und erhält die Titelverbindung als glasigen Feststoff.
Ausbeute: 3,718 g (79,2 % d. Th.)
Wassergehalt: 10,9 %

| Elementaranalyse (bezogen auf wasserfreie Substanz): | | | | | |
|---|---|---|---|---|---|
| ber. | C 33,59 | H 3,25 | F 34,74 | N 6,03 | S 3,45 |
| gef. | C 33,69 | H 3,36 | F 34,82 | N 6,10 | S 3,38 |

e) Gadoliniumkomplex, Mononatriumsalz von 3,9-Bis(carboxymethyl)-6-[(4-perfluoroctylsulfonyl)-piperazin-1-carbonylmethyl]-3,6,9-triazaundecandisäure
In ein Gemisch aus 60 ml destilliertem Wasser und 30 ml Ethanol werden 3,13 g (3,0 mmol, berechnet auf 10,9 % Wassergehalt) der unter Beispiel 28d)'hergestellten Säure gegeben. Unter Rühren und Erwärmen auf 50 °C werden anteilweise 543,8 mg (1,5 mmol) Gadoliniumoxid zugegeben. Nach beendeter Zugabe wird bis zur Lösung gerührt. Dann stellt man den pH-Wert der Lösung durch Zugabe von Natronlauge auf 7,2 ein und engt ein. Dabei tritt starkes Schäumen auf. Der Rückstand wird zweimal mit destilliertem Wasser kodestilliert. Die Titelverbindung wird als glasiger Feststoff erhalten.
Ausbeute: 3,678 g (quantitativ)
Wassergehalt: 9,2%

| Elementaranalyse (bezogen auf wasserfreie Substanz): | | | | | | | |
|---|---|---|---|---|---|---|---|
| ber. | C 28,24 | H 2,37 | F 29,21 | Gd 14,22 | N 5,07 | Na 2,08 | S 2,90 |
| gef. | C 28,36 | H 2,41 | F 29,14 | Gd 14,30 | N 5,15 | Na 2,12 | S 2,83 |

### Beispiel 29

### Gadoliniumkomplex von 3,6,9-Tris(carboxymethyl)-3,6,9-triazaundecandisäure-bis[(4-perfluoroctylsulfonyl)-piperazin]-amid

a) 3,6,9-Tris(carboxymethyl)-3,6,9-triazaundecandisäure-bis[(4-perfluoroctylsulfonyl)-piperazin]-amid
In 30 ml trockenem Dimethylformamid werden 5,683 g (10 mmol) der unter 27a) hergestellten Verbindung sowie 1,518 g (15 mmol) Triethylamin gelöst und anteilweise unter Rühren und Feuchtigkeitsausschluß mit 1,787 g (5 mmol) Diethylentriaminpentaessigsäure-Bisanhydrid versetzt. Man läßt über Nacht rühren, engt dann ein, versetzt mit Wasser, stellt den pH-Wert mit 3N Salzsäure auf etwa 3 ein und extrahiert zweimal mit je 100 ml n Butanol. Die organischen Lösungen werden vereinigt, eingeengt und einer Chromatographie an Kieselgel RP-18 unterworfen. Als Elutionsmittel dienen Wasser und Tetrahydrofuran. Die Titelverbindung wird als glasartiger Feststoff erhalten.
Ausbeute: 6,741 g (81,4 % d. Th.)
Wassergehalt: 9,8 %

| Elementaranalyse (bezogen auf wasserfreie Substanz): | | | | | |
|---|---|---|---|---|---|
| ber. | C 30,55 | H 2,50 | F 43,24 | N 6,56 | S 4,29 |
| gef. | C 30,67 | H 2,55 | F 43,33 | N 6,49 | S 4,21 |

b) Gadoliniumkomplex von 3,6,9-Tris(carboxymethyl)-3,6,9-triazaundecandisäure-bis[(4-perfluoroctylsulfonyl)-piperazin]-amid
In einem Gemisch aus 120 ml destilliertem Wasser, 60 ml Ethanol und 20 ml Chloroform werden 6,570 g (4 mmol, berechnet auf 9,8 % Wassergehalt) der unter 23c) hergestellten Verbindung gegeben. Unter Rühren und Erwärmen auf 50 °C werden anteilweise 725 mg (82,0 mmol) Gadoliniumoxid zugegeben. Man rührt bis zur Lösung, engt dann ein, wobei
b) Gadoliniumkomplex von 3,6,9-Tris(carboxymethyl)-3,6,9-triazaundecandisäure-bis[(4-perfluoroctylsulfonyl)-piperazin]-amid
In einem Gemisch aus 120 ml destilliertem Wasser, 60 ml Ethanol und 20 ml Chloroform werden 6,570 g (4 mmol, berechnet auf 9,8 % Wassergehalt) der unter 23c) hergestellten Verbindung gegeben. Unter Rühren und Erwärmen auf 50 °C werden anteilweise 725 mg (82,0 mmol) Gadoliniumoxid zugegeben. Man rührt bis zur Lösung, engt dann ein, wobei starkes Schäumen auftritt und unterwirft den Rückstand der Kodestillation mit destilliertem Wasser. Die Kodestillation wird zweimal wiederholt. Die Titelverbindung wird als glasartiger Feststoff erhalten.
Ausbeute: 7,191 g (quantitativ)
Wassergehalt: 8,1 %

| Elementaranalyse (bezogen auf wasserfreie Substanz): | | | | | | |
|---|---|---|---|---|---|---|
| ber. | C 27,69 | H 2,08 | F 39,19 | Gd 9,54 | N 5,95 | S 3,89 |
| gef. | C 27,83 | H 2,15 | F 39,10 | Gd 6,91 | N 6,03 | S 3,88 |

### Beispiel 30

a) 11-[N-Ethyl-N-(perfluoroctylsulfonyl)-amino]undecansäurebenzylester
20 g (37,94 mmol) N-Ethyl-N-perfluoroctylsulfonamid und 15,73 g (113,8 mmol) Kaliumcarbonat werden in 200 ml Aceton suspendiert und bei 60 °C 26,96 g (75,87 mmol) 11-Bromundecansäurebenzylester zugetropft. Man rührt 3 Stunden bei 60 °C. Man filtriert von den Salzen ab und dampft das Filtrat im Vakuum zur Trockne ein. Der Rückstand wird an Kieselgel chromatographiert (Laufmittel:
Hexan/Dichlormethan/Aceton = 10/10/1). Nach Eindampfen der produkthaltigen Fraktionen kristallisiert man den Rückstand aus Methanol/Ether um.
Ausbeute: 26,46 g (87 % der Theorie) eines farblosen, kristallinen Pulvers.

| Elementaranalyse: | | | | | |
|---|---|---|---|---|---|
| ber. | C 41,95 | H 4,02 | N 1,75 | F 40,29 | S 4,00 |
| gef. | C 41,78 | H 4,17 | N 1,68 | F 40,12 | S 3,88 |

Filtrat im Vakuum zur Trockne eingedampft. Der Rückstand wird aus Ether/Hexan umkristallisiert.
Ausbeute: 16,69 g (94 % der Theorie) eines farblosen, kristallinen Feststoffs.

| Elementaranalyse: | | | | | |
|---|---|---|---|---|---|
| ber. | C 35,45 | H 3,68 | N 1,97 | F 45,39 | S 4,51 |
| gef. | C 35,31 | H 3,81 | N 1,85 | F 45,25 | S 4,42 |

c) Gadolinium-Komplex von 10-[2-Hydroxy-4-aza-5-oxo-16-aza-16-(perfluoroctylsulfonyl-octadecyl]-1,4,7-tris(carboxymethyl)-1,4,7,10-tetraazacyclododecan
12,16 g (17,09 mmol) der Titelverbindung aus Beispiel 30b) und 1,97 g (18,79 mmol) N-Hydroxysuccinimid werden in einer Mischung aus 50 ml Dimethylformamid/50 ml Chloroform gelöst. Bei 0 °C gibt man 3,88 g (18,79 mmol) Dicyclohexylcarbodiimid zu und rührt 1 Stunde bei 0 °C, anschließend 3 Stunden bei Raumtemperatur. Man kühlt erneut auf 0 °C und gibt 5,19 g (51,27 mmol) Triethylamin/50 ml 2-Propanol zu. Anschließend werden 10,78 g (18,79 mmol) Gadolinium-Komplex von 10-(3-Amino-2-hydroxypropyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 / Laufmittel: Gradient aus Wasser/N-Propanol/Acetonitril).
Ausbeute: 16,82 g (71 % der Theorie) eines farblosen, glasigen Feststoffs.
Wassergehalt: 8,6 %

| Elementaranalyse (bezogen auf wasserfreie Substanz): | | | | | | |
|---|---|---|---|---|---|---|
| ber. | C 36,02 | H 4,30 | F 25,49 | Gd 12,41 | N 6,63 | S 2,53 |
| gef. | C 35,87 | H 4,45 | F 25,28 | Gd 12,29 | N 6,50 | S 2,41 |

d) 10-[2-Hydroxy-4-aza-5-oxo-16-aza-16-(perfluoroctylsulfonyl-octadecyl]-1,4,7-tris(carboxymethyl)-1,4,7,10-tetraazacyclododecan
11,1 g (8,76 mmol) der Titelverbindung aus Beispiel 30 c) werden in einer Mischung aus 100 ml Wasser/100 ml Ethanol gelöst und 1,73 g (13,71 mmol) Oxalsäure-Dihydrat zugesetzt. Man erhitzt 8 Stunden auf 80 °C. Es wird auf 0 °C abgekühlt und vom ausgefallenen Gadoliniumoxalat abfiltriert. Das Filtrat wird zur Trockne eingedampft und der Rückstand an RP-18 gereinigt (RP-18 / Laufmittel: Gradient aus Wasser/i-Propanol/Acetonitril).
Ausbeute: 9,80 g (92 % der Theorie) eines glasigen Feststoffs.
Wassergehalt: 8,5 %

| Elementaranalyse (bezogen auf wasserfreie Substanz): | | | | | |
|---|---|---|---|---|---|
| ber. | C 41,01 | H 5,16 | F 29,02 | N 7,55 | S 2,88 |
| gef. | C 40,87 | H 5,31 | F 28,85 | N 7,40 | S 2,73 |

e) Ytterbium-Komplex von 10-[2-Hydroxy-4-aza-5-oxo-16-aza-16-(perfluoroctylsulfonyloctadecyl)-1,4,7-tris(carboxymethyl)-1,4,7,10-tetraazacyclododecan
Zu 5,64 g (5,07 mmol) der Titelverbindung aus Beispiel 30 d) in 100 ml Wasser/50 ml Ethanol gibt man 1,33 g (2,53 mmol) Ytterbiumcarbonat und rührt 3 Stunden bei 80 °C.
Die Lösung wird filtriert und das Filtrat im Vakuum zur Trockne eingedampft.
Ausbeute: 7,08 g (quantitativ) eines glasigen Feststoffs.
Wassergehalt: 8,1 %

| Elementaranalyse (bezogen auf wasserfreie Substanz): | | | | | | |
|---|---|---|---|---|---|---|
| ber. | C 35,58 | H 4,24 | F 25,17 | N 6,55 | S 2,50 | Yb 13,49 |
| gef. | C 35,43 | H 4,37 | F 25,05 | N 6,48 | S 2,39 | Yb 13,35 |

f) Dysprosium-Komplex von 10-[2-Hydroxy-4-aza-5-oxo-16-aza-16-(perfluoroctylsulfonyl-octadecyl]-1,4,7-tris(carboxymethyl)-1,4,7,10-tetraazacyclododecan
Zu 5,64 g (5,07 mmol) der Titelverbindung aus Beispiel 30 d) in 100 ml Wasser/50 ml Ethanol gibt man 0,95 g (2,53 mmol) Dysprosiumoxid und rührt 3 Stunden bei 80 °C. Die Lösung wird filtriert und das Filtrat im Vakuum zur Trockne eingedampft.
Ausbeute: 7,10 g (quantitativ) eines farblosen glasigen Feststoffs.
Wassergehalt: 9,1 %

| Elementaranalyse (bezogen auf wasserfreie Substanz): | | | | | | |
|---|---|---|---|---|---|---|
| ber. | C 35,87 | H 4,28 | F 25,38 | N 6,60 | S 2,52 | Dy 12,77 |
| gef. | C 35,69 | H 4,39 | F 25,18 | N 6,49 | S 2,43 | Dy 12,70 |

### Beispiel 31

a) 11,11,11,10,10,9,9,8,8,7,7-Tridecafluor-3-oxaundecansäure-tert.- butylester
Zu einer Mischung aus 27,57 g (75.73 mmol) 1H, 1H, 2H, 2H-Perfluoroctan-1-ol und 2,57 g (7.57 mmol) Tetrabutylammoniumhydrogensulfat in 300 ml 60 % wäss. Kalilauge/200 ml Toluol tropft man unter starkem Rühren bei 0 °C 19.51 g (100.0 mmol)Bromessigsäure-tert. -butylester zu. Man rührt eine Stunde bei 0 °C, trennt die organische Phase ab und extrahiert die wässrige Phase 2 mal mit 50 ml Toluol. Die vereinigten organischen Extrakte werden über Natriumsulfat getrocknet und im Vakuum eingedampft. Der Rückstand wird an Kieselgel chromatographiert (Laufmittel: Dichlormethan).
Ausbeute 28.97 g (80 % der Theorie) eines farblosen Öls.

| Elementaranalyse: | | | |
|---|---|---|---|
| ber. | C 35.16 | H 3.16 | F 51.64 |
| gef. | C 35.08 | H 3.20 | F 51.70 |

b) 11,11,11,10,10,9,9,8,8,7,7-Tridecafluor-3-oxaundecansäure
25.29 g (52.88 mmol) der Titelverbindung aus Beispiel 1a) werden in 300 ml Trifluoressigsäure gelöst und über Nacht bei Raumtemperatur gerührt. Man dampft im Vakuum zur Trockne ein und kristallisiert den Rückstand aus Hexan/Diethylether um.
Ausbeute 20.54 g (92 % der Theorie) eines farblosen kristallinen Feststoffs.

| Elementaranalyse: | | | |
|---|---|---|---|
| ber. | C 28.45 | H 1.67 | F 58.51 |
| gef. | C 28.36 | H 1.60 | F 58.62 |

c) Gadolinium-Komplex von 10-[2-Hydroxy-4-aza-5-oxo-7-oxa-10,10,11,11, 12,12,13,13,14,14,15,15,15-tridecafluor-pentadecyl]-1,4,7-tris(carboxy-methyl)-1,4,7,10-tetraazacyclododecan
7.21 g (17.09 mmol) der Titelverbindung aus Beispiel 31b und 1.97 g (18.79 mmol) N-Hydroxysuccinimid werden in einer Mischung aus 50 ml Dimethylformamid/50 ml Chloroform gelöst. Bei 0 °C gibt man 3.88 g (18.79 mmol) Dicyclohexylcarbodiimid zu und rührt 1 Stunde bei 0 °C, anschließend 3 Stunden bei Raumtemperatur. Man kühlt erneut auf 0 °C und gibt 5.19 g (51.27 mmol) Triethylamin/50 ml 2-Propanol zu. Anschließend werden 10.78 g (18.79 mmol) Gadolinium-Komplex von 10-(3-Amino-2-hydroxypropyl)-1.4.7-tris(carboxymethyl)-1.4.7.10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatograpie gereinigt (RP-18/Laufmittel: Gradient aus Wasser/n-Propanol/Acetonitril).
Ausbeute 12,68 g (71 % der Theorie) eines farblosen glasigen Feststoffes.
Wassergehalt: 6,4 %

| Elementaranalyse (bezogen auf wasserfreie Substanz): | | | | | |
|---|---|---|---|---|---|
| ber. | C 33.16 | H 3.61 | F 25.26 | Gd 16.08 | N 7,16 |
| gef. | C 32.85 | H 3.84 | F 25.01 | Gd 15.87 | N 7.03 |

### Beispiel 32

a) 15,15,15,14,14,13,13,12,12,11,11,10,10,9,9,8,8-7,7-Henicosafluor-3-oxapentadecansäure-tert.-butylester
Zu einer Mischung aus 42.72 g (75.73 mmol) 1H, 1H, 2H, 2H-Perfluoroctan-1-ol und 2,57 g (7.57 mmol) Tetrabutylammoniumhydrogensulfat in 300 ml 60 % wäss. Kalilauge/200 ml Toluol tropft man unter starkem Rühren bei 0 °C 19.51 g (100.0 mmol)Bromessigsäure-tert.-butylester zu. Man rührt eine Stunde bei 0 °C, trennt die organische Phase ab und extrahiert die wässrige Phase 2 mal mit 50 ml Toluol. Die vereinigten organischen Extrakte werden über Natriumsulfat getrocknet und im Vakuum eingedampft. Der Rückstand wird an Kieselgel chromatographiert (Laufmittel: Dichlormethan).
Ausbeute 42.12 g (82 % der Theorie) eines farblosen Öls.

| Elementaranalyse: | | | |
|---|---|---|---|
| ber. | C 31.87 | H 2.23 | F 58.82 |
| gef. | C 31.73 | H 2.20 | F 58.90 |

b) 15,15,15,14,14,13,13,12,12,11,11,10,10,9,9,8,8,7,7-Henicosafluor-3-oxapentadecansäure-tert.-butylester
35.87 g (52.88 mmol) der Titelverbindung aus Beispiel 1a) werden in 300 ml Trifluoressigsäure gelöst und über Nacht bei Raumtemperatur gerührt. Man dampft im Vakuum zur Trockne ein und kristallisiert den Rückstand aus Hexan/Diethylether um.
Ausbeute 30.60 g (93 % der Theorie) eines farblosen kristallinen Feststoffs.

| Elementaranalyse: | | | |
|---|---|---|---|
| ber. | C 27.03 | H 1.13 | F 64.12 |
| gef. | C 26.91 | H 1.20 | F 64.02 |

c) Gadolinium-Komplex von 10-[2-Hydroxy-4-aza-5-oxo-7-oxa-10,10,11,11, 12,12,13,13,14,14,15,15,16,16,17,17,18,18,19,19,19-henicosafluornonadecyl]-1,4,7-tris(carboxymethyl)-1,4,7-tris(carboxymethyl)-1,4,7,10-tetraazacyclododecan
10,63 g (17.09 mmol) der Titelverbindung aus Beispiel 32b und 1.97 g (18.79 mmol) N-Hydroxysuccinimid werden in einer Mischung aus 50 ml Dimethylformamid/50 ml Chloroform gelöst. Bei 0 °C gibt man 3.88 g (18.79 mmol) Dicyclohexylcarbodiimid zu und rührt 1 Stunde bei 0 °C, anschließend 3 Stunden bei Raumtemperatur. Man kühlt erneut auf 0 °C und gibt 5.19 g (51.27 mmol) Triethylamin/50 ml 2-Propanol zu. Anschließend werden 10.78 g (18.79 mmol) Gadolinium-Komplex von 10-(3-Amino-2-hydroxypropyl)-1.4.7-tris(carboxymethyl)-1.4.7.10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatograpie gereinigt (RP-18/Laufmittel: Gradient aus Wasser/n-Propanol/Acetonitril).
Ausbeute 14,73 g (69 % der Theorie) eines farblosen glasigen Feststoffes.
Wassergehalt: 5,7 %

| Elementaranalyse (bezogen auf wasserfreie Substanz): | | | | | |
|---|---|---|---|---|---|
| ber. | C 31.61 | H 2.99 | F 33.87 | Gd 13.35 | N 5.95 |
| gef. | C 31.49 | H 3.15 | F 33.68 | Gd 13.21 | N 6.01 |

### Beispiel 33

a) N-(2-Brompropionyl)glycin-benzylester
Zu 100 g (296,4 mmol) Glycinbenzylester-p-Toluolsulfonsäuresalz und 33,0 g (326,1 mmol) Triethylamin in 400 ml Methylenchlorid tropft man bei 0 °C 55,9 g (326,1 mmol) 2-Brompropionsäurechlorid zu. Man läßt die Temperatur nicht über 5 °C kommen. Nach beendeter Zugabe wird eine Stunde bei 0 °C gerührt, anschließend 2 Stunden bei Raumtemperatur. Man setzt 500 ml Eiswasser zu und stellt die Wasserphase mit 10 % aqu. Salzsäure auf pH 2. Die organische Phase wird abgetrennt, je einmal mit 300 ml 5 % aqu. Sodalösung und 400 ml Wasser gewaschen. Man trocknet die organische Phase über Magnesiumsulfat und dampft im Vakuum zur Trockne ein. Der Rückstand wird aus Diisopropylether umkristallisiert.
Ausbeute: 68,51 g (75 % d. Th.) eines farblosen kristallinen Pulvers
Schmelzpunkt: 69-70°C

| Elementaranalyse: | | | | |
|---|---|---|---|---|
| ber. | C 48,02 | H 4,70 | N 4,67 | Br 26,62 |
| gef. | C 47,91 | H 4,82 | N 4,51 | Br 26,47 |

b) 1-[4-(Benzyloxycarbonyl)-1-methyl-2-oxo-3-azabutyl]-1,4,7,10-tetraazacyclododecan
Zu 55,8 g (324,4 mmol) 1,4,7,10-Tetraazacyclododecan, gelöst in 600 ml Chloroform, gibt man 50 g (162,2 mmol) der Titelverbindung aus Beispiel 1a) und rührt über Nacht bei Raumtemperatur. Man gibt 500 ml Wasser zu, trennt die organische Phase ab und wascht sie noch jeweils 2 mal mit 400 ml Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1).
Ausbeute. 40,0 g [63 % d. Th.bezogen auf eingesetztes la)] eines leicht gelblichen zähen Öls.

| Elementaranalyse: | | | |
|---|---|---|---|
| ber. | C 61,36 | H 8,50 | N 17,89 |
| gef. | C 61,54 | H 8,68 | N 17,68 |

c) 10-[4-(Benzyloxycarbonyl)-1-methyl-2-oxo-3-azabutyl]-1,4,7-tris(tert.-butoxycarbonylmethyl)-1,4,7,10-tetraazacyclododecan (Natriumbromid-Komplex)
Zu 20 g (51,08 mmol) der Titelverbindung aus Beispiel 1b) und 17,91 (169 mmol) Natriumcarbonat in 300 ml Acetonitril gibt man 33 g (169 mmol) Bromessigsäure-tert.-butylester zu und rührt 24 Stunden bei 60 °C. Man kühlt auf 0 °C ab, filtriert von den Salzen ab und dampft das Filtrat zur Trockne ein. Der Rückstand wird an Kieselgel chromatographiert (Laufmittel: Essigsäureethylester/Ethanol: 15/1). Die das Produkt enthaltenden Fraktionen werden eingedampft und der Rückstand aus Diisopropylether umkristallisiert.
Ausbeute: 34,62 g (81 % d. Th.) eines farblosen kristallinen Pulvers
Schmelzpunkt: 116 - 117 °C

| Elementaranalyse: | | | | | |
|---|---|---|---|---|---|
| ber. | C 54,54 | H 7,59 | N 8,37 | Na 2,74 | Br 9,56 |
| gef. | C 54,70 | H 7,65 | N 8,24 | Na 2,60 | Br 9,37 |

d) 10-(4-Carboxy-1-methyl-2-oxo-3-azabutyl)-1,4,7-tris(tert.-butoxy carbonylmethyl)-1,4,7,10-tetraazacyclododecan (Natriumbromid-Komplex)
30 g (35,85 mmol) der Titelverbindung aus Beispiel 1c werden in 500 ml Isopropanol gelöst und 3 g Palladiumkatalysator (10 % Pd/C) hinzugegeben. Man hydriert über Nacht bei Raumtemperatur. Es wird vom Katalysator abfiltriert, das Filtrat im Vakuum zur Trockne eingedampft und aus Aceton umkristallisiert.
Ausbeute: 22,75 g (85 % d. Th.) eines farblosen kristallinen Pulvers
Schmelzpunkt: 225 °C (Zers.)

| Elementaranalyse: | | | | | |
|---|---|---|---|---|---|
| ber. | C 49,86 | H 7,69 | N 9,38 | Na 3,07 | Br 10,71 |
| gef. | C 49,75 | H 7,81 | N 9,25 | Na 2,94 | Br 10,58 |

e) 10-[1-Methyl-2-oxo-3-aza-5-oxo-5- {4-perfluorooctylsulfonyl-piperazin-1-yl }-pentyl]-1,4,7-tris(carboxymethyl)-1,4,7,10-tetraazacyclododecan
10 g (13,39 mmol) der Titelverbindung aus Beispiel 33 d und 7,61 g (13.39 mmol) der Titelverbindung aus Beispiel 27a werden in 150 ml Tetrahydrofuran gelöst. Bei 0 °C gibt man 3,97 g (16.07 mmol) N-Ethoxycarbonyl-2-ethoxy-1,2-dihydrochinolin (EEDQ) zu, rührt 3 Stunden bei 0 °C, anschließend 12 Stunden bei Raumtemperatur. Man dampft im Vakuum zur Trockne ein. Der Rückstand wird in 150 ml Trifluoressigsäure aufgenommen und 12 Stunden bei Raumtemperatur gerührt. Es wird zur Trockne eingedampft, der Rückstand in Wasser gelöst und mit 10 %iger wässriger Natronlauge auf pH 3.2 eingestellt. Zur Reinigung wird an RP-18 chromatographiert (Gradient aus Wasser/Acetonitril/Tetrahydrofuran).
Ausbeute 9,67 g (63 % der Theorie) eines hygroskopischen Feststoffes.
Wassergehalt: 10,5 %

| Elementaranalyse (bezogen auf wasserfreie Substanz): | | | | | |
|---|---|---|---|---|---|
| ber. | C 36.30 | H 3.93 | N 9,56 | F 31.49 | S 3.13 |
| gef. | C 36.14 | H 3.98 | N 9.40 | F 31.67 | S 3.02 |

f) Gadolinium-Komplex von 10-[1-Methyl-2-oxo-3-aza-5-oxo-5-{4-perfluorooctylsulfonyl-piperazin-1-yl}-pentyl]-1,4,7-tris(carboxymethyl)-1,4,7,10-tetraazacyclododecan
5 g (4.87 mmol) der Titelverbindung aus Beispiel 33 e werden in 60 ml Wasser gelöst und 0.883 g (2.44 mmol) Gadolinium-oxid zugegeben. Man rührt 3 Stunden bei 90 °C. Die Lösung wird filtriert und das Filtrat gefriergetrocknet
Ausbeute 6,47 g (quantitativ) eines voluminösen amorphen Pulvers.
Wassergehalt: 11,3 %

| Elementaranalyse (bezogen auf wasserfreie Substanz): | | | | | | |
|---|---|---|---|---|---|---|
| ber. | C 31.56 | H 3.16 | N 8.31 | F 27.37 | S 2,72 | Gd 13.33 |
| gef. | C 31.37 | H 3.35 | N 8.18 | F 27.19 | S 2.92 | Gd 13.05 |

### Beispiel 34

a) 4-Perfluoroctansulfonylpiperazin-1-ylpentandiamsäure
Zu einer Suspension von 11.41 g (100.0 mmol) Glutarsäureanhydrid in 100 ml Tetrahydrofuran tropft man unter kräftigem Rühren bei 0 °C eine Lösung von 10.62 g (105.0 mmol) Triethylamin und 59.67 g (105.0 mmol) der Titelverbindung aus Beispiel 27a) in 50 ml Tetrahydrofuran und läßt über Nacht auf Raumtemperatur kommen. Man säuert das Reaktionsgemisch mit 100 ml 2N HCL an und extrahiert 3 mal mit 100 ml Tetrahydrofuran. Die vereinigten organischen Extrakte werden mit Natriumsulfat getrocknet, filtriert und eingeengt. Der Rückstand wird aus 2-Propanol/Ethylacetat umkristallisiert.
Ausbeute 52.30 g (73 % der Theorie) eines farblosen kristallinen Feststoffes.

| Elementaranalyse: | | | | | |
|---|---|---|---|---|---|
| ber. | C 29.92 | H 2.22 | N 4.11 | F 47.33 | S 4.70 |
| gef. | C 29.90 | H 2.18 | N 4.07 | F 47.42 | S 4.79 |

b) Gadolinium-Komplex von 10-[2-Hydroxy-4-aza-5,9-dioxo-9-{4- perfluoroctyl)-piperazin-1-yl}-nonyl]-1,4,7-tris(carboxymethyl)-1,4,7-tris(carboxymethyl)-1,4,7,10-tetraazacyclododecan
11.66 g (17.09 mmol) der Titelverbindung aus Beispiel 34a und 1.97 g (18.79 mmol) N-Hydroxysuccinimid werden in einer Mischung aus 50 ml Dimethylformamid/50 ml Chloroform gelöst. Bei 0 °C gibt man 3.88 g (18.79 mmol) Dicyclohexylcarbodiimid zu und rührt 1 Stunde bei 0 °C, anschließend 3 Stunden bei Raumtemperatur. Man kühlt erneut auf 0 °C und gibt 5.19 g (51.27 mmol) Triethylamin/50 ml 2-Propanol zu. Anschließend werden 10.78 g (18.79 mmol) Gadolinium-Komplex von 10-(3-Amino-2-hydroxypropyl)-1.4.7-tris(carboxymethyl)-1.4.7.10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatograpie gereinigt (RP-18/Laufmittel: Gradient aus Wasser/n-Propanol/Acetonitril).
Ausbeute 16,7 g (73 % der Theorie) eines farblosen glasigen Feststoffes.
Wassergehalt: 7,5 %

| Elementaranalyse (bezogen auf wasserfreie Substanz): | | | | | | |
|---|---|---|---|---|---|---|
| ber. | C 32.99 | H 3.50 | F 26.09 | Gd 12.70 | N 7,92 | S 2.59 |
| gef. | C 32.75 | H 3.68 | F 25.88 | Gd 12.55 | N 7.84 | S 2.63 |

### Beispiel 35

a) N-Benzylperfluoroctansulfonamid
Zu einer Mischung von 10.62 g (105.0 mmol) Triethylamin und 10.72 g (100.0 mmol) Benzylamin tropft man bei 80°C unter kräftigem Rühren 50.21 g (100.0 mmol) Perfluoroctansulfonylfluorid. Man rührt 2 Tage bei 80°C, versetzt das Rektionsgemisch mit 300 ml Wasser und extrahiert 3 mal mit Ethylacetat. Die vereinigten organischen Extrakte werden über Natriumsulfat getrocknet, filtriert und eingeengt. Der Rückstand wird an Kieselgel chromatographiert (Laufmittel: Dichlormethan/Methanol = 4/1).
Ausbeute: 45.96 g (78% d.Th.) einer farblosen Flüssigkeit

| Elementaranalyse: | | | | | |
|---|---|---|---|---|---|
| ber. | C 30.57, | H 1.37, | N 2.38, | S 5.44, | F 54.81 |
| gef. | C 30.49 | H 1.30, | N 2.42, | S 5.50, | F 54.90 |

b) N-Benzyl-N-(perfluoroctylsulfonyl)-aminoessigsäure-t-butylester
22,4 g (37,94 mmol) der Titelverbindung aus Beispiel 35 a und 15.73 g (113,8 mmol) Kaliumcarbonat werden in 200 ml Aceton suspendiert und bei 60°C 14,80 g (75,87 mmol) Bromessigsäure-tert.-butylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen kristallisiert man den Rückstand aus Methanol/Ether um.
Ausbeute: 24,02 g (90% d. Th.) eines wachsartigen farblosen Feststoffes

| Elementaranalyse: | | | | | |
|---|---|---|---|---|---|
| ber. | C 35.86, | H 2.58, | N 1.99, | S 4.56, | F 45.91 |
| gef. | C 35.67 | H 2.71, | N 2.13, | S 4.45, | F 45.83 |

c) N-Benzyl-N-(perfluoroctylsulfonyl)-aminoessigsäure
20 g (28,43 mmol) der Titelverbindung aus Beispiel 35 b werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 17,48 g (95% d. Th.) eines farblosen kristallinen Feststoffes

| Elementaranalyse: | | | | | |
|---|---|---|---|---|---|
| ber. | C 31,54, | H 1.56, | N 2.16, | S 4.95, | F 49.89 |
| gef. | C 31.38 | H 1.70, | N 2.05, | S 4.87, | F 49.71 |

d) Gadolinium-Komplex von 10-[2-Hydroxy-4-aza-5-oxo-7-aza-7-(perfluoroctylsulfonyl)-8-phenyl-octyl]-1,4,7-tris(carboxymethyl)-1,4,7,10-tetraazacyciododecan
11,06 g (17,09 mmol) der Titelverbindung aus Beispiel 35 c und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C ab und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 16,49 g (75% d. Th.) eines farblosen glasigen Feststoffes
Wassergehalt: 6,5%

| Elementaranalyse: | | | | | | |
|---|---|---|---|---|---|---|
| ber. | C 33,95, | H 3,18, | N 6.99, | S 2.67, | F 26,85 | Gd 13.07 |
| gef. | C 33,81 | H 3,24, | N 6.82, | S 2.54, | F 26,64 | Gd 12.91 |

### Beispiel 36

a) N-Decylperfluoroctansulfonamid
Zu einer Mischung von 10.62 g (105.0 mmol) Triethylamin und 15.73 g (100.0 mmol) Decylamin tropft man bei 80°C unter kräftigem Rühren 50.21 g (100.0 mmol) Perfluoroctansulfonylfluorid. Man rührt 2 Tage bei 80°C, versetzt das Reaktionsgemisch mit 300 ml Wasser und extrahiert 3 mal mit Ethylacetat. Die vereinigten organischen Extrakte werden über Natriumsulfat getrocknet, filtriert und eingeengt. Der Rückstand wird an Kieselgel chromatographiert (Laufmittel: Dichlormethan/Methanol = 4/1).
Ausbeute: 43,48 g (68% d. Th.) einer farblosen viskosen Flüssigkeit

| Elementaranalyse: | | | | | |
|---|---|---|---|---|---|
| ber. | C 33.81, | H 3.47, | N 2.19, | S 5.02, | F 50.51 |
| gef. | C 33,71 | H 3.39, | N 2.15, | S 4.93, | F 50.31 |

b) N-Decyl-N-(perfluoroctylsulfonyl)-aminoessigsäure-t.-butylester
24,26 g (37,94 mmol) der Titelverbindung aus Beispiel 36 a und 15,73 g (113,8 mmol) Kaliumcarbonat werden in 200 ml Aceton suspendiert und bei 60°C 14,80 g (75,87 mmol) Bromessigsäure-tert.-butylester zugetropft.Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen kristallisiert man den Rückstand aus Methanol/Ether um.
Ausbeute: 24,87 g (87% d. Th.) eines wachsartigen farblosen Feststoffes

| Elementaranalyse: | | | | | |
|---|---|---|---|---|---|
| ber. | C 38,25, | H 4,28, | N 1,86, | S 4.26, | F 42,86 |
| gef. | C 38,09 | H 4,41, | N 1,74, | S 4.10, | F 42,67 |

c) N-Decyl-N-(perfluoroctylsulfonyl)-aminoessigsäure
20 g (26,54 mmol) der Titelverbindung aus Beispiel 36 b werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 17,22 g (93% d. Th.) eines farblosen kristallinen Feststoffes

| Elementaranalyse: | | | | | |
|---|---|---|---|---|---|
| ber. | C 34,44, | H 3,47, | N 2,01, | S 4.60, | F 46,31 |
| gef. | C 34,28 | H 3,30, | N 1,95, | S 4.65, | F 46,28 |

d) Gadolinium-Komplex von 10-[2-Hydroxy-4-aza-5-oxo-7-aza-7-(perfluoroctylsulfonyl)-heptadecyl]-1,4,7-tris(carboxymethyl)-1,4,7,10-tetraazacyclododecan
11,92 g (17,09 mmol) der Titelverbindung aus Beispiel 36 c und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C ab und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 16,76 g (71% d. Th.) eines farblosen glasigen Feststoffes
Wassergehalt: 6,5%

| Elementaranalyse: | | | | | | |
|---|---|---|---|---|---|---|
| ber. | C 35,46, | H 4,18, | N 6.71, | S 2.56, | F 25,77 | Gd 12,55 |
| gef. | C 35,28 | H 4,33, | N 6.80, | S 2.61, | F 25,65 | Gd 12.41 |

### Beispiel 37

a) N-Hexylperfluoroctansulfonamid
Zu einer Mischung von 10,62 g (105.0 mmol) Triethylamin und 10,12 g (100.0 mmol) Benzylamin tropft man bei 80°C unter kräftigem Rühren 50.21 g (100.0 mmol) Perfluoroctansulfonylfluorid. Man rührt 2 Tage bei 80°C, versetzt das Rektionsgemisch mit 300 ml Wasser und extrahiert 3 mal mit Ethylacetat. Die vereinigten organischen Extrakte werden über Natriumsulfat getrocknet, filtriert und eingeengt. Der Rückstand wird an Kieselgel chromatographiert (Laufmittel: Dichlormethan/Methanol = 4/1).
Ausbeute: 45.50 g (78% d.Th.) einer farblosen Flüssigkeit

| Elementaranalyse: | | | | | |
|---|---|---|---|---|---|
| ber. | C 28,83, | H 2,42, | N 2.40, | S 5.50, | F 55,37 |
| gef. | C 28,29 | H 2,39, | N 2.44, | S 5.55, | F 55,50 |

b) N-Hexyl-N-(perfluoroctylsulfonyl)-aminoessigsäure-t.-butylester
22,13 g (37,94 mmol) der Titelverbindung aus Beispiel 37 a und 15.73 g (113,8 mmol) Kaliumcarbonat werden in 200 ml Aceton suspendiert und bei 60°C 14,80 g (75,87 mmol) Bromessigsäure-tert.-butylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen, kristallisiert man den Rückstand aus Methanol/Ether um.
Ausbeute: 23,02 g (87% d. Th.) eines wachsartigen farblosen Feststoffes

| Elementaranalyse: | | | | | |
|---|---|---|---|---|---|
| ber. | C 34,44, | H 3,47, | N 2,01, | S 4.60, | F 46,31 |
| gef. | C 34,31 | H 3,61, | N 1,97, | S 4.65, | F 46,25 |

c) N-Hexyl-N-(perfluoroctylsulfonyl)-aminoessigsäure
20 g (28,43 mmol) der Titelverbindung aus Beispiel 37 b werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 16,74 g (91% d. Th.) eines farblosen kristallinen Feststoffes

| Elementaranalyse: | | | | | |
|---|---|---|---|---|---|
| ber. | C 29,96, | H 2,51, | N 2.18, | S 5,00, | F 50,36 |
| gef. | C 29,87 | H 2.70, | N 2.05, | S 4.84, | F 50,17 |

d) Gadolinium-Komplex von 10-[2-Hydroxy-4-aza-5-oxo-7-aza-7-(perfluoroctylsulfonyl)-tridecyl]-1,4,7- tris(carboxymethyl)-1,4,7,10-tetraazacyclododecan
10,96 g (17,09 mmol) der Titelverbindung aus Beispiel 37 c und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexylcarbodiimid zu und rührt 1 Stunde bei 0°C, anschließend 3 Stunden bei Raumtemperatur. Man kühlt erneut auf 0°C ab und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylhamstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 16,46 g (75% d. Th.) eines farblosen glasigen Feststoffes
Wassergehalt: 6,8%

| Elementaranalyse: | | | | | | |
|---|---|---|---|---|---|---|
| ber. | C 33,11, | H 3,70, | N 7,02, | S 2.68, | F 26,98 | Gd 13.14 |
| gef. | C 33,01 | H 3,84, | N 6.95, | S 2.57, | F 26,85 | Gd 13,03 |

### Beispiel 38

a) 11 -[N-Ethyl-N-(perfluoroctylsulfonyl)-amino]-hexansäurebenzylester
20 g (37,94 mmol) N-Ethyl-N-perfluoroctylsulfonylamid und 15.73 g (113,8 mmol) Kaliumcarbonat werden in 200 ml Aceton suspendiert und bei 60°C 21,64 g (75,87 mmol) 6-Bromhexansäurebenzylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton = 10/10/1). Nach Eindampfen der produkthaltigen Fraktionen, kristallisiert man den Rückstand aus Methanol/Ether um.
Ausbeute: 25,26 g (91% d. Th.) eines farblosen kristallinen Pulvers

| Elementaranalyse: | | | | | |
|---|---|---|---|---|---|
| ber. | C 37,77, | H 3,03, | N 1.91, | S 4.38, | F 44,15 |
| gef. | C 37,61 | H 3,18, | N 1,84, | S 4.27, | F 44,01 |

b) 11-[N-Ethyl-N-(perfluoroctylsulfonyl)-amino]-hexansäure
20 g (27,34 mmol) der Titelverbindung aus Beispiel 38 b werden in 300 ml Isopropanol/200 ml Dichlormethan gelöst und 3 g Palladiumkatalysator (10% Pd/C) zugegeben. Man hydriert über Nacht bei Raumtemperatur. Es wird vom Katalysator abfiltriert und das Filtrat im Vakuum zur Trockne eingedampft. Der Rückstand wird aus Ether/Hexan umkristallisiert.
Ausbeute: 16,13 g (92% d. Th.) eines farblosen kristallinen Feststoffes

| Elementaranalyse: | | | | | |
|---|---|---|---|---|---|
| ber. | C 29,96, | H 2.51, | N 2,18, | S 5,00, | F 50,36 |
| gef. | C 29,81 | H 2.70, | N 2.09, | S 4.93, | F 50,14 |

d) Gadolinium-Komplex von 10-[2-Hydroxy-4-aza-5-oxo-11-aza-11-(perfluoroctylsulfonyl)-tridecyl]-1,4,7-tris(carboxymethyl)-1,4,7,10-tetraazacyclododecan
10,96 g (17,09 mmol) der Titelverbindung aus Beispiel 38 b und 1,97 g (18.79 mmol) N-Hydroxysuccinimid werden in einer Mischung aus 50 ml Dimethylformamid/50 ml Chloroform gelöst. Bei 0°C gibt man 3,88 g (18,79 mmol) Dicyclohexyicarbodiimid zu und rührt 1 Stunde bei 0°C, anschließend 3 Stunden bei Raumtemperatur. Man kühlt erneut auf 0°C ab und gibt 5.19 g(51,27 mmol) Triethylamin/50 ml 2-Propanol zu. Anschließend werden 10,78 g (18.79 mmol) Gadolinium-Komplex von 10-(3-Amino-2-hydroxy-propyl)-1,4,7-tris(carboxymethyl)-1,4,7,10-tetraazacyclododecan (WO 95/17451) gelöst in 50 ml Wasser zugegeben und 3 Stunden bei Raumtemperatur gerührt. Man dampft zur Trockne ein, nimmt den Rückstand in einer Mischung aus 200 ml Methanol/100 ml Chloroform auf und filtriert vom Dicyclohexylharnstoff ab. Das Filtrat wird zur Trockne eingedampft und durch RP-Chromatographie gereinigt (RP-18 Laufmittel: Gradient aus Wasser/n-Propanol/Acetonitril).
Ausbeute: 15,0 g (69% d. Th.) eines farblosen glasigen Feststoffes
Wassergehalt: 5,9%

| Elementaranalyse: | | | | | | |
|---|---|---|---|---|---|---|
| ber. | C 33,11, | H 3,70, | N 7,02, | S 2.68, | F 26,98 | Gd 13.14 |
| gef. | C 33,01 | H 3,83, | N 6.91, | S 2.49, | F 26,83 | Gd 13,05 |

### Beispiel 39

Von der Verbindung aus Beispiel 1c) wurde eine 100 mmol/1 Lösung in Wasser hergestellt, die als röntgendichten Marker einen Zusatz von 100 mg J/ml Isovist ® enthielt. Diese Lösung ist klar durchsichtig und besitzt eine gelartig feste Konsistenz, läßt sich aber problemlos von Hand oder mit einer Infusionspumpe über einen 20 cm langen, 0,58 mm Øᵢ, Schlauch mit einer Geschwindgkeit von 160 µl/min applizieren.

### Beispiel 40

### Löslichkeit des Gadolinium-Komplexes aus Beispiel 1c) in verschiedenen Lösungsmitteln und mit verschiedenen Zusätzen und Darreichungsformen.

Von der Verbindung aus Beispiel 1c) wurde eine 100 mmol/L Lösung in Wasser hergestellt. Diese Lösung ist klar durchsichtig und besitzt eine gelartig feste Konsistenz, läßt sich aber problemlos von Hand oder mit einer Infusionspumpe über einen 20 cm langen, 0.58 mm Øᵢ, Schlauch mit einer Geschwindigkeit von 160 µL/min applizieren. Unterhalb von etwa 10-20 mmol/L ergibt sich eine frei fließende Lösung.

Der Zusatz von 10 mmol/L (Endkonzentration) HCI bzw. NaOH oder hohen Salzkonzentrationen (3 mol/L NaCl) hat keinen Einfluß auf die Konsistenz der Zubereitung.

Der Zusatz von 8 mol/L (Endkonzentration) Harnstoff ergibt eine frei fließende Lösung auch bei sehr hohen Substanzkonzentrationen (400 mmol/L).

Der Zusatz von Detergenzien zu der wässerigen Formulierung der Verbindung aus Beispiel lc) wirkt sich sehr unterschiedlich aus. Tween® 80 (Polyoxyethylen-sorbitan-oleat, nichtionisches Detergens, HLB 15, 4.6% Endkonzentration) bewirkt keine Veränderung der Konsistenz, während Triton® X-100 (Octylphenol-Polyethylen-glycolether, nichtionisches Detergens, HLB 13.5, 1.7%) zu einer Verflüssigung der gelartigen Konsistenz führt.

In Rinderplasma ist die Substanz ebenso löslich wie in reinem Wasser und ergibt eine Lösung mit vergleichbarer Konsistenz.

In reinem Propylenglycol oder DMSO ist die Verbindung aus Beispiel 1c) praktisch unlöslich. In 66% Propylenglycol/Wasser bzw. 66% DMSO/Wasser ist die Substanz jedoch auch in höherer Konzentration (250 mmol/L bzw. 150 mmol/L) gut löslich und ergibt eine frei fließende Lösung.

In 96% Ethanol ist die Löslichkeit der Substanz größer als 500 mmol/L. Es ergibt sich eine frei fließende Lösung.

### Beispiel 41

### Löslichkeit des Dysprosium-Komplexes aus Beispiel 30f) in verschiedenen Lösungsmitteln und mit verschiedenen Zusätzen und Darreichungsformen.

Von der Verbindung aus Beispiel 30f) wurde eine 100 mmol/L Lösung in Wasser hergestellt. Diese Lösung ist klar durchsichtig und besitzt eine gelartig feste Konsistenz, läßt sich aber problemlos von Hand oder mit einer Infusionspumpe über einen 20 cm langen, 0.58 mm Øᵢ, Schlauch mit einer Geschwindigkeit von 160 µL/min applizieren. Unterhalb von etwa 10-20 mmol/L ergibt sich eine frei fließende Lösung.

Der Zusatz von 10 mmol/L (Endkonzentration) HC1 bzw. NaOH oder hohen Salzkonzentrationen (3 mol/L NaCl) hat keinen Einfluß auf die Konsistenz der Zubereitung.

Der Zusatz von 8 mol/L (Endkonzentration) Harnstoff ergibt eine frei fließende Lösung auch bei sehr hohen Substanzkonzentrationen (400 mmol/L).

Der Zusatz von Detergenzien zu der wässerigen Formulierung der Verbindung aus Beispiel 30f) wirkt sich sehr unterschiedlich aus. Tween® 80 (Polyoxyethylen-sorbitan-oleat, nichtionisches Detergens, HLB 15, 4.6% Endkonzentration) bewirkt keine Veränderung der Konsistenz, während Triton® X-100 (Octylphenol-Polyethylen-glycolether, nichtionisches Detergens, HLB 13.5, 1.7%) zu einer Verflüssigung der gelartigen Konsistenz führt.

In Rinderplasma ist die Substanz ebenso löslich wie in reinem Wasser und ergibt eine Lösung mit vergleichbarer Konsistenz.

In reinem Propylenglycol oder DMSO ist die Verbindung aus Beispiel 30 f) praktisch unlöslich. In 66% Propylenglycol/Wasser bzw. 66% DMSO/Wasser ist die Substanz jedoch auch in höherer Konzentration (250 mmol/L bzw. 150 mmol/L) gut löslich und ergibt eine frei fließende Lösung.

In 96% Ethanol ist die Löslichkeit der Substanz größer als 500 mmol/L. Es ergibt sich eine frei fließende Lösung.

### Beispiel 42

### Einarbeitung von Cytostatika in die Lösung der Verbindung aus Beispiel 1 c)

Die zur Embolisation von Tumoren verwendeten Materialien eignen sich prinzipiell auch zur lokalen Applikation von Cytostatica, die auf diese Weise im Tumor in sehr hohen, systemisch aber niedrigen und damit gut tolerierbaren Konzentrationen vorliegen. Außerdem dient der Embolus einer verzögerten Freisetzung über mehrere Tage, was die Wirksamkeit des Cytostatikums weiter steigert.

Üblicherweise werden für die Behandlung des HCC 5-Fluoruracil, Doxorubicin, Mitomycin C oder Cisplatin neben einigen anderen Cytostatika verwendet. Verwendet man zur Herstellung der Lösung der Verbindung aus Beispiel 1 c) eine wässrige Lösung des Cytostatikums, wird dieses homogen in die Formulierung eingebettet. Die verwendete Konzentration des Cytostatikums hängt von der Menge der zu applizierenden Lösung ab, so daß die maximal tolerierbare Dosis in mg/kg KG oder in mg/m² Körperoberfläche nicht überschritten wird. Für die einzelnen Cytostatika können folgende Lösungen für die Herstellung einer Formulierung aus der Verbindung 1c) verwendet werden:
- Cisplatin 1 - 20 mg/ml
- Mitomycin C 0,5 - 10 mg/ml
- Doxorubicin 1 - 20 mg/ml
- 5-Fluoruracil 10 - 200 mg/ml

### Beispiel 43

Demonstration der Embolisation in einem Tiermodell. Als gut darstellbare Gefäße wurden die Nierengefäße der Ratte embolisiert.

Dem Tier wurde ein Katheter (20 cm lang, 0,58 mm Øᵢ in die A. mesentrerica eingebunden und in die A. renalis der linken Niere vorgeschoben. Die Lösung der Verbindung aus Beispiel 1 c) (hier 50 mmol/1 mit 150 mg J/ml Isovist ®) wurde mit 160 µl/min appliziert. Gesamtvolumen 250 µl. Anschließend wurde der Katheter in die A. mesenterica zurückgezogen, um die Blutversorgung der Nieren im weiteren Verlauf nicht zu beinträchtigen. In den folgenden Röntgenaufnahmen waren die Gefäße der linken Niere scharf gegenüber dem Nierenparenchym abgegrenzt. Bis 45 Minuten nach Gabe der Verbindung aus Beispiel lc) nahm die Signalintensität der Gefäße in der Niere deutlich ab. Dies ist auf ein Herausdiffundieren des Röntgenkontrastmittels Isovist ® und seine renale Elimination zurückzuführen. Zur Kontrolle der Nierendurchblutung wurde 1 Stunde nach der lokalen Applikation der Verbindung aus Beispiel lc) 400 µl des nierengängigen Röntgenkontrastmittels Ultravist ® 300 mg J/ml i.v. in die Schwanzvene gegeben. Nur die nicht embolisierte rechte Niere mit Harnleiter zeigte ein deutliches Enhancement, ein Indiz für die gute Durchblutung und Funktionstüchtigkeit dieser Niere. Die embolisierte linke Niere und ihr Harnleiter dagegen zeigten kein Enhancement, was auf den Fortbestand der Embolie hindeutet.

Die Wirksamkeit des Verfahrens wird durch Bild 1 und 2 wiedergegeben:

### Bild1

### Embolisierung der Niere einer Ratte

Direkt nach intraarterieller, lokaler Gabe von 250 µl einer Lösung der Verbindung aus Beispiel 1 c) (50 mmol/L + 150 mg J/ml Isovist ® )in die katherisierte Nierenarterie (linke Niere, im Bild rechts)

### Bild 2

### Kontrollversuch

5' p.a. Ultravist ®, 400 µl i.v.
1 Stunde nach lokaler Gabe der Verbindung aus Beispiel 1c) wurde Ultravist ® über die Schwanzvene gegeben. Die rechte Niere scheidet das Röntgenkontrastmittel aus, die behandelte (embolisierte) linke Niere bleibt dunkel. Die Blase wird (unten rechts) ebenfalls hell.

### Beispiel 44

### Demonstration der Embolisation in einem weiteren Tiermodell. Als gut darstellbare Gefäße wurden die Lebergefäße am Kaninchen embolisiert.

Dem narkotisierten Tier wurde ein Katheter (60 cm lang, 1,5 mm Øₐ) über die A. femoralis bis in die Leberarterie durch die Aorta vorgeschoben. Die Lösung von Gadoliniumkomplex aus Beispiel 1c) (50 mmol/L mit 150 mg J/ml Isovist ®) wurde mit 160 µL/min appliziert. Gesamtvolumen 800 µL. Anschließend wurde der Katheter in die Aorta zurückgezogen, um die arterielle Blutversorgung der Leber im weiteren Verlauf nicht zu beeinträchtigen. In den folgenden Röntgenaufnahmen waren die Gefäße der Leber scharf gegenüber dem Leberparenchym abgegrenzt. Innerhalb von 60 Minuten nach Gabe der Verbindung aus Beispiel 1c) nahm die Signalintensität der Gefäße in der Leber nur langsam ab, was auf einer fortbestehenden Embolisation durch die Substanz hindeutet. Zum Teil ist die Abnahme der Signalintensität auf ein Herausdiffündieren des Röntgenkontrastmittels Isovist ® zurückzuführen.

### Beispiel 45

### Therapie eines Leber-Carcinoms am Kaninchen

Fünf männlichen Chinchilla-Kaninchen wurde durch Injektion von VX-2 Tumorzellen in den linken Leberlappen ein Tumor gesetzt. Dem narkotisiertem Tier wurde ein Katheter (60 cm lang, 1 mm Øₐ) über die Arteria femoralis bis in die Nähe des Tumors vorgeschoben. Dann wurde eine Lösung der Titelverbindung aus Beispiel 1 c [75 mmol/l mit 50 mg Cisplatin (=Carboplatin ®) appliziert. Gesamtvolumen 8 ml.

Anschließend wurde der Katheter entfernt. Nach 7 Tagen wurden die Tiere mittels MRI untersucht. Es zeigte sich, daß der Tumor sich in seiner Größe verringert hat (durchschnittlicher Wachstumsfaktor 0,8 ± 0,2). Bei den Kontrolltieren (3 Tiere) wurden jedoch Wachstumsraten von 3,7 ± 1,5 beobachtet.

## Patentansprüche

1. Pharmazeutische Mittel enthaltend mindestens eine perfluoralkylhaltige Verbindung der allgemeinen Formel I
R^{F}-L-A I
worin
R^{F} eine perfluorierte, geradkettige oder verzweigte Kohlenstoffkette mit der Formel -CₙF₂ₙX ist, in der
X ein endständiges Fluor-, Chlor-, Brom-, Jod- oder Wasserstoffatom darstellt und n für die Zahlen 4 - 30 steht,
L eine direkte Bindung, eine Methylengruppe, eine -NHCO-Gruppe, eine Gruppe 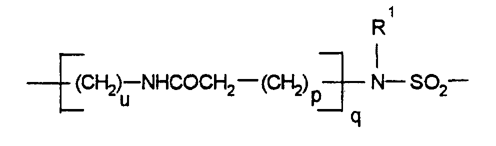 wobei p die Zahlen 0 bis 10, q und u unabhängig voneinander die Zahlen 0 oder 1 und
R¹ ein Wasserstoffatom, eine Methylgruppe, eine -CH₂-OH-Gruppe, eine -CH₂-CO₂H-Gruppe oder eine C₂-C₁₅-Kette ist, die gegebenenfalls unterbrochen ist durch 1 bis 3 Sauerstoffatome, 1 bis 2 >CO-Gruppen oder eine gegebenenfalls substituierte Arylgruppe und/oder substituiert ist mit 1 bis 4 Hydroxylgruppen, 1 bis 2 C₁-C₄-Alkoxygruppen, 1 bis 2 Carboxygruppen, eine Gruppe -SO₃H-bedeuten,
oder eine geradkettige, verzweigte, gesättigte oder ungesättigte C₂-C₃₀-Kohlenstoffkette ist, die gegebenenfalls 1 bis 10 Sauerstoffatome, 1 bis 3 -NR¹-Gruppen, 1 bis 2 Schwefelatome, ein Piperazin, eine -CONR¹-Gruppe, eine -NR¹CO-Gruppe, eine -SO₂-Gruppe, eine -NR¹-CO₂-Gruppe, 1 bis 2 -CO-Gruppen,
eine Gruppe 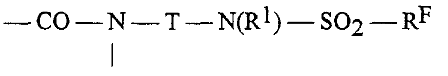 oder 1 bis 2 gegebenenfalls substituierte Aryle enthält und/oder durch diese Gruppen unterbrochen ist, und/oder gegebenenfalls substituiert ist mit
1 bis 3 -OR¹-Gruppen, 1 bis 2 Oxogruppen, 1 bis 2 -NH-COR¹-Gruppen, 1 bis 2 -CONHR¹-Gruppen, 1 bis 2 -(CH₂)ₚ-CO₂H-Gruppen, 1 bis 2 Gruppen -(CH₂)ₚ-(O)_{q}-CH₂CH₂-R^{F},
wobei
R¹, R^{F} und p und q die oben angegebenen Bedeutungen haben und
T eine C₂-C₁₀-Kette bedeutet, die gegebenenfalls durch 1 bis 2 Sauerstoffatome oder 1 bis 2 -NHCO-Gruppen unterbrochen ist,
A für ein Metallkomplex oder dessen Salze organischer und/oder anorganischer Basen oder Aminosäuren oder Aminosäureamide steht, und zwar für einen Komplex der allgemeinen Formel II 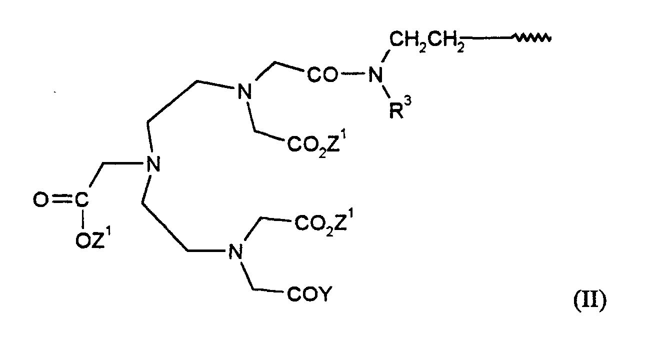 in der R³, Z¹ und Y unabhängig voneinander sind und
R³ die Bedeutung von R¹ hat oder -(CH₂)ₘ-L-R^{F} bedeutet, wobei m 0, 1 oder 2 ist und L und R^{F} die o.g. Bedeutung haben,
Z¹ ein Metallionenäquivalent der Ordnungszahlen 12, 20 - 30, 39, 42, 44 oder 57 - 83 bedeutet,
Y -OZ¹ oder 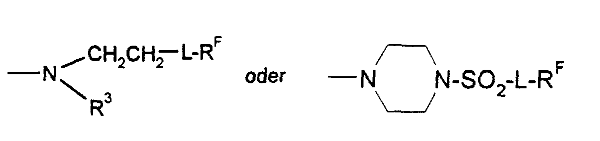 bedeutet, wobei Z^{1,} L, R^{F} und R³ die o. g. Bedeutungen haben,
oder für einen Komplex der allgemeinen Formel III 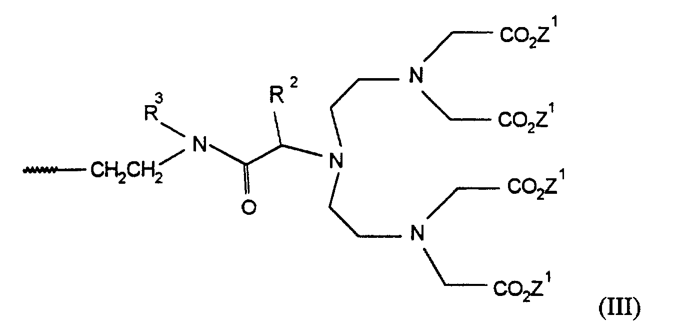 in der R³ und Z¹ die oben genannten Bedeutungen aufweisen und R² die Bedeutung von R¹ hat
oder
für einen Komplex der allgemeinen Formel IV 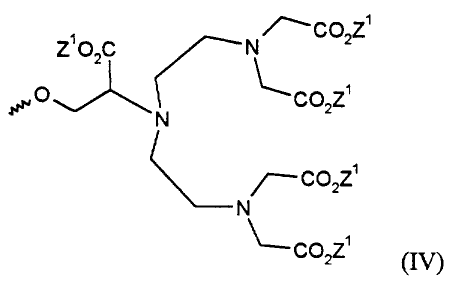 in der Z¹ die oben genannte Bedeutung hat
oder
für einen Komplex der allgemeinen Formel V 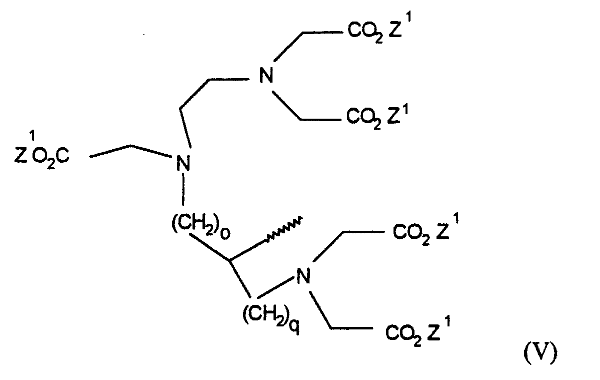 in der Z¹ die oben genannte Bedeutung hat und o und q für die Ziffern O oder 1 stehen und die Summe o + q = 1 ergibt,
oder
für einen Komplex der allgemeinen Formel VI 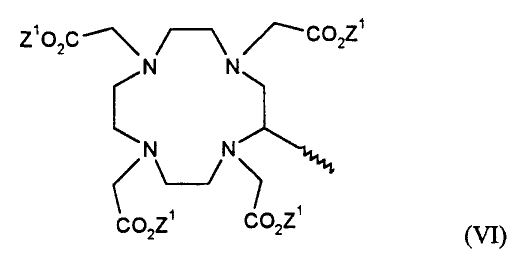 in der Z¹ die oben genannte Bedeutung hat
oder
für einen Komplex der allgemeinen Formel VII 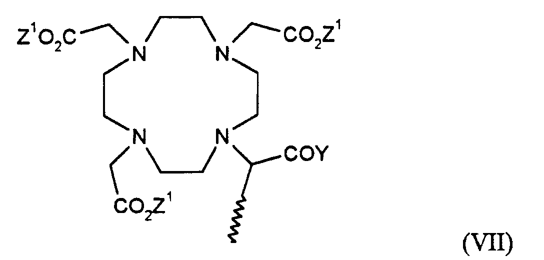 in der Z¹ und Y die oben genannten Bedeutungen haben
oder
für einen Komplex der allgemeinen Formel VIII 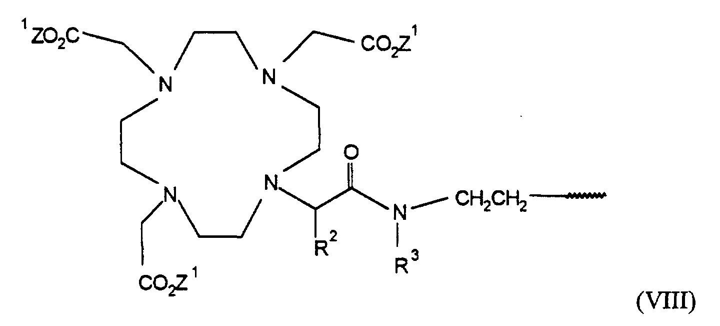 in der R³ und Z¹ die oben genannten Bedeutungen haben und R² die o.g. Bedeutung von R¹ hat.
oder
für einen Komplex der allgemeinen Formel IX 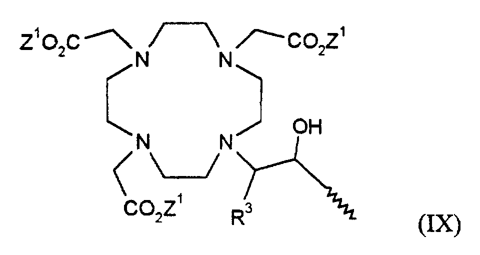 in der R³ und Z¹ die oben genannten Bedeutungen haben,
oder
für einen Komplex der allgemeinen Formel X 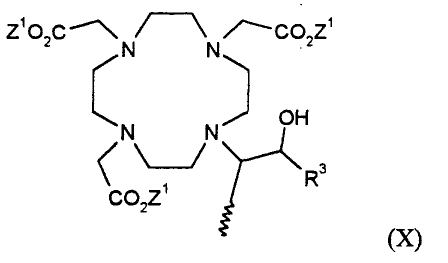 in der R³ und Z¹ die oben genannten Bedeutungen haben,
oder
für einen Komplex der allgemeinen Formel XI 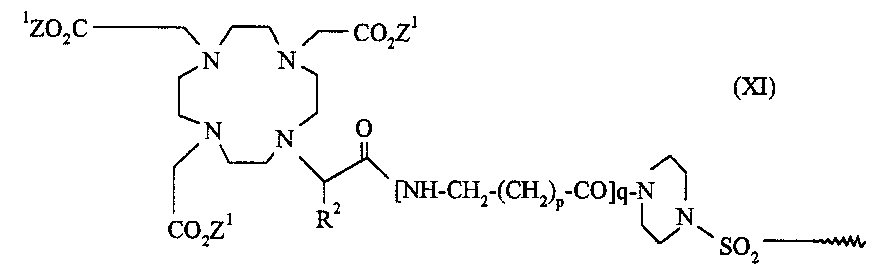 in der Z¹ die oben genannte Bedeutung hat und R² die Bedeutung von R¹ hat.
oder
für einen Komplex der allgemeinen Formel XII 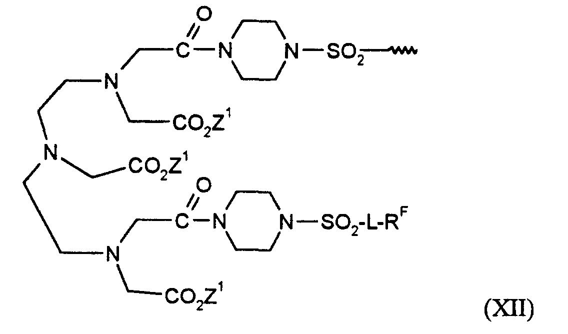 in der L, R^{F} und Z¹ die oben genannten Bedeutungen haben,
oder
für einen Komplex der allgemeinen Formel XIII 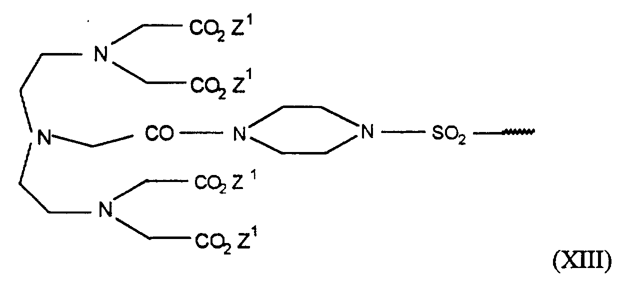 in der Z¹ die oben genannte Bedeutung hat, gegebenenfalls mit den in der Galenik üblichen Zusätzen, für die Tumortherapie.

2. Pharmazeutische Mittel enthaltend mindestens eine perfluoralkylhaltige Verbindung der allgemeinen Formel I gemäß Anspruch 1, gegebenenfalls mit den in der Galenik üblichen Zusätzen für die Therapie des hepatozellulären Carcinoms (HCC).

3. Pharmazeutische Mittel enthaltend mindestens eine perfluoralkylhaltige Verbindung der allgemeinen Formel I gemäß Anspruch 1, gegebenenfalls mit den in der Galenik üblichen Zusätzen für die interventionelle Radiologie.

4. Pharmazeutische Mittel enthaltend mindestens eine Verbindung gemäß einem der Ansprüche 1 bis 3, dadurch gekennzeichnet, daß n in der Formel -CₙF₂ₙ X für die Zahlen 4 - 15 steht.

5. Pharmazeutische Mittel enthaltend mindestens eine Verbindung gemäß einem der Ansprüche 1 bis 4, dadurch gekennzeichnet, daß X in der Formel -CₙF₂ₙX ein Fluoratom bedeutet.

6. Pharmazeutische Mittel gemäß Anspruch 1 enthaltend Gadolinium-Komplex von 10-[2-Hydroxy-4-aza-5-oxo-7-aza-7-(perfluoroctylsulfonyl)-nonyl]-1,4,7-tris(carboxymethyl)-1,4,7,10-tetraazacyclododecan.

7. Pharmazeutische Mittel enthaltend mindestens eine perfluoralkylhaltige Verbindung der allgemeinen Formel I gemäß einem der Ansprüche 1 bis3 und mindestens ein Chemotherapeutikum.

8. Pharmazeutische Mittel gemäß Anspruch 7 enthaltend als Chemotherapeutikum 5-Fluoruracil, Cisplatin, Doxorubicin und/oder Mitomycin C.

9. Pharmazeutische Mittel enthaltend mindestens eine perfluoralkylhaltige Verbindung der allgemeinen Formel I gemäß einem der Ansprüche 1 bis 3 und mindestens ein Kontrastmittel für die NMR- oder Röntgen-Diagnostik.

10. Pharmazeutische Mittel gemäß einem der Ansprüche 7 oder 8 und mindestens ein Kontrastmittel für die NMR- oder Röntgen-Diagnostik.

11. Verwendung von mindestens einer physiologisch verträglichen Verbindung der Formel I gemäß Anspruch 1 zur Herstellung von Medikamenten für die Tumortherapie.

12. Verwendung von mindestens einer physiologisch verträglichen Verbindung der Formel I gemäß Anspruch 1 zur Herstellung von Medikamenten für die Therapie des hepatozellulären Carcinoms (HCC).

13. Verwendung von mindestens einer physiologisch verträglichen Verbindung der Formel I gemäß Anspruch 1 zur Herstellung von Medikamenten für die interventionelle Radiologie.

14. Verwendung von mindestens einer physiologisch verträglichen Verbindung gemäß Anspruch 5 zur Herstellung von Medikamenten für die Tumortherapie oder interventionelle Radiologie.

15. Verwendung gemäß einem der Ansprüche 11 bis13 von mindestens einer physiologisch verträglichen Verbindung der Formel I gemäß Anspruch 1 zusammen mit mindestens einem Chemotherapeutikum.

16. Verwendung gemäß Anspruch 15, dadurch gekennzeichnet, daß als Chemotherapeutikum 5-Fluoruracil, Cisplatin, Doxorubicin und/oder Mitomycin C benutzt wird.

17. Verwendung gemäß einem der Ansprüche 11 bis 13 von mindestens einer physiologisch verträglichen Verbindung der allgemeinen Formel I gemäß Anspruch 1 zusammen mit mindestens einem Kontrastmittel für die NMR- oder Röntgen-Diagnostik.

18. Verwendung gemäß einem der Ansprüche 11 bis 13 von mindestens einer physiologisch verträglichen Verbindung der allgemeinen Formel I gemäß Anspruch 1 zusammen mit mindestens einen Kontrastmittel für die NMR- oder Röntgen-Diagnostik und mindestens einem Chemotherapeutikum.

## Claims

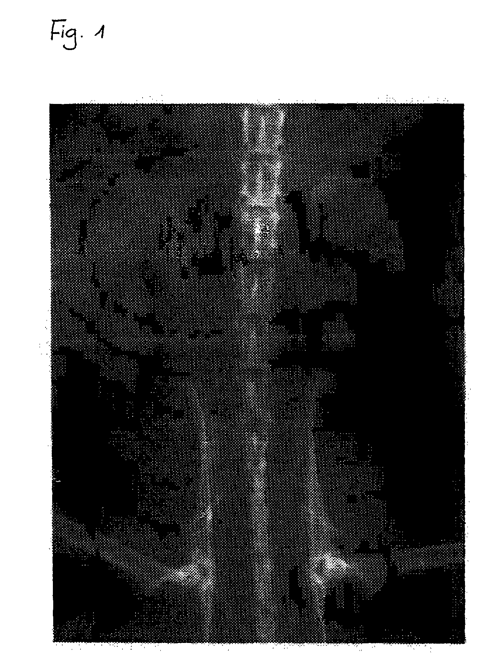

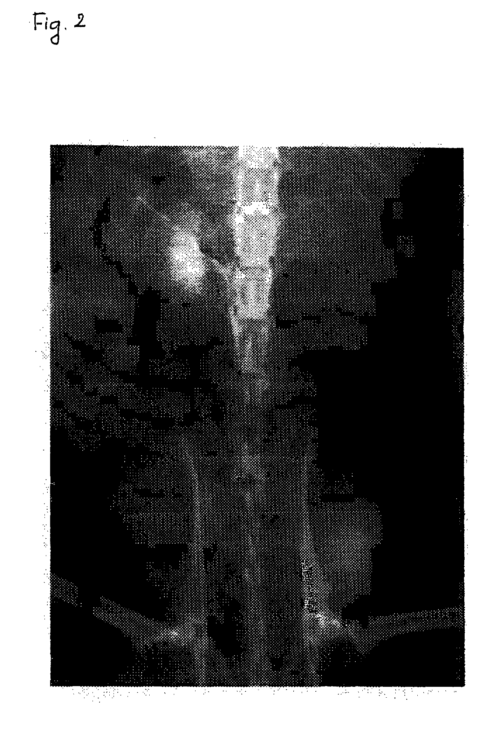

1. Pharmaceutical compositions comprising at least one perfluoroalkyl-containing compound of the general formula I
R^{F}-L-A I
in which
R^{F} is a perfluorinated, straight-chain or branched hydrocarbon chain having the formula -CₙF₂ₙX, in which
X is a terminal fluorine, chlorine, bromine, iodine or hydrogen atom and n represents the numbers 4-30,
L is a direct bond, a methylene group, an -NHCO-group, a group 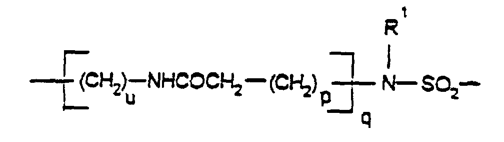 where p is the numbers 0 to 10, q and u independently of one another are the numbers 0 or 1 and
R¹ is a hydrogen atom, a methyl group, a -CH₂-OH group, a -CH₂-CO₂H group or a C₂-C₁₅ chain, which is optionally interrupted by 1 to 3 oxygen atoms, 1 or 2 >CO groups or an optionally substituted aryl group and/or is substituted by 1 to 4 hydroxyl groups, 1 or 2 C₁-C₄-alkoxy groups, 1 or 2 carboxyl groups, a group -SO₃H,
or is a straight-chain, branched, saturated or unsaturated C₂-C₃₀-carbon chain, which optionally contains 1 to 10 oxygen atoms, 1 to 3 -NR¹- groups, 1 or 2 sulphur atoms, a piperazine, a -CONR¹- group, an -NR¹CO- group, an -SO₂- group, an -NR¹-CO₂- group, 1 or 2 CO groups, a group 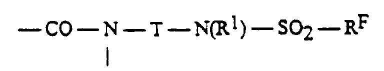 or 1 or 2 optionally substituted aryls and/or is interrupted by these groups,
and/or is optionally substituted by
1 to 3 -OR¹- groups, 1 or 2 oxo groups, 1 or 2 -NH-COR¹- groups, 1 or 2 -CONHR¹- groups, 1 or 2 -(CH₂)ₚ-CO₂H groups, 1 or 2 groups - (CH₂)ₚ-(O)_{q}-CH₂CH₂-R^{F},
where
R¹, R^{F} and p and q have the meanings indicated above and
T is a C₂-C₁₀-chain, which is optionally interrupted by 1 or 2 oxygen atoms or 1 or 2 -NHCO- groups,
A is a metal complex or its salts of organic and/or inorganic bases or amino acids or amino acid amides, to be specific a complex of the general formula II 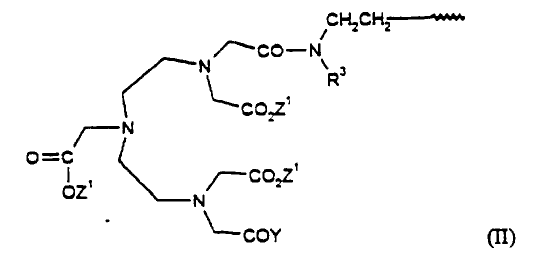 in which R³, Z¹ and Y are independent of one another and
R³ has the meaning of R¹ or is -(CH₂)ₘ-L-R^{F}, where m is 0, 1 or 2 and L and R^{F} have the abovementioned meaning,
Z¹ is a metal ion equivalent of atomic numbers 12, 20 - 30, 39, 42, 44 or 57 - 83,
Y is -OZ¹ or 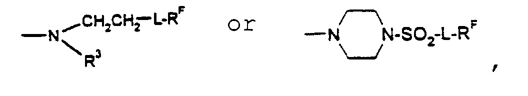 where Z¹, L, R^{F} and R³ have the abovementioned meanings,
or
a complex of the general formula III 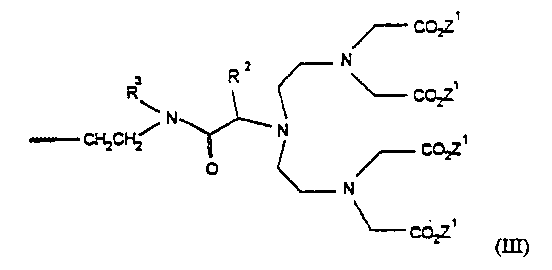 in which R³ and Z¹ have the abovementioned meanings and R² has the meaning of R¹
or
a complex of the general formula IV 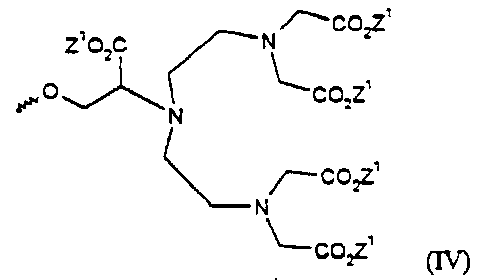 in which Z¹ has the abovementioned meaning
or
a complex of the general formula V 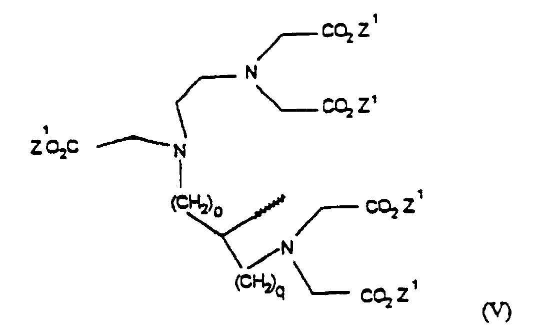 in which Z¹ has the abovementioned meaning and o and q are the figures 0 or 1 and the sum o + q = 1,
or
a complex of the general formula VI 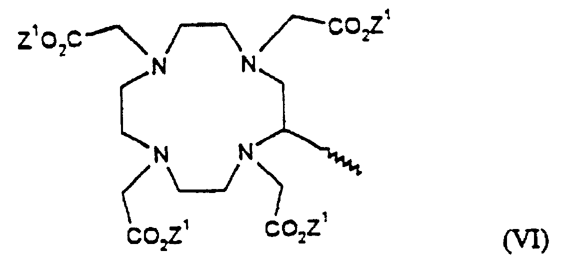 in which Z¹ has the abovementioned meaning
or
a complex of the general formula VII 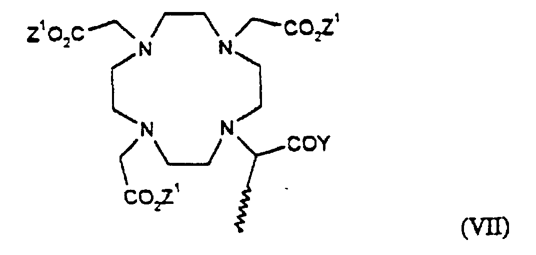 in which Z¹ and Y have the abovementioned meanings
or
a complex of the general formula VIII 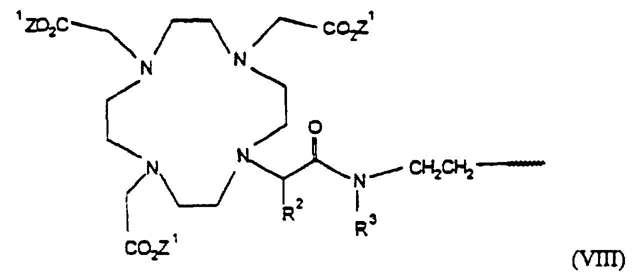 in which R³ and Z¹ have the abovementioned meanings and R² has the abovementioned meaning of R¹,
or
a complex of the general formula IX 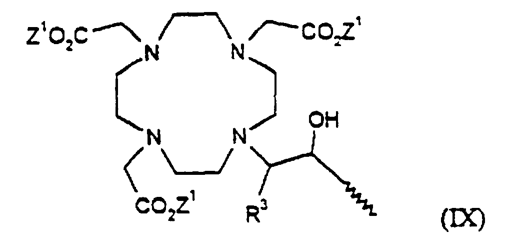 in which R³ and Z¹ have the abovementioned meanings,
or
a complex of the general formula X 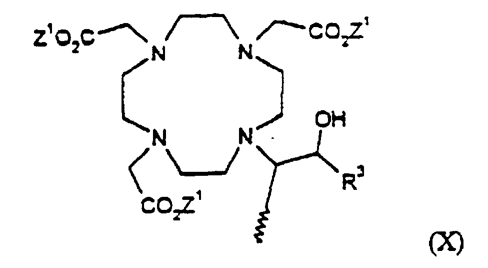 in which R³ and Z¹ have the abovementioned meanings,
or
a complex of the general formula XI 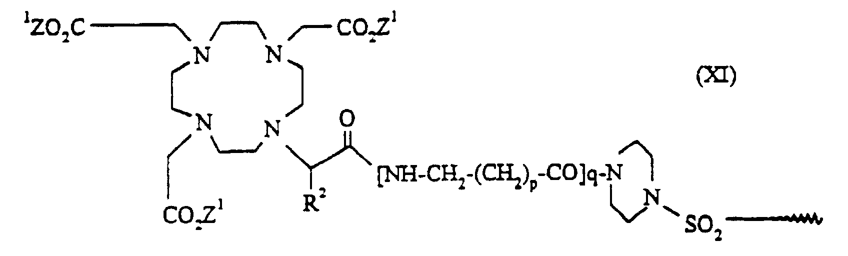 in which Z¹ has the abovementioned meaning and R² has the meaning of R¹,
or
a complex of the general formula XII 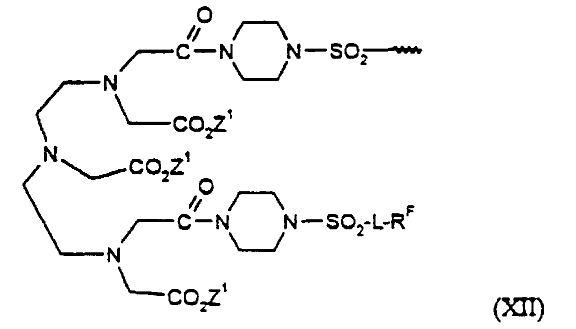 in which L, R^{F} and Z¹ have the abovementioned meanings,
or
a complex of the general formula XIII 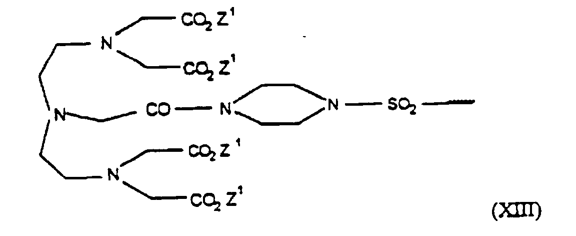 in which Z¹ has the abovementioned meaning, if appropriate with the additives customary in pharmacy, for tumour therapy.

2. Pharmaceutical compositions comprising at least one perfluoroalkyl-containing compound of the general formula I according to Claim 1, if appropriate with the additives customary in pharmacy for the therapy of hepatocellular carcinoma (HCC) .

3. Pharmaceutical compositions comprising at least one perfluoroalkyl-containing compound of the general formula I according to Claim 1, if appropriate with the additives customary in pharmacy for interventional radiology.

4. Pharmaceutical compositions comprising at least one compound according to one of Claims 1 to 3, characterized in that n in the formula -CₙF₂ₙX represents the numbers 4 - 15.

5. Pharmaceutical compositions comprising at least one compound according to one of Claims 1 to 4, characterized in that X in the formula -CₙF₂ₙX is a fluorine atom.

6. Pharmaceutical compositions according to Claim 1, comprising the gadolinium complex of 10-[2-hydroxy-4-aza-5-oxo-7-aza-7-perfluorooctylsulphonylnonyl]-1,4,7-triscarboxymethyl-1,4,7,10-tetraazacyclododecane.

7. Pharmaceutical compositions comprising at least one perfluoroalkyl-containing compound of the general formula I according to one of Claims 1 to 3 and at least one chemotherapeutic.

8. Pharmaceutical compositions according to Claim 7 comprising, as chemotherapeutic, 5-fluorouracil, cisplatin, doxorubicin and/or mitomycin C.

9. Pharmaceutical compositions comprising at least one perfluoroalkyl-containing compound of the general formula I according to one of Claims 1 to 3 and at least one contrast agent for NMR or X-ray diagnosis.

10. Pharmaceutical compositions according to one of Claims 7 and 8 and at least one contrast agent for NMR or X-ray diagnosis.

11. Use of at least one physiologically tolerable compound of the formula I according to Claim 1 for the production of medicaments for tumour therapy.

12. Use of at least one physiologically tolerable compound of the formula I according to Claim 1 for the production of medicaments for the therapy of hepatocellular carcinoma (HCC).

13. Use of at least one physiologically tolerable compound of the formula I according to Claim 1 for the production of medicaments for interventional radiology.

14. Use of at least one physiologically tolerable compound according to Claim 5 for the production of medicaments for tumour therapy or interventional radiology.

15. Use according to one of Claims 11 to 13 of at least one physiologically tolerable compound of the formula I according to Claim 1 together with at least one chemotherapeutic.

16. Use according to Claim 15, characterized in that the chemotherapeutic used is 5-fluorouracil, cisplatin, doxorubicin and/or mitomycin C.

17. Use according to one of Claims 11 to 13 of at least one physiologically tolerable compound of the general formula I according to Claim 1 together with at least one contrast agent for NMR or X-ray diagnosis.

18. Use according to one of Claims 11 to 13 of at least one physiologically tolerable compound of the general formula I according to Claim 1 together with at least one contrast agent for NMR or X-ray diagnosis and at least one chemotherapeutic.

## Revendications

1. Produit pharmaceutique contenant au moins un composé perfluoroalkylé de formule générale I
R^{F}-L-A (I)
dans laquelle
R^{F} est une chaîne carbonée perfluorée, linéaire ou ramifiée, de formule -CₙF₂ₙX, dans laquelle
X représente un atome d'hydrogène ou de fluor, chlore, brome, iode, et n représente un nombre allaut de 4 à 30,
L est une liaison directe, un groupe méthylène, un aroupe -NHCO-, un groupe 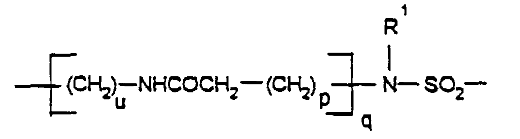
p étant un nombre allant de 0 à 10, q et u représentant, indépendamment l'un de l'autre, le nombre 0 ou 1, et
R¹ représentant un atome d'hydrogène, un groupe méthyle, un groupe -CH₂OH, un groupe -CH₂-CO₂H ou une chaîne en C₂-C₁₅, qui est éventuellement interrompue par 1 à 3 atomes d'oxygène, 1 ou 2 groupes >CO ou un groupe aryle éventuellement substitué et/ou est substituée par 1 à 4 groupes hydroxy, 1 ou 2 groupes alcoxy en C₁-C₄, 1 ou 2 groupes carboxy, un groupe -SO₃H-,
ou une chaîne carbonée en C₂-C₃₀ linéaire ou ramifiée, saturée ou non saturée, qui éventuellement contient 1 à 10 atomes d'oxygène, 1 à 3 groupes NR¹, 1 ou 2 atomes de soufre, une pipérazine, un groupe -CONR¹, 1 ou 2 groupes -NR¹CO, un groupe -SO₂, un groupe -NR¹-CO₂, 1 ou 2 groupes -CO, un groupe 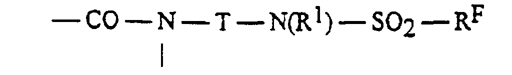 ou 1 ou 2 groupes aryle éventuellement substitués et/ou est interrompue par ces groupes,
et/ou est éventuellement substituée par
1 à 3 groupes -OR¹, 1 ou 2 groupes oxo, 1 ou 2 groupes -NH-COR¹, 1 ou 2 groupes -CONHR¹, 1 ou 2 groupes -(CH₂)ₚ-CO₂H, 1 ou 2 groupes -(CH₂)ₚ-(O)_{q}-CH₂CH₂-R^{F},
R¹, R^{F} et p et q ayant les significations données plus haut et
T représentant une chaîne en C₂-C₁₀ qui est éventuellement interrompue par 1 ou 2 atomes d'oxygène ou 1 ou 2 groupes -NHCO-,
A représente un complexe métallique ou ses sels avec des bases organiques et/ou minérales ou des aminoacides ou des amides d'aminoacides, à savoir, un complexe de formule générale II 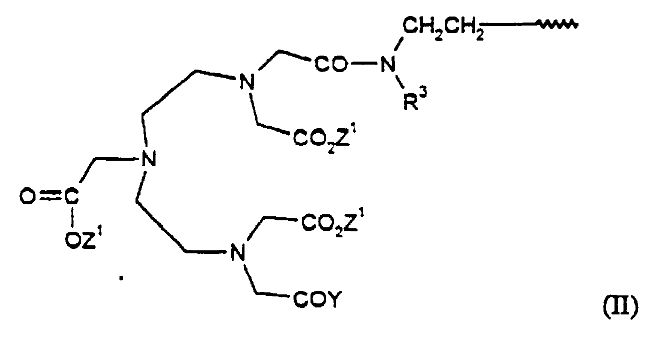 dans laquelle
R³, Z¹ et Y sont indépendants l'un de l'autre et
R³ a la signification de R¹ ou représente un groupe - (CH₂)ₘ-L-R^{F},
m étant 0, 1 ou 2 et L et R^{F} ayant la signification donnée plus haut,
Z¹ représente un équivalent d'ion métallique de nombre atomique 12, 20-30, 39, 42, 44 ou 57-83,
Y représente -OZ¹ ou 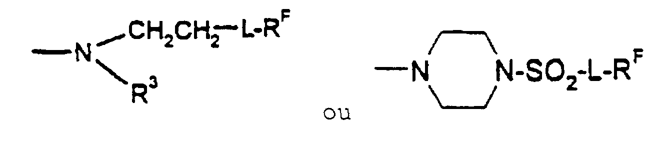
Z¹, L, R^{F} et R³ ayant les significations données plus haut,
ou
représente un complexe de formule générale III 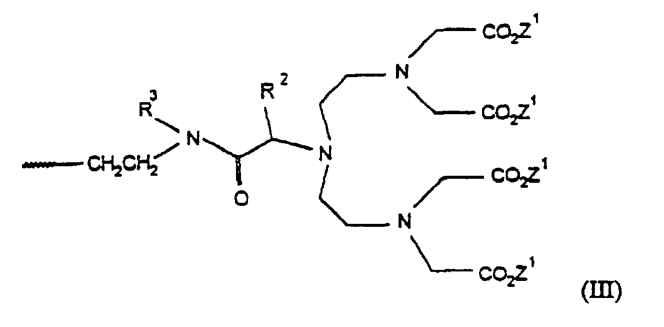 dans laquelle R³ et Z¹ ont les significations données plus haut et R² a la signification de R¹
ou
représente un complexe de formule générale IV 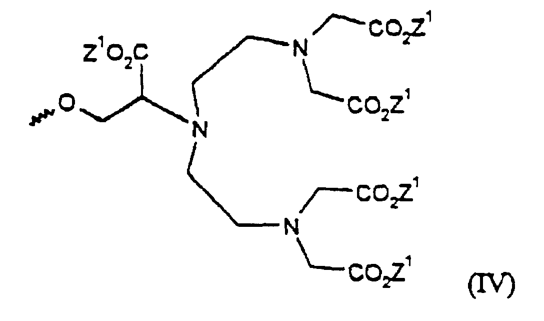 dans laquelle Z¹ a la signification donnée plus haut
ou
représente un complexe de formule générale V 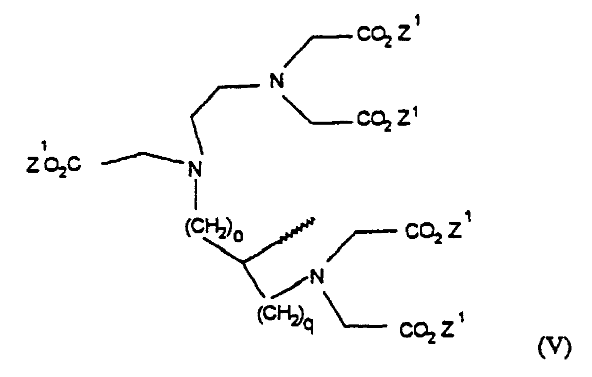 dans laquelle Z¹ a la signification donnée plus haut et o et q représentent le nombre 0 ou 1, et la somme o + q est égale à 1,
ou
représente un complexe de formule générale VI 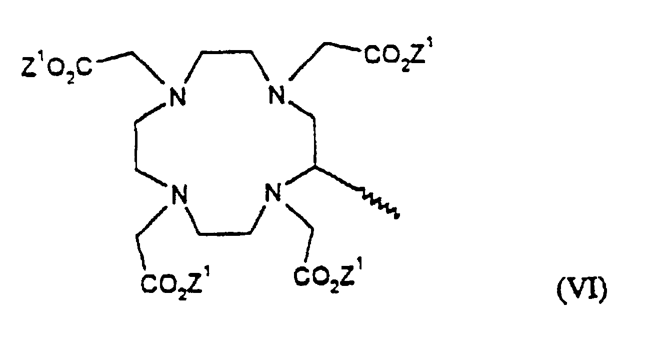 dans laquelle Z¹ a la signification donnée plus haut,
ou
représente un complexe de formule générale VII 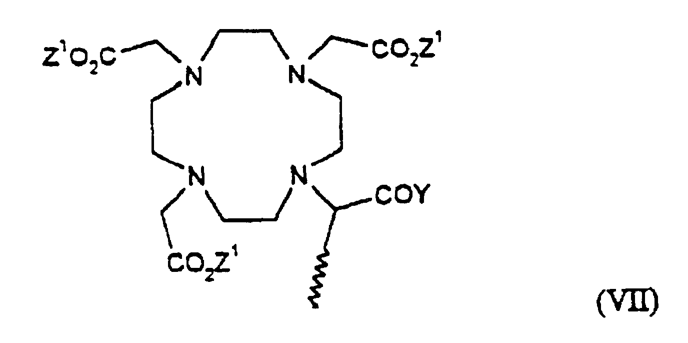 dans laquelle Z¹ et Y ont les significations données plus haut
ou
représente un complexe de formule générale VIII 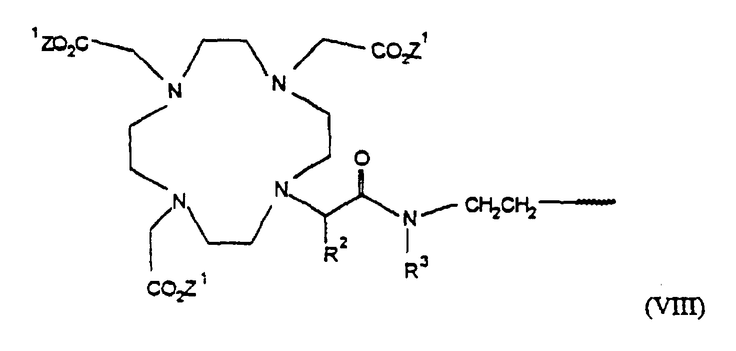 dans laquelle R³ et Z¹ ont les significations données plus haut et R² a la signification de R¹ donnée plus haut,
ou
représente un complexe de formule générale IX 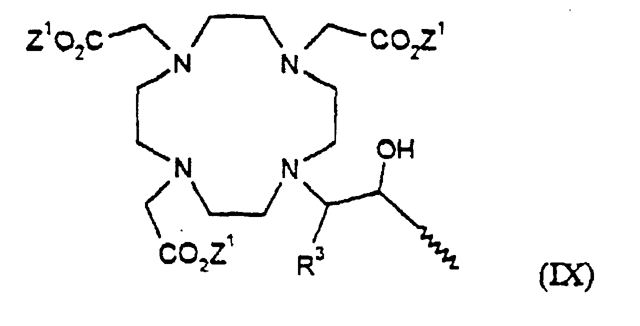 dans laquelle R³ et Z¹ ont les significations données plus haut,
ou
représente un complexe de formule générale X 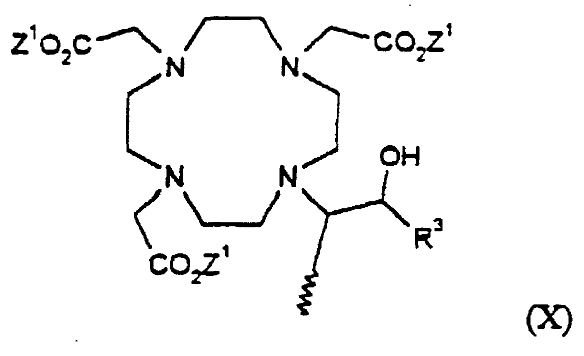 dans laquelle R³ et Z¹ ont les significations données plus haut
ou
représente un complexe de formule générale XI 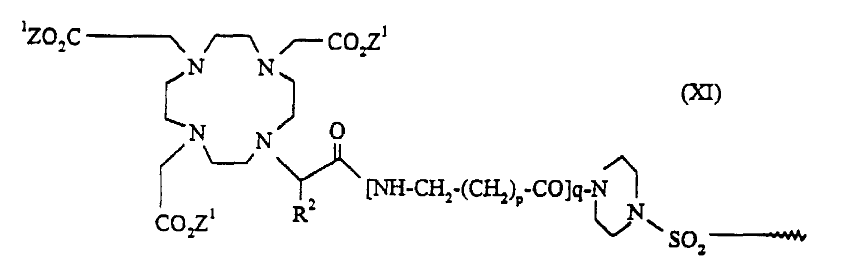 dans laquelle Z¹ a la signification donnée plus haut et R² a la signification de R¹
ou
représente un complexe de formule générale XII 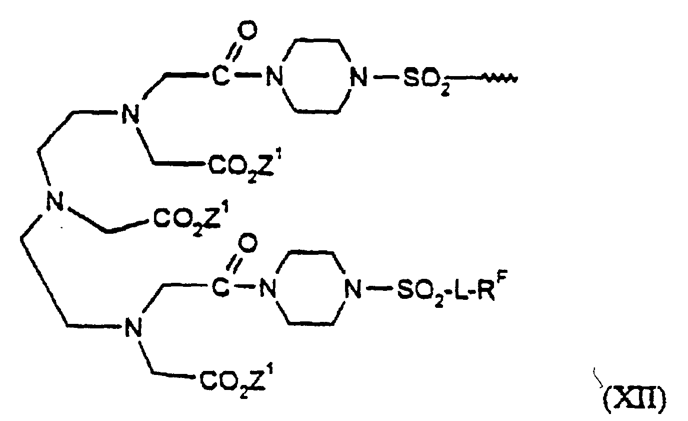 dans laquelle L, R^{F} et Z¹ ont les significations données plus haut,
ou
représente un complexe de formule générale XIII 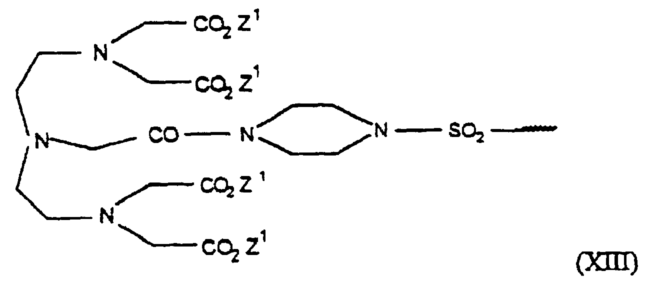 dans laquelle Z¹ a la signification donnée plus haut, éventuellement avec les additifs usuels en formulation galénique, pour le traitement de tumeurs.

2. Produit pharmaceutique contenant au moins un composé perfluoroalkylé de formule générale I selon la revendication 1, éventuellement avec les additifs usuels en formulation galénique, pour le traitement du carcinome hépatocellulaire (HCC) .

3. Produit pharmaceutique contenant au moins un composé perfluoroalkylé de formule générale I selon la revendication 1, éventuellement avec les additifs usuels en formulation galénique, pour la radiologie interventionnelle.

4. Produit pharmaceutique contenant au moins un composé selon l'une des revendications 1 à 3, caractérisé en ce que n dans la formule -CₙF₂ₙX représente un nombre allant de 4 à 15.

5. Produit pharmaceutique contenant au moins un composé selon l'une des revendications 1 à 4, caractérisé en ce que X dans la formule -CₙF₂ₙX représente un atome de fluor.

6. Produit pharmaceutique selon la revendication 1, contenant un complexe de gadolinium de 10-[2-hydroxy-4-aza-5-oxo-7-aza-7-(perfluoro-octylsulfonyl) -nonyl]-1,4,7-tris (carboxyméthyl)-1,4,7,10-tétra-azacyclododécane.

7. Produit pharmaceutique contenant au moins un composé perfluoroalkylé de formule générale I selon l'une des revendications 1 à 3 et au moins un agent chimiothérapeutique.

8. Produit pharmaceutique selon la revendication 7, contenant comme agent chimiothérapeutique du 5-fluoro-uracile, du cisplatine, de la doxorubicine et/ou de la mitomycine C.

9. Produit pharmaceutique contenant au moins un composé perfluoroalkylé de formule générale I selon l'une des revendications 1 à 3 et au moins un agent de contraste pour le diagnostic aux rayons X ou par RMN.

10. Produit pharmaceutique selon la revendication 7 ou 8 et au moins un agent de contraste pour le diagnostic aux rayons X ou par RMN.

11. Utilisation d'au moins un composé physiologiquement acceptable de formule I selon la revendication 1, pour la fabrication de médicaments destinés au traitement de tumeurs.

12. Utilisation d'au moins un composé physiologiquement acceptable de formule I selon la revendication 1, pour la fabrication de médicaments destinés au traitement du carcinome hépatocellulaire (HCC).

13. Utilisation d'au moins un composé physiologiquement acceptable de formule I selon la revendication 1, pour la fabrication de médicaments destinés à la radiologie interventionnelle.

14. Utilisation d'au moins un composé physiologiquement acceptable selon la revendication 5, pour la fabrication de médicaments destinés au traitement de tumeurs ou à la radiologie interventionnelle.

15. Utilisation, selon l'une des revendications 11 à 13, d'au moins un composé physiologiquement acceptable de formule I selon la revendication 1, conjointement avec au moins un agent chimiothérapeutique.

16. Utilisation selon la revendication 15, caractérisée en ce que l'on utilise comme agent chimiothérapeutique le 5-fluoro-uracile, le cisplatine, la doxorubicine et/ou la mitomycine C.

17. Utilisation, selon l'une des revendications 11 à 13, d'au moins un composé physiologiquement acceptable de formule générale I selon la revendication 1, conjointement avec au moins un agent de contraste pour le diagnostic aux rayons X ou par RMN.

18. Utilisation, selon l'une des revendications 11 à 13, d'au moins un composé physiologiquement acceptable de formule générale I selon la revendication 1, conjointement avec au moins un agent de contraste pour le diagnostic aux rayons X ou par RMN et au moins un agent chimiothérapeutique.
